# EUROPEAN PATENT SPECIFICATION

(11) **EP 2 516 650 B1**
(45) Date of publication and mention of the grant of the patent: **17.09.2014**
(21) Application number: 10803687.2
(22) Date of filing: 21.12.2010
(51) Int. Cl.: C12N 15/70, C12P 7/64, C12N 15/52, C12N 9/10, C12N 9/88, C12N 9/02

(54) **BRANCHED-CHAIN FATTY ACIDS AND BIOLOGICAL PRODUCTION THEREOF**
HERSTELLUNG VON FETTSÄUREN UND DERIVATEN DARAUS
ACIDES GRAS À CHAÎNE RAMIFIÉE ET LEUR PRODUCTION BIOLOGIQUE

(30) Priority: 20.12.2010 US 972822; 22.12.2009 US 289039 P
(43) Date of publication of application: 31.10.2012
(73) Proprietor: The Procter & Gamble Company, Cincinnati, OH 45202 (US)
(72) Inventor: SAUNDERS, Charles, Winston, Fairfield Ohio 45014 (US); XU, Jun, Mason Ohio 45040 (US); GREEN, Phillip, Richard, Wyoming Ohio 45231 (US); CODY, David, Blair, West Harrison Indiana 47060 (US); KHAMBATTA, Zubin, Sarosh, Fairfield Ohio 45014 (US)
(74) Representative: Hirsch, Uwe Thomas M.H.
(86) International application number: PCT/US2010/061544
(87) International publication number: WO 2011/087787

(56) References cited:
- ATSUMI SHOTA ET AL: "Directed Evolution of Methanococcus jannaschii Citramalate Synthase for Biosynthesis of 1-Propanol and 1-Butanol by Escherichia coli", APPLIED AND ENVIRONMENTAL MICROBIOLOGY, vol. 74, no. 24, December 2008 (2008-12), pages 7802-7808, XP002627081, ISSN: 0099-2240 cited in the application
- SINGH ATUL K ET AL: "FabH selectivity for anteiso branched-chain fatty acid precursors in low-temperature adaptation in Listeria monocytogenes", FEMS MICROBIOLOGY LETTERS, vol. 301, no. 2, 26 October 2009 (2009-10-26), pages 188-192, XP002627082, ISSN: 0378-1097
- CHOI K H ET AL: "beta-ketoacyl-acyl carrier protein synthase III (FabH) is a determining factor in branched-chain fatty acid biosynthesis", JOURNAL OF BACTERIOLOGY, AMERICAN SOCIETY FOR MICROBIOLOGY, WASHINGTON, DC; US, vol. 182, no. 2, 1 January 2000 (2000-01-01), pages 365-370, XP002451176, ISSN: 0021-9193, DOI: DOI:10.1128/JB.182.2.365-370.2000 cited in the application
- SMIRNOVA N ET AL: "Branched-chain fatty acid biosynthesis in Escherichia coli", JOURNAL OF INDUSTRIAL MICROBIOLOGY AND BIOTECHNOLOGY, vol. 27, no. 4, October 2001 (2001-10), pages 246-251, XP002627083, ISSN: 1367-5435
- WILLECKE K ET AL: "FATTY-ACID REQUIRING MUTANT OF BACILLUS-SUBTILIS DEFECTIVE IN BRANCHED CHAIN ALPHA KETO-ACID DEHYDROGENASE", JOURNAL OF BIOLOGICAL CHEMISTRY, vol. 246, no. 17, 1971, pages 5264-5272, XP002627084, ISSN: 0021-9258
- KANEDA T: "ISO-FATTY AND ANTEISO-FATTY ACIDS IN BACTERIA BIOSYNTHESIS FUNCTION AND TAXONOMIC SIGNIFICANCE", MICROBIOLOGICAL REVIEWS, vol. 55, no. 2, 1991, pages 288-302, XP002627085, ISSN: 0146-0749 cited in the application

### SEQUENCE LISTING

<110> Saunders, et al.
<120> BRANCHED-CHAIN FATTY ACIDS AND BIOLOGICAL PRODUCTION THEREOF
<130> 30766/45879
<150> 61/289,039 <151> 2009-12-22
<160> 92
<170> PatentIn version 3.5
<210> 1
   <211> 4842
   <212> DNA
   <213> Bacillus subtilis
<400> 1
   acagacagga gtgagtcacc atggcaactg agtatgacgt agtcattctg ggcggcggta 60
   ccggcggtta tgttgcggcc atcagagccg ctcagctcgg cttaaaaaca gccgttgtgg 120
   aaaaggaaaa actcggggga acatgtctgc ataaaggctg tatcccgagt aaagcgctgc 180
   ttagaagcgc agaggtatac cggacagctc gtgaagccga tcaattcgga gtggaaacgg 240
   ctggcgtgtc cctcaacttt gaaaaagtgc agcagcgtaa gcaagccgtt gttgataagc 300
   ttgcagcggg tgtaaatcat ttaatgaaaa aaggaaaaat tgacgtgtac accggatatg 360
   gacgtatcct tggaccgtca atcttctctc cgctgccggg aacaatttct gttgagcggg 420
   gaaatggcga agaaaatgac atgctgatcc cgaaacaagt gatcattgca acaggatcaa 480
   gaccgagaat gcttccgggt cttgaagtgg acggtaagtc tgtactgact tcagatgagg 540
   cgctccaaat ggaggagctg ccacagtcaa tcatcattgt cggcggaggg gttatcggta 600
   tcgaatgggc gtctatgctt catgattttg gcgttaaggt aacggttatt gaatacgcgg 660
   atcgcatatt gccgactgaa gatctagaga tttcaaaaga aatggaaagt cttcttaaga 720
   aaaaaggcat ccagttcata acaggggcaa aagtgctgcc tgacacaatg acaaaaacat 780
   cagacgatat cagcatacaa gcggaaaaag acggagaaac cgttacctat tctgctgaga 840
   aaatgcttgt ttccatcggc agacaggcaa atatcgaagg catcggccta gagaacaccg 900
   atattgttac tgaaaatggc atgatttcag tcaatgaaag ctgccaaacg aaggaatctc 960
   atatttatgc aatcggagac gtaatcggtg gcctgcagtt agctcacgtt gcttcacatg 1020
   agggaattat tgctgttgag cattttgcag gtctcaatcc gcatccgctt gatccgacgc 1080
   ttgtgccgaa gtgcatttac tcaagccctg aagctgccag tgtcggctta accgaagacg 1140
   aagcaaaggc gaacgggcat aatgtcaaaa tcggcaagtt cccatttatg gcgattggaa 1200
   aagcgcttgt atacggtgaa agcgacggtt ttgtcaaaat cgtggctgac cgagatacag 1260
   atgatattct cggcgttcat atgattggcc cgcatgtcac cgacatgatt tctgaagcgg 1320
   gtcttgccaa agtgctggac gcaacaccgt gggaggtcgg gcaaacgatt cacccgcatc 1380
   caacgctttc tgaagcaatt ggagaagctg cgcttgccgc agatggcaaa gccattcatt 1440
   tttaaaagca taaaggaggg gcttgaatga gtacaaaccg acatcaagca ctagggctga 1500
   ctgatcagga agccgttgat atgtatagaa ccatgctgtt agcaagaaaa atcgatgaaa 1560
   gaatgtggct gttaaaccgt tctggcaaaa ttccatttgt aatctcttgt caaggacagg 1620
   aagcagcaca ggtaggagcg gctttcgcac ttgaccgtga aatggattat gtattgccgt 1680
   actacagaga catgggtgtc gtgctcgcgt ttggcatgac agcaaaggac ttaatgatgt 1740
   ccgggtttgc aaaagcagca gatccgaact caggaggccg ccagatgccg ggacatttcg 1800
   gacaaaagaa aaaccgcatt gtgacgggat catctccggt tacaacgcaa gtgccgcacg 1860
   cagtcggtat tgcgcttgcg ggacgtatgg agaaaaagga tatcgcagcc tttgttacat 1920
   tcggggaagg gtcttcaaac caaggcgatt tccatgaagg ggcaaacttt gccgctgtcc 1980
   ataagctgcc ggttattttc atgtgtgaaa acaacaaata cgcaatctca gtgccttacg 2040
   ataagcaagt cgcatgtgag aacatttccg accgtgccat aggctatggg atgcctggcg 2100
   taactgtgaa tggaaatgat ccgctggaag tttatcaagc ggttaaagaa gcacgcgaaa 2160
   gggcacgcag aggagaaggc ccgacattaa ttgaaacgat ttcttaccgc cttacaccac 2220
   attccagtga tgacgatgac agcagctaca gaggccgtga agaagtagag gaagcgaaaa 2280
   aaagtgatcc cctgcttact tatcaagctt acttaaagga aacaggcctg ctgtccgatg 2340
   agatagaaca aaccatgctg gatgaaatta tggcaatcgt aaatgaagcg acggatgaag 2400
   cggagaacgc cccatatgca gctcctgagt cagcgcttga ttatgtttat gcgaagtagg 2460
   gaggaagaac aaatgtcagt aatgtcatat attgatgcaa tcaatttggc gatgaaagaa 2520
   gaaatggaac gagattctcg cgttttcgtc cttggggaag atgtaggaag aaaaggcggt 2580
   gtgtttaaag cgacagcggg actctatgaa caatttgggg aagagcgcgt tatggatacg 2640
   ccgcttgctg aatctgcaat cgcaggagtc ggtatcggag cggcaatgta cggaatgaga 2700
   ccgattgctg aaatgcagtt tgctgatttc attatgccgg cagtcaacca aattatttct 2760
   gaagcggcta aaatccgcta ccgcagcaac aatgactgga gctgtccgat tgtcgtcaga 2820
   gcgccatacg gcggaggcgt gcacggagcc ctgtatcatt ctcaatcagt cgaagcaatt 2880
   ttcgccaacc agcccggact gaaaattgtc atgccatcaa caccatatga cgcgaaaggg 2940
   ctcttaaaag ccgcagttcg tgacgaagac cccgtgctgt tttttgagca caagcgggca 3000
   taccgtctga taaagggcga ggttccggct gatgattatg tcctgccaat cggcaaggcg 3060
   gacgtaaaaa gggaaggcga cgacatcaca gtgatcacat acggcctgtg tgtccacttc 3120
   gccttacaag ctgcagaacg tctcgaaaaa gatggcattt cagcgcatgt ggtggattta 3180
   agaacagttt acccgcttga taaagaagcc atcatcgaag ctgcgtccaa aactggaaag 3240
   gttcttttgg tcacagaaga tacaaaagaa ggcagcatca tgagcgaagt agccgcaatt 3300
   atatccgagc attgtctgtt cgacttagac gcgccgatca aacggcttgc aggtcctgat 3360
   attccggcta tgccttatgc gccgacaatg gaaaaatact ttatggtcaa ccctgataaa 3420
   gtggaagcgg cgatgagaga attagcggag ttttaaagac gtaagggagg atacaatcat 3480
   ggcaattgaa caaatgacga tgccgcagct tggagaaagc gtaacagagg ggacgatcag 3540
   caaatggctt gtcgcccccg gtgataaagt gaacaaatac gatccgatcg cggaagtcat 3600
   gacagataag gtaaatgcag aggttccgtc ttcttttact ggtacgataa cagagcttgt 3660
   gggagaagaa ggccaaaccc tgcaagtcgg agaaatgatt tgcaaaattg aaacagaagg 3720
   cgcgaatccg gctgaacaaa aacaagaaca gccagcagca tcagaagccg ctgagaaccc 3780
   tgttgcaaaa agtgctggag cagccgatca gcccaataaa aagcgctact cgccagctgt 3840
   tctccgtttg gccggagagc acggcattga cctcgatcaa gtgacaggaa ctggtgccgg 3900
   cgggcgcatc acacgaaaag atattcagcg cttaattgaa acaggcggcg tgcaagaaca 3960
   gaatcctgag gagctgaaaa cagcagctcc tgcaccgaag tctgcatcaa aacctgagcc 4020
   aaaagaagag acgtcatatc ctgcgtctgc agccggtgat aaagaaatcc ctgtcacagg 4080
   tgtaagaaaa gcaattgctt ccaatatgaa gcgaagcaaa acagaaattc cgcatgcttg 4140
   gacgatgatg gaagtcgacg tcacaaatat ggttgcatat cgcaacagta taaaagattc 4200
   ttttaagaag acagaaggct ttaatttaac gttcttcgcc ttttttgtaa aagcggtcgc 4260
   tcaggcgtta aaagaattcc cgcaaatgaa tagcatgtgg gcgggggaca aaattattca 4320
   gaaaaaggat atcaatattt caattgcagt tgccacagag gattctttat ttgttccggt 4380
   gattaaaaac gctgatgaaa aaacaattaa aggcattgcg aaagacatta ccggcctagc 4440
   taaaaaagta agagacggaa aactcactgc agatgacatg cagggaggca cgtttaccgt 4500
   caacaacaca ggttcgttcg ggtctgttca gtcgatgggc attatcaact accctcaggc 4560
   tgcgattctt caagtagaat ccatcgtcaa acgcccggtt gtcatggaca atggcatgat 4620
   tgctgtcaga gacatggtta atctgtgcct gtcattagat cacagagtgc ttgacggtct 4680
   cgtgtgcgga cgattcctcg gacgagtgaa acaaatttta gaatcgattg acgagaagac 4740
   atctgtttac taaataagca aaaagagcat tttttgaagt tttgtttcaa aaaatgctct 4800
   ttttctatgc tttattattc agcgatccgt attttcattt cg 4842
<210> 2
   <211> 44
   <212> DNA
   <213> Artificial Sequence
<220>
   <223> Synthetic primer
<400> 2
   gagcatgggc ccacagacag gagtgagtca ccatggcaac tgag 44
<210> 3
   <211> 50
   <212> DNA
   <213> Artificial Sequence
<220>
   <223> Synthetic primer
<400> 3
   gagaccacgc gtcgaaatga aaatacggat cgctgaataa taaagcatag 50
<210> 4
   <211> 1425
   <212> DNA
   <213> Escherichia coli
<400> 4
   atggcaactg agtatgacgt agtcattctg ggcggcggta ccggcggtta tgttgcggcc      60
   atcagagccg ctcagctcgg cttaaaaaca gccgttgtgg aaaaggaaaa actcggggga      120
   acatgtctgc ataaaggctg tatcccgagt aaagcgctgc ttagaagcgc agaggtatac     180
   cggacagctc gtgaagccga tcaattcgga gtggaaacgg ctggcgtgtc cctcaacttt     240
   gaaaaagtgc agcagcgtaa gcaagccgtt gttgataagc ttgcagcggg tgtaaatcat     300
   ttaatgaaaa aaggaaaaat tgacgtgtac accggatatg gacgtatcct tggaccgtca     360
   atcttctctc cgctgccggg aacaatttct gttgagcggg gaaatggcga agaaaatgac     420
   atgctgatcc cgaaacaagt gatcattgca acaggatcaa gaccgagaat gcttccgggt     480
   cttgaagtgg acggtaagtc tgtactgact tcagatgagg cgctccaaat ggaggagctg     540
   ccacagtcaa tcatcattgt cggcggaggg gttatcggta tcgaatgggc gtctatgctt     600
   catgattttg gcgttaaggt aacggttatt gaatacgcgg atcgcatatt gccgactgaa     660
   gatctagaga tttcaaaaga aatggaaagt cttcttaaga aaaaaggcat ccagttcata     720
   acaggggcaa aagtgctgcc tgacacaatg acaaaaacat cagacgatat cagcatacaa     780
   gcggaaaaag acggagaaac cgttacctat tctgctgaga aaatgcttgt ttccatcggc     840
   agacaggcaa atatcgaagg catcggccta gagaacaccg atattgttac tgaaaatggc     900
   atgatttcag tcaatgaaag ctgccaaacg aaggaatctc atatttatgc aatcggagac     960
   gtaatcggtg gcctgcagtt agctcacgtt gcttcacatg agggaattat tgctgttgag    1020
   cattttgcag gtctcaatcc gcatccgctt gatccgacgc ttgtgccgaa gtgcatttac    1080
   tcaagccctg aagctgccag tgtcggctta accgaagacg aagcaaaggc gaacgggcat    1140
   aatgtcaaaa tcggcaagtt cccatttatg gcgattggaa aagcgcttgt atacggtgaa    1200
   agcgacggtt ttgtcaaaat cgtggctgac cgagatacag atgatattct cggcgttcat    1260
   atgattggcc cgcatgtcac cgacatgatt tctgaagcgg gtcttgccaa agtgctggac    1320
   gcaacaccgt gggaggtcgg gcaaacgatt cacccgcatc caacgctttc tgaagcaatt    1380
   ggagaagctg cgcttgccgc agatggcaaa gccattcatt tttaa                    1425
<210> 5
   <211> 38
   <212> DNA
   <213> Artificial Sequence
<220>
   <223> Synthetic primer
<400> 5
   gagaccatgg ctaaagctgg aatacttggt gttggacg 38
<210> 6
   <211> 30
   <212> DNA
   <213> Artificial Sequence
<220>
   <223> Synthetic primer
<400> 6
   cgctcctgca gtcttgtgtg cacctcacct 30
<210> 7
   <211> 939
   <212> DNA
   <213> Bacillus subtilis
<400> 7
   atgaaagctg gaatacttgg tgttggacgt tacattcctg agaaggtttt aacaaatcat      60
   gatcttgaaa aaatggttga aacttctgac gagtggattc gtacaagaac aggaatagaa     120
   gaaagaagaa tcgcagcaga tgatgtgttt tcatcacata tggctgttgc agcagcgaaa     180
   aatgcgctgg aacaagctga agtggctgct gaggatctgg atatgatctt ggttgcaact     240
   gttacacctg atcagtcatt ccctacggtc tcttgtatga ttcaagaaca actcggcgcg     300
   aagaaagcgt gtgctatgga tatcagcgcg gcttgtgcgg gcttcatgta cggggttgta     360
   accggtaaac aatttattga atccggaacc tacaagcatg ttctagttgt tggtgtagag     420
   aagctctcaa gcattaccga ctgggaagac cgcaatacag ccgttctgtt tggagacgga     480
   gcaggcgctg cggtagtcgg gccagtcagt gatgacagag gaatcctttc atttgaacta     540
   ggagccgacg gcacaggcgg tcagcacttg tatctgaatg aaaaacgaca tacaatcatg     600
   aatggacgag aagttttcaa atttgcagtc cgccaaatgg gagaatcatg cgtaaatgtc     660
   attgaaaaag ccggactttc aaaagaggat gtcgactttt tgattccgca tcaggcgaac     720
   atccgtatca tggaagctgc tcgcgagcgt ttagagcttc ctgtcgaaaa gatgtctaaa     780
   actgttcata aatatggaaa tacttctgcc gcatccattc cgatctctct tgtagaagaa     840
   ttggaagccg gtaaaatcaa agacggcgat gtggtcgtta tggtagggtt cggcggagga     900
   ctaacatggg gcgccattgc aatccgctgg ggccgataa                            939
<210> 8
   <211> 38
   <212> DNA
   <213> Artificial Sequence
<220>
   <223> Synthetic primer
<400> 8
   gagacgctcg agatgaaagc tggaatactt ggtgttgg 38
<210> 9
   <211> 30
   <212> DNA
   <213> Artificial Sequence
<220>
   <223> Synthetic primer
<400> 9
   cgctcctgca gtcttgtgtg cacctcacct 30
<210> 10
   <211> 312
   <212> PRT
   <213> Bacillus subtilis
<400> 10
   Met Lys Ala Gly Ile Leu Gly Val Gly Arg Tyr Ile Pro Glu Lys Val
   1               5                   10                  15
   Leu Thr Asn His Asp Leu Glu Lys Met Val Glu Thr Ser Asp Glu Trp
               20                  25                  30
   Ile Arg Thr Arg Thr Gly Ile Glu Glu Arg Arg Ile Ala Ala Asp Asp
           35                  40                  45
   Val Phe Ser Ser His Met Ala Val Ala Ala Ala Lys Asn Ala Leu Glu
       50                  55                  60
   Gln Ala Glu Val Ala Ala Glu Asp Leu Asp Met Ile Leu Val Ala Thr
   65                  70                  75                  80
   Val Thr Pro Asp Gln Ser Phe Pro Thr Val Ser Cys Met Ile Gln Glu
                   85                  90                  95
   Gln Leu Gly Ala Lys Lys Ala Cys Ala Met Asp Ile Ser Ala Ala Cys
               100                 105                 110
   Ala Gly Phe Met Tyr Gly Val Val Thr Gly Lys Gln Phe Ile Glu Ser
           115                 120                 125
   Gly Thr Tyr Lys His Val Leu Val Val Gly Val Glu Lys Leu Ser Ser
       130                 135                 140
   Ile Thr Asp Trp Glu Asp Arg Asn Thr Ala Val Leu Phe Gly Asp Gly
   145                 150                 155                 160
   Ala Gly Ala Ala Val Val Gly Pro Val Ser Asp Asp Arg Gly Ile Leu
                   165                 170                 175
   Ser Phe Glu Leu Gly Ala Asp Gly Thr Gly Gly Gln His Leu Tyr Leu
               180                 185                 190
   Asn Glu Lys Arg His Thr Ile Met Asn Gly Arg Glu Val Phe Lys Phe
           195                 200                 205
   Ala Val Arg Gln Met Gly Glu Ser Cys Val Asn Val Ile Glu Lys Ala
       210                 215                 220
   Gly Leu Ser Lys Glu Asp Val Asp Phe Leu Ile Pro His Gln Ala Asn
   225                 230                 235                 240
   Ile Arg Ile Met Glu Ala Ala Arg Glu Arg Leu Glu Leu Pro Val Glu
                   245                 250                 255
   Lys Met Ser Lys Thr Val His Lys Tyr Gly Asn Thr Ser Ala Ala Ser
               260                 265                 270
   Ile Pro Ile Ser Leu Val Glu Glu Leu Glu Ala Gly Lys Ile Lys Asp
           275                 280                 285
   Gly Asp Val Val Val Met Val Gly Phe Gly Gly Gly Leu Thr Trp Gly
       290                 295                 300
   Ala Ile Ala Ile Arg Trp Gly Arg
   305                 310
<210> 11
   <211> 36
   <212> DNA
   <213> Artificial Sequence
<220>
   <223> Synthetic primer
<400> 11
   gagaccatgg caaaagcaaa aattacagct atcggc 36
<210> 12
   <211> 32
   <212> DNA
   <213> Artificial Sequence
<220>
   <223> Synthetic primer
<400> 12
   gctcctgcag ggaagaaaca tcagaagaac ag 32
<210> 13
   <211> 978
   <212> DNA
   <213> Bacillus subtilis
<400> 13
   atgtcaaaag caaaaattac agctatcggc acctatgcgc cgagcagacg tttaaccaat      60
   gcagatttag aaaagatcgt tgatacctct gatgaatgga tcgttcagcg cacaggaatg     120
   agagaacgcc ggattgcgga tgaacatcaa tttacctctg atttatgcat agaagcggtg     180
   aagaatctca agagccgtta taaaggaacg cttgatgatg tcgatatgat cctcgttgcc     240
   acaaccacat ccgattacgc ctttccgagt acggcatgcc gcgtacagga atatttcggc     300
   tgggaaagca ccggcgcgct ggatattaat gcgacatgcg ccgggctgac atacggcctc     360
   catttggcaa atggattgat cacatctggc cttcatcaaa aaattctcgt catcgccgga     420
   gagacgttat caaaggtaac cgattatacc gatcgaacga catgcgtact gttcggcgat     480
   gccgcgggtg cgctgttagt agaacgagat gaagagacgc cgggatttct tgcgtctgta     540
   caaggaacaa gcgggaacgg cggcgatatt ttgtatcgtg ccggactgcg aaatgaaata     600
   aacggtgtgc agcttgtcgg ttccggaaaa atggtgcaaa acggacgcga ggtatataaa     660
   tgggccgcaa gaaccgtccc tggcgaattt gaacggcttt tacataaagc aggactcagc     720
   tccgatgatc tcgattggtt tgttcctcac agcgccaact tgcgcatgat cgagtcaatt     780
   tgtgaaaaaa caccgttccc gattgaaaaa acgctcacta gtgttgagca ctacggaaac     840
   acgtcttcgg tttcaattgt tttggcgctc gatctcgcag tgaaagccgg gaagctgaaa     900
   aaagatcaaa tcgttttgct tttcgggttt ggcggcggat taacctatac aggattgctt     960
   attaaatggg ggatgtaa                                                   978
<210> 14
   <211> 41
   <212> DNA
   <213> Artificial Sequence
<220>
   <223> Synthetic primer
<400> 14
   gagacgctcg agatgtcaaa agcaaaaatt acagctatcg g 41
<210> 15
   <211> 35
   <212> DNA
   <213> Artificial Sequence
<220>
   <223> Synthetic primer
<400> 15
   gctcctgcag gaaggaagaa acatcagaag aacag 35
<210> 16
   <211> 325
   <212> PRT
   <213> Bacillus subtilis
<400> 16
   Met Ser Lys Ala Lys Ile Thr Ala Ile Gly Thr Tyr Ala Pro Ser Arg
   1               5                   10                  15
   Arg Leu Thr Asn Ala Asp Leu Glu Lys Ile Val Asp Thr Ser Asp Glu
               20                  25                  30
   Trp Ile Val Gln Arg Thr Gly Met Arg Glu Arg Arg Ile Ala Asp Glu
           35                  40                  45
   His Gln Phe Thr Ser Asp Leu Cys Ile Glu Ala Val Lys Asn Leu Lys
       50                  55                  60
   Ser Arg Tyr Lys Gly Thr Leu Asp Asp Val Asp Met Ile Leu Val Ala
   65                  70                  75                  80
   Thr Thr Thr Ser Asp Tyr Ala Phe Pro Ser Thr Ala Cys Arg Val Gln
                   85                  90                  95
   Glu Tyr Phe Gly Trp Glu Ser Thr Gly Ala Leu Asp Ile Asn Ala Thr
               100                 105                 110
   Cys Ala Gly Leu Thr Tyr Gly Leu His Leu Ala Asn Gly Leu Ile Thr
           115                 120                 125
   Ser Gly Leu His Gln Lys Ile Leu Val Ile Ala Gly Glu Thr Leu Ser
       130                 135                 140
   Lys Val Thr Asp Tyr Thr Asp Arg Thr Thr Cys Val Leu Phe Gly Asp
   145                 150                 155                 160
   Ala Ala Gly Ala Leu Leu Val Glu Arg Asp Glu Glu Thr Pro Gly Phe
                   165                 170                 175
   Leu Ala Ser Val Gln Gly Thr Ser Gly Asn Gly Gly Asp Ile Leu Tyr
               180                 185                 190
   Arg Ala Gly Leu Arg Asn Glu Ile Asn Gly Val Gln Leu Val Gly Ser
           195                 200                 205
   Gly Lys Met Val Gln Asn Gly Arg Glu Val Tyr Lys Trp Ala Ala Arg
       210                 215                 220
   Thr Val Pro Gly Glu Phe Glu Arg Leu Leu His Lys Ala Gly Leu Ser
   225                 230                 235                 240
   Ser Asp Asp Leu Asp Trp Phe Val Pro His Ser Ala Asn Leu Arg Met
                   245                 250                 255
   Ile Glu Ser Ile Cys Glu Lys Thr Pro Phe Pro Ile Glu Lys Thr Leu
               260                 265                 270
   Thr Ser Val Glu His Tyr Gly Asn Thr Ser Ser Val Ser Ile Val Leu
           275                 280                 285
   Ala Leu Asp Leu Ala Val Lys Ala Gly Lys Leu Lys Lys Asp Gln Ile
       290                 295                 300
   Val Leu Leu Phe Gly Phe Gly Gly Gly Leu Thr Tyr Thr Gly Leu Leu
   305                 310                 315                 320
   Ile Lys Trp Gly Met
                   325
<210> 17
   <211> 756
   <212> DNA
   <213> Anas platyrhynchos
<400> 17
   atggataaag ttattgcacg tccgtataaa cggcctaatg cgttatgcag attgatttgt 60
   tttccgtggg caggcggaaa ctgctcattt ttcatcagat ggtgtgaagc gttttcaagc 120
   attatcgttg tgtctgtgat tcgccttgct ggcagagaat gccgcgatac agaaccgttt 180
   cctgaagata tggctgaagt cgtaaatgaa atcacgaacg ccctgcttaa agatttgcaa 240
   gaaaaaccgt ttgcattgtt tggccatagc tttggatctt ttgtcagcta tgcactggcg 300
   gtacatctta aagaaaaaca tggattagaa ccggtccaca tgtttttctc aggcagctat 360
   ggccctcatt ctgaatactt tcatttgatg tacaaattgc cggaagtaga agattcacgc 420
   ttattggaac tgattcatac acttggcgga acgccgcctg aatttttgca aaacgaacag 480
   atcacaaaac atctgcttcg tgttctgaaa gaagatcaga aagttcttgt gacgtatcct 540
   tggcatgatg tgcggaaaaa atacttttct tgcgatctga catgttttaa tggctcagat 600
   gagaaaaatc atggctcaga agcatggatt gccatcacat caggcgatac gagcatctac 660
   tctttaccgg gaaaccattt ttacttgatg gaaccttcaa acgaaacatt tctgatcaaa 720
   tacatcacga aatgtattga aaacagcgat atttaa 756
<210> 18
   <211> 756
   <212> DNA
   <213> Anas platyrhynchos
<400> 18
   atggataagg tgattgcccg tccatacaaa aggccaaatg ctctctgtag gctgatttgc 60
   tttccctggg ctggaggtaa ctgttctttc tttattcgat ggtgcgaagc cttcagcagc 120
   ataattgtag tgtccgttat aaggcttgct ggaagagaat gtcgtgatac ggagcctttt 180
   ccagaagaca tggcagaagt agttaatgaa attacaaatg ctttgttaaa agatctgcaa 240
   gaaaaaccat ttgcattatt tggtcacagt tttggatctt tcgttagtta tgcacttgca 300
   gtacacttga aagagaagca cggattagag ccggtccata tgtttttctc agggtcatat 360
   ggcccacata gtgaatattt tcatctgatg tacaaattgc ctgaagtaga agatagtcgc 420
   ttgcttgaac ttatacacac attaggagga actcctcctg agtttctgca aaatgaacaa 480
   atcaccaaac atttgttacg tgttttaaaa gaagaccaga aagttcttgt cacatatcct 540
   tggcacgatg taagaaagaa atacttctct tgtgatctta cctgctttaa cgggtctgat 600
   gaaaaaaacc atggctcaga agcctggatt gcaataacca gtggagatac ttccatttac 660
   agtcttcctg gaaatcactt ttatctaatg gagccttcta acgaaacttt cttgataaaa 720
   tacataacta aatgtataga aaattctgac atatga 756
<210> 19
   <211> 792
   <212> DNA
   <213> Rattus norvegicus
<400> 19
   atggaaacag ctgttaatgc caaatcaccg agaaacgaaa aagtgcttaa ctgcttgtac      60
   caaaacccgg atgcagtttt taaactgatt tgttttcctt gggctggcgg aggcagcatt     120
   cattttgcca aatggggaca gaaaatcaac gattctttgg aagtccatgc agtacgcctg     180
   gcgggaagag aaacacgcct tggcgaaccg tttgcgaacg atatttacca aatcgcagat     240
   gaaatcgtta cggcgctgct tcctatcatc caggataaag catttgcatt tttcggacat     300
   agctttggct cttatattgc tttaatcaca gccttgttgc tgaaagaaaa atacaaaatg     360
   gaaccgttgc atatctttgt gtcaggcgct agcgcccctc attctacatc acgtccgcaa     420
   gtccctgatt tgaacgaact gacggaagaa caagtacggc atcatctttt agattttgga     480
   ggcacgccga aacatttgat cgaagatcaa gatgtcttga gaatgtttat ccctttgctg     540
   aaagcagatg cgggagttgt gaaaaaattt atctttgata aaccgtctaa agctcttctg     600
   tcactggata ttacaggatt tttaggctca gaagatacga ttaaagatat tgaaggatgg     660
   caggatctta caagcggcaa atttgatgta cacatgctgc cgggcgatca tttttatctt     720
   atgaaacctg ataacgaaaa ctttatcaaa aactacatcg cgaaatgctt ggaactgtca     780
   tcactgacgt aa                                                         792
<210> 20
   <211> 792
   <212> DNA
   <213> Rattus norvegicus
<400> 20
   atggagacag cagtcaatgc taagagtccc aggaatgaaa aggttttgaa ctgtttgtat 60
   caaaatcctg atgcagtttt caagctgatc tgcttccctt gggcaggagg cggctccatc 120
   cattttgcca agtggggcca aaagattaac gactctctgg aagtgcatgc tgtaagactg 180
   gctggaagag aaacccgact tggagaacct ttcgcaaatg acatctacca gatagctgat 240
   gaaatcgtga ccgccctgtt gcccatcatt caggataaag cttttgcgtt ttttggccac 300
   agttttggat cctacattgc tcttattact gctctgctcc taaaggagaa atacaaaatg 360
   gagccgctgc atatttttgt atccggtgca tccgcccctc actcaacatc ccggcctcaa 420
   gttcctgatc ttaacgaatt gacagaagaa caagtcagac atcaccttct ggatttcgga 480
   ggcacgccca agcatctcat agaagaccag gatgttctga ggatgttcat tcctttgctg 540
   aaggcagatg ctggcgttgt gaaaaaattc atctttgaca agccctccaa agctcttctc 600
   tctctggaca taacgggctt ccttggatct gaagatacaa taaaggacat agaaggctgg 660
   caagacctaa ccagtgggaa gtttgatgtc cacatgctgc caggcgacca cttttatctg 720
   atgaagcccg acaacgagaa ctttatcaag aactacatag ccaagtgctt ggaactctcg 780
   tcactcactt ga 792
<210> 21
   <211> 993
   <212> DNA
   <213> Bacillus subtilis
<400> 21
   atgagtacaa accgacatca agcactaggg ctgactgatc aggaagccgt tgatatgtat 60
   agaaccatgc tgttagcaag aaaaatcgat gaaagaatgt ggctgttaaa ccgttctggc 120
   aaaattccat ttgtaatctc ttgtcaagga caggaagcag cacaggtagg agcggctttc 180
   gcacttgacc gtgaaatgga ttatgtattg ccgtactaca gagacatggg tgtcgtgctc 240
   gcgtttggca tgacagcaaa ggacttaatg atgtccgggt ttgcaaaagc agcagatccg 300
   aactcaggag gccgccagat gccgggacat ttcggacaaa agaaaaaccg cattgtgacg 360
   ggatcatctc cggttacaac gcaagtgccg cacgcagtcg gtattgcgct tgcgggacgt 420
   atggagaaaa aggatatcgc agcctttgtt acattcgggg aagggtcttc aaaccaaggc 480
   gatttccatg aaggggcaaa ctttgccgct gtccataagc tgccggttat tttcatgtgt 540
   gaaaacaaca aatacgcaat ctcagtgcct tacgataagc aagtcgcatg tgagaacatt 600
   tccgaccgtg ccataggcta tgggatgcct ggcgtaactg tgaatggaaa tgatccgctg 660
   gaagtttatc aagcggttaa agaagcacgc gaaagggcac gcagaggaga aggcccgaca 720
   ttaattgaaa cgatttctta ccgccttaca ccacattcca gtgatgacga tgacagcagc 780
   tacagaggcc gtgaagaagt agaggaagcg aaaaaaagtg atcccctgct tacttatcaa 840
   gcttacttaa aggaaacagg cctgctgtcc gatgagatag aacaaaccat gctggatgaa 900
   attatggcaa tcgtaaatga agcgacggat gaagcggaga acgccccata tgcagctcct 960
   gagtcagcgc ttgattatgt ttatgcgaag tag 993
<210> 22
   <211> 984
   <212> DNA
   <213> Bacillus subtilis
<400> 22
   atgtcagtaa tgtcatatat tgatgcaatc aatttggcga tgaaagaaga aatggaacga 60
   gattctcgcg ttttcgtcct tggggaagat gtaggaagaa aaggcggtgt gtttaaagcg 120
   acagcgggac tctatgaaca atttggggaa gagcgcgtta tggatacgcc gcttgctgaa 180
   tctgcaatcg caggagtcgg tatcggagcg gcaatgtacg gaatgagacc gattgctgaa 240
   atgcagtttg ctgatttcat tatgccggca gtcaaccaaa ttatttctga agcggctaaa 300
   atccgctacc gcagcaacaa tgactggagc tgtccgattg tcgtcagagc gccatacggc 360
   ggaggcgtgc acggagccct gtatcattct caatcagtcg aagcaatttt cgccaaccag 420
   cccggactga aaattgtcat gccatcaaca ccatatgacg cgaaagggct cttaaaagcc 480
   gcagttcgtg acgaagaccc cgtgctgttt tttgagcaca agcgggcata ccgtctgata 540
   aagggcgagg ttccggctga tgattatgtc ctgccaatcg gcaaggcgga cgtaaaaagg 600
   gaaggcgacg acatcacagt gatcacatac ggcctgtgtg tccacttcgc cttacaagct 660
   gcagaacgtc tcgaaaaaga tggcatttca gcgcatgtgg tggatttaag aacagtttac 720
   ccgcttgata aagaagccat catcgaagct gcgtccaaaa ctggaaaggt tcttttggtc 780
   acagaagata caaaagaagg cagcatcatg agcgaagtag ccgcaattat atccgagcat 840
   tgtctgttcg acttagacgc gccgatcaaa cggcttgcag gtcctgatat tccggctatg 900
   ccttatgcgc cgacaatgga aaaatacttt atggtcaacc ctgataaagt ggaagcggcg 960
   atgagagaat tagcggagtt ttaa 984
<210> 23
   <211> 1275
   <212> DNA
   <213> Bacillus subtilis
<400> 23
   atggcaattg aacaaatgac gatgccgcag cttggagaaa gcgtaacaga ggggacgatc 60
   agcaaatggc ttgtcgcccc cggtgataaa gtgaacaaat acgatccgat cgcggaagtc 120
   atgacagata aggtaaatgc agaggttccg tcttctttta ctggtacgat aacagagctt 180
   gtgggagaag aaggccaaac cctgcaagtc ggagaaatga tttgcaaaat tgaaacagaa 240
   ggcgcgaatc cggctgaaca aaaacaagaa cagccagcag catcagaagc cgctgagaac 300
   cctgttgcaa aaagtgctgg agcagccgat cagcccaata aaaagcgcta ctcgccagct 360
   gttctccgtt tggccggaga gcacggcatt gacctcgatc aagtgacagg aactggtgcc 420
   ggcgggcgca tcacacgaaa agatattcag cgcttaattg aaacaggcgg cgtgcaagaa 480
   cagaatcctg aggagctgaa aacagcagct cctgcaccga agtctgcatc aaaacctgag 540
   ccaaaagaag agacgtcata tcctgcgtct gcagccggtg ataaagaaat ccctgtcaca 600
   ggtgtaagaa aagcaattgc ttccaatatg aagcgaagca aaacagaaat tccgcatgct 660
   tggacgatga tggaagtcga cgtcacaaat atggttgcat atcgcaacag tataaaagat 720
   tcttttaaga agacagaagg ctttaattta acgttcttcg ccttttttgt aaaagcggtc 780
   gctcaggcgt taaaagaatt cccgcaaatg aatagcatgt gggcggggga caaaattatt 840
   cagaaaaagg atatcaatat ttcaattgca gttgccacag aggattcttt atttgttccg 900
   gtgattaaaa acgctgatga aaaaacaatt aaaggcattg cgaaagacat taccggccta 960
   gctaaaaaag taagagacgg aaaactcact gcagatgaca tgcagggagg cacgtttacc 1020
   gtcaacaaca caggttcgtt cgggtctgtt cagtcgatgg gcattatcaa ctaccctcag 1080
   gctgcgattc ttcaagtaga atccatcgtc aaacgcccgg ttgtcatgga caatggcatg 1140
   attgctgtca gagacatggt taatctgtgc ctgtcattag atcacagagt gcttgacggt 1200
   ctcgtgtgcg gacgattcct cggacgagtg aaacaaattt tagaatcgat tgacgagaag 1260
   acatctgttt actaa 1275
<210> 24
   <211> 474
   <212> PRT
   <213> Bacillus subtilis
<400> 24
   Met Ala Thr Glu Tyr Asp Val Val Ile Leu Gly Gly Gly Thr Gly Gly
   1               5                   10                  15
   Tyr Val Ala Ala Ile Arg Ala Ala Gln Leu Gly Leu Lys Thr Ala Val
               20                  25                  30
   Val Glu Lys Glu Lys Leu Gly Gly Thr Cys Leu His Lys Gly Cys Ile
           35                  40                  45
   Pro Ser Lys Ala Leu Leu Arg Ser Ala Glu Val Tyr Arg Thr Ala Arg
       50                  55                  60
   Glu Ala Asp Gln Phe Gly Val Glu Thr Ala Gly Val Ser Leu Asn Phe
   65                  70                  75                  80
   Glu Lys Val Gln Gln Arg Lys Gln Ala Val Val Asp Lys Leu Ala Ala
                   85                  90                  95
   Gly Val Asn His Leu Met Lys Lys Gly Lys Ile Asp Val Tyr Thr Gly
               100                 105                 110
   Tyr Gly Arg Ile Leu Gly Pro Ser Ile Phe Ser Pro Leu Pro Gly Thr
           115                 120                 125
   Ile Ser Val Glu Arg Gly Asn Gly Glu Glu Asn Asp Met Leu Ile Pro
       130                 135                 140
   Lys Gln Val Ile Ile Ala Thr Gly Ser Arg Pro Arg Met Leu Pro Gly
   145                 150                 155                 160
   Leu Glu Val Asp Gly Lys Ser Val Leu Thr Ser Asp Glu Ala Leu Gln
                   165                 170                 175
   Met Glu Glu Leu Pro Gln Ser Ile Ile Ile Val Gly Gly Gly Val Ile
               180                 185                 190
   Gly Ile Glu Trp Ala Ser Met Leu His Asp Phe Gly Val Lys Val Thr
           195                 200                 205
   Val Ile Glu Tyr Ala Asp Arg Ile Leu Pro Thr Glu Asp Leu Glu Ile
       210                 215                 220
   Ser Lys Glu Met Glu Ser Leu Leu Lys Lys Lys Gly Ile Gln Phe Ile
   225                 230                 235                 240
   Thr Gly Ala Lys Val Leu Pro Asp Thr Met Thr Lys Thr Ser Asp Asp
                   245                 250                 255
   Ile Ser Ile Gln Ala Glu Lys Asp Gly Glu Thr Val Thr Tyr Ser Ala
               260                 265                 270
   Glu Lys Met Leu Val Ser Ile Gly Arg Gln Ala Asn Ile Glu Gly Ile
           275                 280                 285
   Gly Leu Glu Asn Thr Asp Ile Val Thr Glu Asn Gly Met Ile Ser Val
       290                 295                 300
   Asn Glu Ser Cys Gln Thr Lys Glu Ser His Ile Tyr Ala Ile Gly Asp
   305                 310                 315                 320
   Val Ile Gly Gly Leu Gln Leu Ala His Val Ala Ser His Glu Gly Ile
                   325                 330                 335
   Ile Ala Val Glu His Phe Ala Gly Leu Asn Pro His Pro Leu Asp Pro
   340 345 350
   Thr Leu Val Pro Lys Cys Ile Tyr Ser Ser Pro Glu Ala Ala Ser Val
   355 360 365
   Gly Leu Thr Glu Asp Glu Ala Lys Ala Asn Gly His Asn Val Lys Ile
   370 375 380
   Gly Lys Phe Pro Phe Met Ala Ile Gly Lys Ala Leu Val Tyr Gly Glu
   385 390 395 400
   Ser Asp Gly Phe Val Lys Ile Val Ala Asp Arg Asp Thr Asp Asp Ile
   405 410 415
   Leu Gly Val His Met Ile Gly Pro His Val Thr Asp Met Ile Ser Glu
   420 425 430
   Ala Gly Leu Ala Lys Val Leu Asp Ala Thr Pro Trp Glu Val Gly Gln
   435 440 445
   Thr Ile His Pro His Pro Thr Leu Ser Glu Ala Ile Gly Glu Ala Ala
   450 455 460
   Leu Ala Ala Asp Gly Lys Ala Ile His Phe
   465 470
<210> 25
   <211> 330
   <212> PRT
   <213> Bacillus subtilis
<400> 25
   Met Ser Thr Asn Arg His Gln Ala Leu Gly Leu Thr Asp Gln Glu Ala
   1 5 10 15
   Val Asp Met Tyr Arg Thr Met Leu Leu Ala Arg Lys Ile Asp Glu Arg
   20 25 30
   Met Trp Leu Leu Asn Arg Ser Gly Lys Ile Pro Phe Val Ile Ser Cys
   35 40 45
   Gln Gly Gln Glu Ala Ala Gln Val Gly Ala Ala Phe Ala Leu Asp Arg
   50 55 60
   Glu Met Asp Tyr Val Leu Pro Tyr Tyr Arg Asp Met Gly Val Val Leu
   65 70 75 80
   Ala Phe Gly Met Thr Ala Lys Asp Leu Met Met Ser Gly Phe Ala Lys
   85 90 95
   Ala Ala Asp Pro Asn Ser Gly Gly Arg Gln Met Pro Gly His Phe Gly
   100 105 110
   Gln Lys Lys Asn Arg Ile Val Thr Gly Ser Ser Pro Val Thr Thr Gln
   115 120 125
   Val Pro His Ala Val Gly Ile Ala Leu Ala Gly Arg Met Glu Lys Lys
   130 135 140
   Asp Ile Ala Ala Phe Val Thr Phe Gly Glu Gly Ser Ser Asn Gln Gly
   145 150 155 160
   Asp Phe His Glu Gly Ala Asn Phe Ala Ala Val His Lys Leu Pro Val
   165 170 175
   Ile Phe Met Cys Glu Asn Asn Lys Tyr Ala Ile Ser Val Pro Tyr Asp
   180 185 190
   Lys Gln Val Ala Cys Glu Asn Ile Ser Asp Arg Ala Ile Gly Tyr Gly
   195 200 205
   Met Pro Gly Val Thr Val Asn Gly Asn Asp Pro Leu Glu Val Tyr Gln
   210 215 220
   Ala Val Lys Glu Ala Arg Glu Arg Ala Arg Arg Gly Glu Gly Pro Thr
   225 230 235 240
   Leu Ile Glu Thr Ile Ser Tyr Arg Leu Thr Pro His Ser Ser Asp Asp
   245 250 255
   Asp Asp Ser Ser Tyr Arg Gly Arg Glu Glu Val Glu Glu Ala Lys Lys
   260 265 270
   Ser Asp Pro Leu Leu Thr Tyr Gln Ala Tyr Leu Lys Glu Thr Gly Leu
   275 280 285
   Leu Ser Asp Glu Ile Glu Gln Thr Met Leu Asp Glu Ile Met Ala Ile
   290 295 300
   Val Asn Glu Ala Thr Asp Glu Ala Glu Asn Ala Pro Tyr Ala Ala Pro
   305 310 315 320
   Glu Ser Ala Leu Asp Tyr Val Tyr Ala Lys
   325 330
<210> 26
   <211> 327
   <212> PRT
   <213> Bacillus subtilis
<400> 26
   Met Ser Val Met Ser Tyr Ile Asp Ala Ile Asn Leu Ala Met Lys Glu
   1               5                   10                  15
   Glu Met Glu Arg Asp Ser Arg Val Phe Val Leu Gly Glu Asp Val Gly
               20                  25                  30
   Arg Lys Gly Gly Val Phe Lys Ala Thr Ala Gly Leu Tyr Glu Gln Phe
           35                  40                  45
   Gly Glu Glu Arg Val Met Asp Thr Pro Leu Ala Glu Ser Ala Ile Ala
       50                  55                  60
   Gly Val Gly Ile Gly Ala Ala Met Tyr Gly Met Arg Pro Ile Ala Glu
   65                  70                  75                  80
   Met Gln Phe Ala Asp Phe Ile Met Pro Ala Val Asn Gln Ile Ile Ser
                   85                  90                  95
   Glu Ala Ala Lys Ile Arg Tyr Arg Ser Asn Asn Asp Trp Ser Cys Pro
               100                 105                 110
   Ile Val Val Arg Ala Pro Tyr Gly Gly Gly Val His Gly Ala Leu Tyr
           115                 120                 125
   His Ser Gln Ser Val Glu Ala Ile Phe Ala Asn Gln Pro Gly Leu Lys
       130                 135                 140
   Ile Val Met Pro Ser Thr Pro Tyr Asp Ala Lys Gly Leu Leu Lys Ala
   145                 150                 155                 160
   Ala Val Arg Asp Glu Asp Pro Val Leu Phe Phe Glu His Lys Arg Ala
                   165                 170                 175
   Tyr Arg Leu Ile Lys Gly Glu Val Pro Ala Asp Asp Tyr Val Leu Pro
               180                 185                 190
   Ile Gly Lys Ala Asp Val Lys Arg Glu Gly Asp Asp Ile Thr Val Ile
           195                 200                 205
   Thr Tyr Gly Leu Cys Val His Phe Ala Leu Gln Ala Ala Glu Arg Leu
       210                 215                 220
   Glu Lys Asp Gly Ile Ser Ala His Val Val Asp Leu Arg Thr Val Tyr
   225                 230                 235                 240
   Pro Leu Asp Lys Glu Ala Ile Ile Glu Ala Ala Ser Lys Thr Gly Lys
                   245                 250                 255
   Val Leu Leu Val Thr Glu Asp Thr Lys Glu Gly Ser Ile Met Ser Glu
               260                 265                 270
   Val Ala Ala Ile Ile Ser Glu His Cys Leu Phe Asp Leu Asp Ala Pro
           275                 280                 285
   Ile Lys Arg Leu Ala Gly Pro Asp Ile Pro Ala Met Pro Tyr Ala Pro
       290                 295                 300
   Thr Met Glu Lys Tyr Phe Met Val Asn Pro Asp Lys Val Glu Ala Ala
   305                 310                 315                 320
   Met Arg Glu Leu Ala Glu Phe
                   325
<210> 27
   <211> 424
   <212> PRT
   <213> Bacillus subtilis
<400> 27
   Met Ala Ile Glu Gln Met Thr Met Pro Gln Leu Gly Glu Ser Val Thr
   1               5                   10                  15
   Glu Gly Thr Ile Ser Lys Trp Leu Val Ala Pro Gly Asp Lys Val Asn
               20                  25                  30
   Lys Tyr Asp Pro Ile Ala Glu Val Met Thr Asp Lys Val Asn Ala Glu
           35                  40                  45
   Val Pro Ser Ser Phe Thr Gly Thr Ile Thr Glu Leu Val Gly Glu Glu
       50                  55                  60
   Gly Gln Thr Leu Gln Val Gly Glu Met Ile Cys Lys Ile Glu Thr Glu
   65                  70                  75                  80
   Gly Ala Asn Pro Ala Glu Gln Lys Gln Glu Gln Pro Ala Ala Ser Glu
                   85                  90                  95
   Ala Ala Glu Asn Pro Val Ala Lys Ser Ala Gly Ala Ala Asp Gln Pro
               100                 105                 110
   Asn Lys Lys Arg Tyr Ser Pro Ala Val Leu Arg Leu Ala Gly Glu His
           115                 120                 125
   Gly Ile Asp Leu Asp Gln Val Thr Gly Thr Gly Ala Gly Gly Arg Ile
       130                 135                 140
   Thr Arg Lys Asp Ile Gln Arg Leu Ile Glu Thr Gly Gly Val Gln Glu
   145                 150                 155                 160
   Gln Asn Pro Glu Glu Leu Lys Thr Ala Ala Pro Ala Pro Lys Ser Ala
                   165                 170                 175
   Ser Lys Pro Glu Pro Lys Glu Glu Thr Ser Tyr Pro Ala Ser Ala Ala
               180                 185                 190
   Gly Asp Lys Glu Ile Pro Val Thr Gly Val Arg Lys Ala Ile Ala Ser
           195                 200                 205
   Asn Met Lys Arg Ser Lys Thr Glu Ile Pro His Ala Trp Thr Met Met
       210                 215                 220
   Glu Val Asp Val Thr Asn Met Val Ala Tyr Arg Asn Ser Ile Lys Asp
   225                 230                 235                 240
   Ser Phe Lys Lys Thr Glu Gly Phe Asn Leu Thr Phe Phe Ala Phe Phe
                   245                 250                 255
   Val Lys Ala Val Ala Gln Ala Leu Lys Glu Phe Pro Gln Met Asn Ser
               260                 265                 270
   Met Trp Ala Gly Asp Lys Ile Ile Gln Lys Lys Asp Ile Asn Ile Ser
           275                 280                 285
   Ile Ala Val Ala Thr Glu Asp Ser Leu Phe Val Pro Val Ile Lys Asn
       290                 295                 300
   Ala Asp Glu Lys Thr Ile Lys Gly Ile Ala Lys Asp Ile Thr Gly Leu
   305                 310                 315                 320
   Ala Lys Lys Val Arg Asp Gly Lys Leu Thr Ala Asp Asp Met Gln Gly
                   325                 330                 335
   Gly Thr Phe Thr Val Asn Asn Thr Gly Ser Phe Gly Ser Val Gln Ser
               340                 345                 350
   Met Gly Ile Ile Asn Tyr Pro Gln Ala Ala Ile Leu Gln Val Glu Ser
           355                 360                 365
   Ile Val Lys Arg Pro Val Val Met Asp Asn Gly Met Ile Ala Val Arg
       370                 375                 380
   Asp Met Val Asn Leu Cys Leu Ser Leu Asp His Arg Val Leu Asp Gly
   385                 390                 395                 400
   Leu Val Cys Gly Arg Phe Leu Gly Arg Val Lys Gln Ile Leu Glu Ser
                   405                 410                 415
   Ile Asp Glu Lys Thr Ser Val Tyr
               420
<210> 28
   <211> 251
   <212> PRT
   <213> Bacillus subtilis
<400> 28
   Met Asp Lys Val Ile Ala Arg Pro Tyr Lys Arg Pro Asn Ala Leu Cys
   1               5                   10                  15
   Arg Leu Ile Cys Phe Pro Trp Ala Gly Gly Asn Cys Ser Phe Phe Ile
               20                  25                  30
   Arg Trp Cys Glu Ala Phe Ser Ser Ile Ile Val Val Ser Val Ile Arg
           35                  40                  45
   Leu Ala Gly Arg Glu Cys Arg Asp Thr Glu Pro Phe Pro Glu Asp Met
       50                  55                  60
   Ala Glu Val Val Asn Glu Ile Thr Asn Ala Leu Leu Lys Asp Leu Gln
   65                  70                  75                  80
   Glu Lys Pro Phe Ala Leu Phe Gly His Ser Phe Gly Ser Phe Val Ser
                   85                  90                  95
   Tyr Ala Leu Ala Val His Leu Lys Glu Lys His Gly Leu Glu Pro Val
               100                 105                 110
   His Met Phe Phe Ser Gly Ser Tyr Gly Pro His Ser Glu Tyr Phe His
           115                 120                 125
   Leu Met Tyr Lys Leu Pro Glu Val Glu Asp Ser Arg Leu Leu Glu Leu
       130                 135                 140
   Ile His Thr Leu Gly Gly Thr Pro Pro Glu Phe Leu Gln Asn Glu Gln
   145                 150                 155                 160
   Ile Thr Lys His Leu Leu Arg Val Leu Lys Glu Asp Gln Lys Val Leu
                   165                 170                 175
   Val Thr Tyr Pro Trp His Asp Val Arg Lys Lys Tyr Phe Ser Cys Asp
               180                 185                 190
   Leu Thr Cys Phe Asn Gly Ser Asp Glu Lys Asn His Gly Ser Glu Ala
           195                 200                 205
   Trp Ile Ala Ile Thr Ser Gly Asp Thr Ser Ile Tyr Ser Leu Pro Gly
       210                 215                 220
   Asn His Phe Tyr Leu Met Glu Pro Ser Asn Glu Thr Phe Leu Ile Lys
   225                 230                 235                 240
   Tyr Ile Thr Lys Cys Ile Glu Asn Ser Asp Ile
                   245                 250
<210> 29
   <211> 263
   <212> PRT
   <213> Bacillus subtilis
<400> 29
   Met Glu Thr Ala Val Asn Ala Lys Ser Pro Arg Asn Glu Lys Val Leu
   1               5                   10                  15
   Asn Cys Leu Tyr Gln Asn Pro Asp Ala Val Phe Lys Leu Ile Cys Phe
               20                  25                  30
   Pro Trp Ala Gly Gly Gly Ser Ile His Phe Ala Lys Trp Gly Gln Lys
           35                  40                  45
   Ile Asn Asp Ser Leu Glu Val His Ala Val Arg Leu Ala Gly Arg Glu
       50                  55                  60
   Thr Arg Leu Gly Glu Pro Phe Ala Asn Asp Ile Tyr Gln Ile Ala Asp
   65                  70                  75                  80
   Glu Ile Val Thr Ala Leu Leu Pro Ile Ile Gln Asp Lys Ala Phe Ala
                   85                  90                  95
   Phe Phe Gly His Ser Phe Gly Ser Tyr Ile Ala Leu Ile Thr Ala Leu
               100                 105                 110
   Leu Leu Lys Glu Lys Tyr Lys Met Glu Pro Leu His Ile Phe Val Ser
           115                 120                 125
   Gly Ala Ser Ala Pro His Ser Thr Ser Arg Pro Gln Val Pro Asp Leu
       130                 135                 140
   Asn Glu Leu Thr Glu Glu Gln Val Arg His His Leu Leu Asp Phe Gly
   145                 150                 155                 160
   Gly Thr Pro Lys His Leu Ile Glu Asp Gln Asp Val Leu Arg Met Phe
                   165                 170                 175
   Ile Pro Leu Leu Lys Ala Asp Ala Gly Val Val Lys Lys Phe Ile Phe
               180                 185                 190
   Asp Lys Pro Ser Lys Ala Leu Leu Ser Leu Asp Ile Thr Gly Phe Leu
           195                 200                 205
   Gly Ser Glu Asp Thr Ile Lys Asp Ile Glu Gly Trp Gln Asp Leu Thr
       210                 215                 220
   Ser Gly Lys Phe Asp Val His Met Leu Pro Gly Asp His Phe Tyr Leu
   225                 230                 235                 240
   Met Lys Pro Asp Asn Glu Asn Phe Ile Lys Asn Tyr Ile Ala Lys Cys
                   245                 250                 255
   Leu Glu Leu Ser Ser Leu Thr
               260
<210> 30
   <211> 37
   <212> DNA
   <213> Artificial Sequence
<220>
   <223> Synthetic primer
<400> 30
   gtgccatggc tcatattaca tacgatctgc cggttgc 37
<210> 31
   <211> 41
   <212> DNA
   <213> Artificial Sequence
<220>
   <223> Synthetic primer
<400> 31
   gatcgaattc atccttaggc gtcaacgaaa ccggtgattt g 41
<210> 32
   <211> 993
   <212> DNA
   <213> Artificial Sequence
<220>
   <223> Synthetic nucleotide
<400> 32
   atggctcata ttacatacga tctgccggtt gctattgatg acattattga agcgaaacaa      60
   cgactggctg ggcgaattta taaaacaggc atgcctcgct ccaactattt tagtgaacgt     120
   tgcaaaggtg aaatattcct gaagtttgaa aatatgcagc gtacgggttc atttaaaatt     180
   cgtggcgcat ttaataaatt aagttcactg accgatgcgg aaaaacgcaa aggcgtggtg     240
   gcctgttctg cgggcaacca tgcgcaaggg gtttccctct cctgcgcgat gctgggtatc     300
   gacggtaaag tggtgatgcc aaaaggtgcg ccaaaatcca aagtagcggc aacgtgcgac     360
   tactccgcag aagtcgttct gcatggtgat aacttcaacg acactatcgc taaagtgagc     420
   gaaattgtcg aaatggaagg ccgtattttt atcccacctt acgatgatcc gaaagtgatt     480
   gctggccagg gaacgattgg tctggaaatt atggaagatc tctatgatgt cgataacgtg     540
   attgtgccaa ttggtggtgg cggtttaatt gctggtattg cggtggcaat taaatctatt     600
   aacccgacca ttcgtgttat tggcgtacag tctgaaaacg ttcacggcat ggcggcttct     660
   ttccactccg gagaaataac cacgcaccga actaccggca ccctggcgga tggttgtgat     720
   gtctcccgcc cgggtaattt aacttacgaa atcgttcgtg aattagtcga tgacatcgtg     780
   ctggtcagcg aagacgaaat cagaaacagt atgattgcct taattcagcg caataaagtc     840
   gtcaccgaag gcgcaggcgc tctggcatgt gctgcattat taagcggtaa attagaccaa     900
   tatattcaaa acagaaaaac cgtcagtatt atttccggcg gcaatatcga tctttctcgc     960
   gtctctcaaa tcaccggttt cgttgacgcc taa                                  993
<210> 33
   <211> 330
   <212> PRT
   <213> Artificial Sequence
<220>
   <223> Synthetic polynucleotide
<400> 33
   Met Ala His Ile Thr Tyr Asp Leu Pro Val Ala Ile Asp Asp Ile Ile
   1               5                   10                  15
   Glu Ala Lys Gln Arg Leu Ala Gly Arg Ile Tyr Lys Thr Gly Met Pro
               20                  25                  30
   Arg Ser Asn Tyr Phe Ser Glu Arg Cys Lys Gly Glu Ile Phe Leu Lys
           35                  40                  45
   Phe Glu Asn Met Gln Arg Thr Gly Ser Phe Lys Ile Arg Gly Ala Phe
       50                  55                  60
   Asn Lys Leu Ser Ser Leu Thr Asp Ala Glu Lys Arg Lys Gly Val Val
   65                  70                  75                  80
   Ala Cys Ser Ala Gly Asn His Ala Gln Gly Val Ser Leu Ser Cys Ala
                   85                  90                  95
   Met Leu Gly Ile Asp Gly Lys Val Val Met Pro Lys Gly Ala Pro Lys
               100                 105                 110
   Ser Lys Val Ala Ala Thr Cys Asp Tyr Ser Ala Glu Val Val Leu His
           115                 120                 125
   Gly Asp Asn Phe Asn Asp Thr Ile Ala Lys Val Ser Glu Ile Val Glu
       130                 135                 140
   Met Glu Gly Arg Ile Phe Ile Pro Pro Tyr Asp Asp Pro Lys Val Ile
   145                 150                 155                 160
   Ala Gly Gln Gly Thr Ile Gly Leu Glu Ile Met Glu Asp Leu Tyr Asp
                   165                 170                 175
   Val Asp Asn Val Ile Val Pro Ile Gly Gly Gly Gly Leu Ile Ala Gly
               180                 185                 190
   Ile Ala Val Ala Ile Lys Ser Ile Asn Pro Thr Ile Arg Val Ile Gly
           195                 200                 205
   Val Gln Ser Glu Asn Val His Gly Met Ala Ala Ser Phe His Ser Gly
       210                 215                 220
   Glu Ile Thr Thr His Arg Thr Thr Gly Thr Leu Ala Asp Gly Cys Asp
   225                 230                 235                 240
   Val Ser Arg Pro Gly Asn Leu Thr Tyr Glu Ile Val Arg Glu Leu Val
                   245                 250                 255
   Asp Asp Ile Val Leu Val Ser Glu Asp Glu Ile Arg Asn Ser Met Ile
               260                 265                 270
   Ala Leu Ile Gln Arg Asn Lys Val Val Thr Glu Gly Ala Gly Ala Leu
           275                 280                 285
   Ala Cys Ala Ala Leu Leu Ser Gly Lys Leu Asp Gln Tyr Ile Gln Asn
       290                 295                 300
   Arg Lys Thr Val Ser Ile Ile Ser Gly Gly Asn Ile Asp Leu Ser Arg
   305                 310                 315                 320
   Val Ser Gln Ile Thr Gly Phe Val Asp Ala
                   325                 330
<210> 34
   <211> 1725
   <212> DNA
   <213> Escherichia coli
<400> 34
   atggagatgt tgtctggagc cgagatggtc gtccgatcgc ttatcgatca gggcgttaaa        60
   caagtattcg gttatcccgg aggcgcagtc cttgatattt atgatgcatt gcataccgtg       120
   ggtggtattg atcatgtatt agttcgtcat gagcaggcgg cggtgcatat ggccgatggc       180
   ctggcgcgcg cgaccgggga agtcggcgtc gtgctggtaa cgtcgggtcc aggggcgacc       240
   aatgcgatta ctggcatcgc caccgcttat atggattcca ttccattagt tgtcctttcc       300
   gggcaggtag cgacctcgtt gataggttac gatgcctttc aggagtgcga catggtgggg       360
   atttcgcgac cggtggttaa acacagtttt ctggttaagc aaacggaaga cattccgcag       420
   gtgctgaaaa aggctttctg gctggcggca agtggtcgcc caggaccagt agtcgttgat       480
   ttaccgaaag atattcttaa tccggcgaac aaattaccct atgtctggcc ggagtcggtc       540
   agtatgcgtt cttacaatcc cactactacc ggacataaag ggcaaattaa gcgtgctctg       600
   caaacgctgg tagcggcaaa aaaaccggtt gtctacgtag gcggtggggc aatcacggcg       660
   ggctgccatc agcagttgaa agaaacggtg gaggcgttga atctgcccgt tgtttgctca       720
   ttgatggggc tgggggcgtt tccggcaacg catcgtcagg cactgggcat gctgggaatg       780
   cacggtacct acgaagccaa tatgacgatg cataacgcgg atgtgatttt cgccgtcggg       840
   gtacgatttg atgaccgaac gacgaacaat ctggcaaagt actgcccaaa tgccactgtt       900
   ctgcatatcg atattgatcc tacttccatt tctaaaaccg tgactgcgga tatcccgatt       960
   gtgggggatg ctcgccaggt cctcgaacaa atgcttgaac tcttgtcgca agaatccgcc      1020
   catcaaccac tggatgagat ccgcgactgg tggcagcaaa ttgaacagtg gcgcgctcgt      1080
   cagtgcctga aatatgacac tcacagtgaa aagattaaac cgcaggcggt gatcgagact      1140
   ctttggcggt tgacgaaggg agacgcttac gtgacgtccg atgtcgggca gcaccagatg      1200
   tttgctgcac tttattatcc attcgacaaa ccgcgtcgct ggatcaattc cggtggcctc      1260
   ggcacgatgg gttttggttt acctgcggca ctgggcgtca aaatggcgtt gccagaagaa      1320
   accgtggttt gcgtcactgg cgacggcagt attcagatga acatccagga actgtctacc      1380
   gcgttgcaat acgagttgcc cgtactggtg gtgaatctca ataaccgcta tctggggatg      1440
   gtgaagcagt ggcaggacat gatctattcc ggccgtcatt cacaatctta tatgcaatcg      1500
   ctacccgatt tcgtccgtct ggcggaagcc tatgggcatg tcgggatcca gatttctcat      1560
   ccgcatgagc tggaaagcaa acttagcgag gcgctggaac aggtgcgcaa taatcgcctg      1620
   gtgtttgttg atgttaccgt cgatggcagc gagcacgtct acccgatgca gattcgcggg      1680
   ggcggaatgg atgaaatgtg gttaagcaaa acggagagaa cctga                      1725
<210> 35
   <211> 492
   <212> DNA
   <213> Escherichia coli
<400> 35
   atgcgccgga tattatcagt cttactcgaa aatgaatcag gcgcgttatc ccgcgtgatt     60
   ggcctttttt cccagcgtgg ctacaacatt gaaagcctga ccgttgcgcc aaccgacgat    120
   ccgacattat cgcgtatgac catccagacc gtgggcgatg aaaaagtact tgagcagatc    180
   gaaaagcaat tacacaaact ggtcgatgtc ttgcgcgtga gtgagttggg gcagggcgcg    240
   catgttgagc gggaaatcat gctggtgaaa attcaggcca gcggttacgg gcgtgacgaa    300
   gtgaaacgta atacggaaat attccgtggg caaattatcg atgtcacacc ctcgctttat    360
   accgttcaat tagcaggcac cagcggtaag cttgatgcat ttttagcatc gattcgcgat     420
   gtggcgaaaa ttgtggaggt tgctcgctct ggtgtggtcg gactttcgcg cggcgataaa    480
   ataatgcgtt ga                                                        492
<210> 36
   <211> 2242
   <212> DNA
   <213> Escherichia coli
<400> 36
   gaattcgagg aggcaggcca tggagatgtt gtctggagcc gagatggtcg tccgatcgct      60
   tatcgatcag ggcgttaaac aagtattcgg ttatcccgga ggcgcagtcc ttgatattta     120
   tgatgcattg cataccgtgg gtggtattga tcatgtatta gttcgtcatg agcaggcggc     180
   ggtgcatatg gccgatggcc tggcgcgcgc gaccggggaa gtcggcgtcg tgctggtaac     240
   gtcgggtcca ggggcgacca atgcgattac tggcatcgcc accgcttata tggattccat     300
   tccattagtt gtcctttccg ggcaggtagc gacctcgttg ataggttacg atgcctttca     360
   ggagtgcgac atggtgggga tttcgcgacc ggtggttaaa cacagttttc tggttaagca     420
   aacggaagac attccgcagg tgctgaaaaa ggctttctgg ctggcggcaa gtggtcgccc     480
   aggaccagta gtcgttgatt taccgaaaga tattcttaat ccggcgaaca aattacccta     540
   tgtctggccg gagtcggtca gtatgcgttc ttacaatccc actactaccg gacataaagg     600
   gcaaattaag cgtgctctgc aaacgctggt agcggcaaaa aaaccggttg tctacgtagg     660
   cggtggggca atcacggcgg gctgccatca gcagttgaaa gaaacggtgg aggcgttgaa     720
   tctgcccgtt gtttgctcat tgatggggct gggggcgttt ccggcaacgc atcgtcaggc     780
   actgggcatg ctgggaatgc acggtaccta cgaagccaat atgacgatgc ataacgcgga     840
   tgtgattttc gccgtcgggg tacgatttga tgaccgaacg acgaacaatc tggcaaagta     900
   ctgcccaaat gccactgttc tgcatatcga tattgatcct acttccattt ctaaaaccgt     960
   gactgcggat atcccgattg tgggggatgc tcgccaggtc ctcgaacaaa tgcttgaact    1020
   cttgtcgcaa gaatccgccc atcaaccact ggatgagatc cgcgactggt ggcagcaaat    1080
   tgaacagtgg cgcgctcgtc agtgcctgaa atatgacact cacagtgaaa agattaaacc    1140
   gcaggcggtg atcgagactc tttggcggtt gacgaaggga gacgcttacg tgacgtccga    1200
   tgtcgggcag caccagatgt ttgctgcact ttattatcca ttcgacaaac cgcgtcgctg    1260
   gatcaattcc ggtggcctcg gcacgatggg ttttggttta cctgcggcac tgggcgtcaa      1320
   aatggcgttg ccagaagaaa ccgtggtttg cgtcactggc gacggcagta ttcagatgaa      1380
   catccaggaa ctgtctaccg cgttgcaata cgagttgccc gtactggtgg tgaatctcaa      1440
   taaccgctat ctggggatgg tgaagcagtg gcaggacatg atctattccg gccgtcattc      1500
   acaatcttat atgcaatcgc tacccgattt cgtccgtctg gcggaagcct atgggcatgt      1560
   cgggatccag atttctcatc cgcatgagct ggaaagcaaa cttagcgagg cgctggaaca      1620
   ggtgcgcaat aatcgcctgg tgtttgttga tgttaccgtc gatggcagcg agcacgtcta      1680
   cccgatgcag attcgcgggg gcggaatgga tgaaatgtgg ttaagcaaaa cggagagaac      1740
   ctgattatgc gccggatatt atcagtctta ctcgaaaatg aatcaggcgc gttatcccgc      1800
   gtgattggcc ttttttccca gcgtggctac aacattgaaa gcctgaccgt tgcgccaacc      1860
   gacgatccga cattatcgcg tatgaccatc cagaccgtgg gcgatgaaaa agtacttgag      1920
   cagatcgaaa agcaattaca caaactggtc gatgtcttgc gcgtgagtga gttggggcag      1980
   ggcgcgcatg ttgagcggga aatcatgctg gtgaaaattc aggccagcgg ttacgggcgt      2040
   gacgaagtga aacgtaatac ggaaatattc cgtgggcaaa ttatcgatgt cacaccctcg      2100
   ctttataccg ttcaattagc aggcaccagc ggtaagcttg atgcattttt agcatcgatt      2160
   cgcgatgtgg cgaaaattgt ggaggttgct cgctctggtg tggtcggact ttcgcgcggc      2220
   gataaaataa tgcgttgaat tc                                               2242
<210> 37
   <211> 38
   <212> DNA
   <213> Artificial Sequence
<220>
   <223> Synthetic primer
<400> 37
   gatgaattcg aggaggcagg ccatggagat gttgtctg 38
<210> 38
   <211> 31
   <212> DNA
   <213> Artificial Sequence
<220>
   <223> Synthetic primer
<400> 38
   ttgagaattc aacgcattat tttatcgccg c 31
<210> 39
   <211> 574
   <212> PRT
   <213> Escherichia coli
<400> 39
   Met Glu Met Leu Ser Gly Ala Glu Met Val Val Arg Ser Leu Ile Asp
   1               5                   10                  15
   Gln Gly Val Lys Gln Val Phe Gly Tyr Pro Gly Gly Ala Val Leu Asp
               20                  25                  30
   Ile Tyr Asp Ala Leu His Thr Val Gly Gly Ile Asp His Val Leu Val
           35                  40                  45
   Arg His Glu Gln Ala Ala Val His Met Ala Asp Gly Leu Ala Arg Ala
       50                  55                  60
   Thr Gly Glu Val Gly Val Val Leu Val Thr Ser Gly Pro Gly Ala Thr
   65                  70                  75                  80
   Asn Ala Ile Thr Gly Ile Ala Thr Ala Tyr Met Asp Ser Ile Pro Leu
                   85                  90                  95
   Val Val Leu Ser Gly Gln Val Ala Thr Ser Leu Ile Gly Tyr Asp Ala
               100                 105                 110
   Phe Gln Glu Cys Asp Met Val Gly Ile Ser Arg Pro Val Val Lys His
           115                 120                 125
   Ser Phe Leu Val Lys Gln Thr Glu Asp Ile Pro Gln Val Leu Lys Lys
       130                 135                 140
   Ala Phe Trp Leu Ala Ala Ser Gly Arg Pro Gly Pro Val Val Val Asp
   145                 150                 155                 160
   Leu Pro Lys Asp Ile Leu Asn Pro Ala Asn Lys Leu Pro Tyr Val Trp
                   165                 170                 175
   Pro Glu Ser Val Ser Met Arg Ser Tyr Asn Pro Thr Thr Thr Gly His
               180                 185                 190
   Lys Gly Gln Ile Lys Arg Ala Leu Gln Thr Leu Val Ala Ala Lys Lys
           195                 200                 205
   Pro Val Val Tyr Val Gly Gly Gly Ala Ile Thr Ala Gly Cys His Gln
       210                 215                 220
   Gln Leu Lys Glu Thr Val Glu Ala Leu Asn Leu Pro Val Val Cys Ser
   225                 230                 235                 240
   Leu Met Gly Leu Gly Ala Phe Pro Ala Thr His Arg Gln Ala Leu Gly
                   245                 250                 255
   Met Leu Gly Met His Gly Thr Tyr Glu Ala Asn Met Thr Met His Asn
               260                 265                 270
   Ala Asp Val Ile Phe Ala Val Gly Val Arg Phe Asp Asp Arg Thr Thr
           275                 280                 285
   Asn Asn Leu Ala Lys Tyr Cys Pro Asn Ala Thr Val Leu His Ile Asp
       290                 295                 300
   Ile Asp Pro Thr Ser Ile Ser Lys Thr Val Thr Ala Asp Ile Pro Ile
   305                 310                 315                 320
   Val Gly Asp Ala Arg Gln Val Leu Glu Gln Met Leu Glu Leu Leu Ser
                   325                 330                 335
   Gln Glu Ser Ala His Gln Pro Leu Asp Glu Ile Arg Asp Trp Trp Gln
               340                 345                 350
   Gln Ile Glu Gln Trp Arg Ala Arg Gln Cys Leu Lys Tyr Asp Thr His
           355                 360                 365
   Ser Glu Lys Ile Lys Pro Gln Ala Val Ile Glu Thr Leu Trp Arg Leu
       370                 375                 380
   Thr Lys Gly Asp Ala Tyr Val Thr Ser Asp Val Gly Gln His Gln Met
   385                 390                 395                 400
   Phe Ala Ala Leu Tyr Tyr Pro Phe Asp Lys Pro Arg Arg Trp Ile Asn
                   405                 410                 415
   Ser Gly Gly Leu Gly Thr Met Gly Phe Gly Leu Pro Ala Ala Leu Gly
               420                 425                 430
   Val Lys Met Ala Leu Pro Glu Glu Thr Val Val Cys Val Thr Gly Asp
           435                 440                 445
   Gly Ser Ile Gln Met Asn Ile Gln Glu Leu Ser Thr Ala Leu Gln Tyr
       450                 455                 460
   Glu Leu Pro Val Leu Val Val Asn Leu Asn Asn Arg Tyr Leu Gly Met
   465                 470                 475                 480
   Val Lys Gln Trp Gln Asp Met Ile Tyr Ser Gly Arg His Ser Gln Ser
                   485                 490                 495
   Tyr Met Gln Ser Leu Pro Asp Phe Val Arg Leu Ala Glu Ala Tyr Gly
               500                 505                 510
   His Val Gly Ile Gln Ile Ser His Pro His Glu Leu Glu Ser Lys Leu
           515                 520                 525
   Ser Glu Ala Leu Glu Gln Val Arg Asn Asn Arg Leu Val Phe Val Asp
       530                 535                 540
   Val Thr Val Asp Gly Ser Glu His Val Tyr Pro Met Gln Ile Arg Gly
   545                 550                 555                 560
   Gly Gly Met Asp Glu Met Trp Leu Ser Lys Thr Glu Arg Thr
                   565                 570
<210> 40
   <211> 163
   <212> PRT
   <213> Escherichia coli
<400> 40
   Met Arg Arg Ile Leu Ser Val Leu Leu Glu Asn Glu Ser Gly Ala Leu
   1               5                   10                  15
   Ser Arg Val Ile Gly Leu Phe Ser Gln Arg Gly Tyr Asn Ile Glu Ser
               20                  25                  30
   Leu Thr Val Ala Pro Thr Asp Asp Pro Thr Leu Ser Arg Met Thr Ile
           35                  40                  45
   Gln Thr Val Gly Asp Glu Lys Val Leu Glu Gln Ile Glu Lys Gln Leu
       50                  55                  60
   His Lys Leu Val Asp Val Leu Arg Val Ser Glu Leu Gly Gln Gly Ala
   65                  70                  75                  80
   His Val Glu Arg Glu Ile Met Leu Val Lys Ile Gln Ala Ser Gly Tyr
                   85                  90                  95
   Gly Arg Asp Glu Val Lys Arg Asn Thr Glu Ile Phe Arg Gly Gln Ile
               100                 105                 110
   Ile Asp Val Thr Pro Ser Leu Tyr Thr Val Gln Leu Ala Gly Thr Ser
           115                 120                 125
   Gly Lys Leu Asp Ala Phe Leu Ala Ser Ile Arg Asp Val Ala Lys Ile
       130                 135                 140
   Val Glu Val Ala Arg Ser Gly Val Val Gly Leu Ser Arg Gly Asp Lys
   145                 150                 155                 160
   Ile Met Arg
<210> 41
   <211> 163
   <212> PRT
   <213> Artificial Sequence
<220>
   <223> Synthetic polypeptide
<400> 41
   Met Arg Arg Ile Leu Ser Val Leu Leu Glu Asn Glu Ser Asp Ala Leu
   1               5                   10                  15
   Ser Arg Val Ile Gly Leu Phe Ser Gln Arg Gly Tyr Asn Ile Glu Ser
               20                  25                  30
   Leu Thr Val Ala Pro Thr Asp Asp Pro Thr Leu Ser Arg Met Thr Ile
           35                  40                  45
   Gln Thr Val Gly Asp Glu Lys Val Leu Glu Gln Ile Glu Lys Gln Leu
       50                  55                  60
   His Lys Leu Val Asp Val Leu Arg Val Ser Glu Leu Gly Gln Gly Ala
   65                  70                  75                  80
   His Val Glu Arg Glu Ile Met Leu Val Lys Ile Gln Ala Ser Gly Tyr
                   85                  90                  95
   Gly Arg Asp Glu Val Lys Arg Asn Thr Glu Ile Phe Arg Gly Gln Ile
               100                 105                 110
   Ile Asp Val Thr Pro Ser Leu Tyr Thr Val Gln Leu Ala Gly Thr Ser
           115                 120                 125
   Gly Lys Leu Asp Ala Phe Leu Ala Ser Ile Arg Asp Val Ala Lys Ile
       130                 135                 140
   Val Glu Val Ala Arg Ser Gly Val Val Gly Leu Ser Arg Gly Asp Lys
   145                 150                 155                 160
   Ile Met Arg
<210> 42
   <211> 1058
   <212> DNA
   <213> Escherichia coli
<400> 42
   gacgtccgat gtcgggcagc accagatgtt tgctgcactt tattatccat tcgacaaacc      60
   gcgtcgctgg atcaattccg gtggcctcgg cacgatgggt tttggtttac ctgcggcact     120
   gggcgtcaaa atggcgttgc cagaagaaac cgtggtttgc gtcactggcg acggcagtat 180
   tcagatgaac atccaggaac tgtctaccgc gttgcaatac gagttgcccg tactggtggt 240
   gaatctcaat aaccgctatc tggggatggt gaagcagtgg caggacatga tctattccgg 300
   ccgtcattca caatcttata tgcaatcgct acccgatttc gtccgtctgg cggaagccta 360
   tgggcatgtc gggatccaga tttctcatcc gcatgagctg gaaagcaaac ttagcgaggc 420
   gctggaacag gtgcgcaata atcgcctggt gtttgttgat gttaccgtcg atggcagcga 480
   gcacgtctac ccgatgcaga ttcgcggggg cggaatggat gaaatgtggt taagcaaaac 540
   ggagagaacc tgattatgcg ccggatatta tcagtcttac tcgaaaatga atcagacgcg 600
   ttatcccgcg tgattggcct tttttcccag cgtggctaca acattgaaag cctgaccgtt 660
   gcgccaaccg acgatccgac atatcgcgta tgaccatcca gaccgtgggc gatgaaaaag 720
   tacttgagca gatcgaaaag caattacaca aactggtcga tgtcttgcgc gtgagtgagt 780
   tggggcaggg cgcgcatgtt gagcgggaaa tcatgctggt gaaaattcag gccagcggtt 840
   acgggcgtga cgaagtgaaa cgtaatacgg aaatattccg tgggcaaatt atcgatgtca 900
   caccctcgct ttataccgtt caattagcag gcaccagcgg taagcttgat gcatttttag 960
   catcgattcg cgatgtggcg aaaattgtgg aggttgctcg ctctggtgtg gtcggacttt 1020
   cgcgcggcga taaaataatg cgttgaattc gatctaga 1058
<210> 43
   <211> 1058
   <212> DNA
   <213> Escherichia coli
<400> 43
   gacgtccgat gtcgggcagc accagatgtt tgctgcactt tattatccat tcgacaaacc      60
   gcgtcgctgg atcaattccg gtggcctcgg cacgatgggt tttggtttac ctgcggcact     120
   gggcgtcaaa atggcgttgc cagaagaaac cgtggtttgc gtcactggcg acggcagtat     180
   tcagatgaac atccaggaac tgtctaccgc gttgcaatac gagttgcccg tactggtggt     240
   gaatctcaat aaccgctatc tggggatggt gaagcagtgg caggacatga tctattccgg     300
   ccgtcattca caatcttata tgcaatcgct acccgatttc gtccgtctgg cggaagccta     360
   tgggcatgtc gggatccaga tttctcatcc gcatgagctg gaaagcaaac ttagcgaggc     420
   gctggaacag gtgcgcaata atcgcctggt gtttgttgat gttaccgtcg atggcagcga     480
   gcacgtctac ccgatgcaga ttcgcggggg cggaatggat gaaatgtggt taagcaaaac     540
   ggagagaacc tgattatgcg ccggatatta tcagtcttac tcgaaaatga atcaggcgcg     600
   ttatcccgcg tgattggcct tttttcccag cgtggctaca acattgaaag cctgaccgtt     660
   gcgccaaccg acgatccgac atatcgcgta tgaccatcca gaccgtgggc gatgaaaaag     720
   tacttgagca gatcgaaaag caattacaca aactggtcga tgtcttgcgc gtgagtgagt     780
   tggggcaggg cgcgcatgtt gagcgggaaa tcatgctggt gaaaattcag gccagcggtt     840
   acgggcgtga cgaagtgaaa cgtaatacgg aaatattccg tgggcaaatt atcgatgtca     900
   caccctcgct ttataccgtt caattagcag gcaccagcgg taagcttgat gcatttttag     960
   catcgattcg cgatgtggcg aaaattgtgg aggttgctcg ctctggtgtg gtcggacttt    1020
   cgcgcggcga taaaataatg cgttgaattc gatctaga                            1058
<210> 44
   <211> 1647
   <212> DNA
   <213> Escherichia coli
<400> 44
   atgaatggcg cacagtgggt ggtacatgcg ttgcgggcac agggtgtgaa caccgttttc      60
   ggttatccgg gtggcgcaat tatgccggtt tacgatgcat tgtatgacgg cggcgtggag     120
   cacttgctat gccgacatga gcagggtgcg gcaatggcgg ctatcggtta tgctcgtgct     180
   accggcaaaa ctggcgtatg tatcgccacg tctggtccgg gcgcaaccaa cctgataacc     240
   gggcttgcgg acgcactgtt agattccatc cctgttgttg ccatcaccgg tcaagtgtcc     300
   gcaccgttta tcggcactga cgcatttcag gaagtggatg tcctgggatt gtcgttagcc     360
   tgtaccaagc acagctttct ggtgcagtcg ctggaagagt tgccgcgcat catggctgaa     420
   gcattcgacg ttgcctgctc aggtcgtcct ggtccggttc tggtcgatat cccaaaagat     480
   atccagttag ccagcggtga cctggaaccg tggttcacca ccgttgaaaa cgaagtgact     540
   ttcccacatg ccgaagttga gcaagcgcgc cagatgctgg caaaagcgca aaaaccgatg     600
   ctgtacgttg gcggtggcgt gggtatggcg caggcagttc cggctttgcg tgaatttctc     660
   gctgccacaa aaatgcctgc cacctgtacg ctgaaagggc tgggcgcagt agaagcagat     720
   tatccgtact atctgggcat gctgggaatg catggcacca aagcggcgaa cttcgcggtg     780
   caggagtgcg acttgctgat cgccgtgggt gcacgttttg atgaccgggt gaccggcaaa     840
   ctgaacacct tcgcaccaca cgccagtgtt atccatatgg atatcgaccc ggcagaaatg     900
   aacaagctgc gtcaggcaca tgtggcatta caaggtgatt taaatgctct gttaccagca     960
   ttacagcagc cgttaaatat caatgactgg cagctacact gcgcgcagct gcgtgatgaa    1020
   catgcctggc gttacgacca tcccggtgac gctatctacg cgccgttgtt gttaaaacaa    1080
   ctgtcagatc gtaaacctgc ggattgcgtc gtgaccacag atgtggggca gcaccagatg    1140
   tgggctgcgc agcacatcgc ccacactcgc ccggaaaatt tcatcacctc cagcggctta    1200
   ggcaccatgg gttttggttt accggcggcg gttggcgcgc aagtcgcgcg accaaacgat    1260
   accgtcgtct gtatctccgg tgacggctct ttcatgatga atgtgcaaga gctgggcacc    1320
   gtaaaacgca agcagttacc gttgaaaatc gtcttactcg ataaccaacg gttagggatg    1380
   gttcgacaat ggcagcaact gtttttccag gaacgatata gcgaaaccac ccttaccgat    1440
   aaccccgatt tcctcatgtt agccagcgcc ttcggcatcc ctggccaaca catcacccgt    1500
   aaagaccagg ttgaagcggc actcgacacc atgctgaaca gtgatgggcc atacctgctt    1560
   catgtctcaa tcgacgaact tgagaacgtc tggccgctgg tgccgcctgg tgccagtaat    1620
   tcagaaatgt tggagaaatt atcatga                                        1647
<210> 45
   <211> 261
   <212> DNA
   <213> Escherichia coli
<400> 45
   atgcaacatc aggtcaatgt atcagctcgc ttcaatccgg aaaccttaga acgtgtttta 60
   cgcgtggtgc gtcatcgtgg tttccacgtc tgctcaatga atatggccgc cgccagcgat 120
   gcacaaaata taaatatcga attgaccgtt gccagcccac ggtcggtcga cttactgttt 180
   agtcagttaa ataaactggt ggacgtcgca cacgttgcca tctgccagag cacaaccaca 240
   tcacaacaaa tccgcgcctg a 261
<210> 46
   <211> 1938
   <212> DNA
   <213> Escherichia coli
<400> 46
   gcggccgcag gacggggaac taactatgaa tggcgcacag tgggtggtac atgcgttgcg 60
   ggcacagggt gtgaacaccg ttttcggtta tccgggtggc gcaattatgc cggtttacga 120
   tgcattgtat gacggcggcg tggagcactt gctatgccga catgagcagg gtgcggcaat 180
   ggcggctatc ggttatgctc gtgctaccgg caaaactggc gtatgtatcg ccacgtctgg 240
   tccgggcgca accaacctga taaccgggct tgcggacgca ctgttagatt ccatccctgt 300
   tgttgccatc accggtcaag tgtccgcacc gtttatcggc actgacgcat ttcaggaagt 360
   ggatgtcctg ggattgtcgt tagcctgtac caagcacagc tttctggtgc agtcgctgga 420
   agagttgccg cgcatcatgg ctgaagcatt cgacgttgcc tgctcaggtc gtcctggtcc 480
   ggttctggtc gatatcccaa aagatatcca gttagccagc ggtgacctgg aaccgtggtt 540
   caccaccgtt gaaaacgaag tgactttccc acatgccgaa gttgagcaag cgcgccagat 600
   gctggcaaaa gcgcaaaaac cgatgctgta cgttggcggt ggcgtgggta tggcgcaggc 660
   agttccggct ttgcgtgaat ttctcgctgc cacaaaaatg cctgccacct gtacgctgaa 720
   agggctgggc gcagtagaag cagattatcc gtactatctg ggcatgctgg gaatgcatgg 780
   caccaaagcg gcgaacttcg cggtgcagga gtgcgacttg ctgatcgccg tgggtgcacg 840
   ttttgatgac cgggtgaccg gcaaactgaa caccttcgca ccacacgcca gtgttatcca 900
   tatggatatc gacccggcag aaatgaacaa gctgcgtcag gcacatgtgg cattacaagg 960
   tgatttaaat gctctgttac cagcattaca gcagccgtta aatatcaatg actggcagct 1020
   acactgcgcg cagctgcgtg atgaacatgc ctggcgttac gaccatcccg gtgacgctat 1080
   ctacgcgccg ttgttgttaa aacaactgtc agatcgtaaa cctgcggatt gcgtcgtgac 1140
   cacagatgtg gggcagcacc agatgtgggc tgcgcagcac atcgcccaca ctcgcccgga 1200
   aaatttcatc acctccagcg gcttaggcac catgggtttt ggtttaccgg cggcggttgg 1260
   cgcgcaagtc gcgcgaccaa acgataccgt cgtctgtatc tccggtgacg gctctttcat 1320
   gatgaatgtg caagagctgg gcaccgtaaa acgcaagcag ttaccgttga aaatcgtctt 1380
   actcgataac caacggttag ggatggttcg acaatggcag caactgtttt tccaggaacg 1440
   atatagcgaa accaccctta ccgataaccc cgatttcctc atgttagcca gcgccttcgg 1500
   catccctggc caacacatca cccgtaaaga ccaggttgaa gcggcactcg acaccatgct 1560
   gaacagtgat gggccatacc tgcttcatgt ctcaatcgac gaacttgaga acgtctggcc 1620
   gctggtgccg cctggtgcca gtaattcaga aatgttggag aaattatcat gatgcaacat 1680
   caggtcaatg tatcagctcg cttcaatccg gaaaccttag aacgtgtttt acgcgtggtg 1740
   cgtcatcgtg gtttccacgt ctgctcaatg aatatggccg ccgccagcga tgcacaaaat 1800
   ataaatatcg aattgaccgt tgccagccca cggtcggtcg acttactgtt tagtcagtta 1860
   aataaactgg tggacgtcgc acacgttgcc atctgccaga gcacaaccac atcacaacaa 1920
   atccgcgcct gagaattc 1938
<210> 47
   <211> 548
   <212> PRT
   <213> Escherichia coli
<400> 47
   Met Asn Gly Ala Gln Trp Val Val His Ala Leu Arg Ala Gln Gly Val
   1               5                   10                  15
   Asn Thr Val Phe Gly Tyr Pro Gly Gly Ala Ile Met Pro Val Tyr Asp
               20                  25                  30
   Ala Leu Tyr Asp Gly Gly Val Glu His Leu Leu Cys Arg His Glu Gln
           35                  40                  45
   Gly Ala Ala Met Ala Ala Ile Gly Tyr Ala Arg Ala Thr Gly Lys Thr
       50                  55                  60
   Gly Val Cys Ile Ala Thr Ser Gly Pro Gly Ala Thr Asn Leu Ile Thr
   65                  70                  75                  80
   Gly Leu Ala Asp Ala Leu Leu Asp Ser Ile Pro Val Val Ala Ile Thr
                   85                  90                  95
   Gly Gln Val Ser Ala Pro Phe Ile Gly Thr Asp Ala Phe Gln Glu Val
   100 105 110
   Asp Val Leu Gly Leu Ser Leu Ala Cys Thr Lys His Ser Phe Leu Val
   115 120 125
   Gln Ser Leu Glu Glu Leu Pro Arg Ile Met Ala Glu Ala Phe Asp Val
   130 135 140
   Ala Cys Ser Gly Arg Pro Gly Pro Val Leu Val Asp Ile Pro Lys Asp
   145 150 155 160
   Ile Gln Leu Ala Ser Gly Asp Leu Glu Pro Trp Phe Thr Thr Val Glu
   165 170 175
   Asn Glu Val Thr Phe Pro His Ala Glu Val Glu Gln Ala Arg Gln Met
   180 185 190
   Leu Ala Lys Ala Gln Lys Pro Met Leu Tyr Val Gly Gly Gly Val Gly
   195 200 205
   Met Ala Gln Ala Val Pro Ala Leu Arg Glu Phe Leu Ala Ala Thr Lys
   210 215 220
   Met Pro Ala Thr Cys Thr Leu Lys Gly Leu Gly Ala Val Glu Ala Asp
   225 230 235 240
   Tyr Pro Tyr Tyr Leu Gly Met Leu Gly Met His Gly Thr Lys Ala Ala
   245 250 255
   Asn Phe Ala Val Gln Glu Cys Asp Leu Leu Ile Ala Val Gly Ala Arg
   260 265 270
   Phe Asp Asp Arg Val Thr Gly Lys Leu Asn Thr Phe Ala Pro His Ala
   275 280 285
   Ser Val Ile His Met Asp Ile Asp Pro Ala Glu Met Asn Lys Leu Arg
   290 295 300
   Gln Ala His Val Ala Leu Gln Gly Asp Leu Asn Ala Leu Leu Pro Ala
   305 310 315 320
   Leu Gln Gln Pro Leu Asn Ile Asn Asp Trp Gln Leu His Cys Ala Gln
   325 330 335
   Leu Arg Asp Glu His Ala Trp Arg Tyr Asp His Pro Gly Asp Ala Ile
   340 345 350
   Tyr Ala Pro Leu Leu Leu Lys Gln Leu Ser Asp Arg Lys Pro Ala Asp
   355 360 365
   Cys Val Val Thr Thr Asp Val Gly Gln His Gln Met Trp Ala Ala Gln
   370 375 380
   His Ile Ala His Thr Arg Pro Glu Asn Phe Ile Thr Ser Ser Gly Leu
   385 390 395 400
   Gly Thr Met Gly Phe Gly Leu Pro Ala Ala Val Gly Ala Gln Val Ala
   405 410 415
   Arg Pro Asn Asp Thr Val Val Cys Ile Ser Gly Asp Gly Ser Phe Met
   420 425 430
   Met Asn Val Gln Glu Leu Gly Thr Val Lys Arg Lys Gln Leu Pro Leu
   435 440 445
   Lys Ile Val Leu Leu Asp Asn Gln Arg Leu Gly Met Val Arg Gln Trp
   450 455 460
   Gln Gln Leu Phe Phe Gln Glu Arg Tyr Ser Glu Thr Thr Leu Thr Asp
   465 470 475 480
   Asn Pro Asp Phe Leu Met Leu Ala Ser Ala Phe Gly Ile Pro Gly Gln
   485 490 495
   His Ile Thr Arg Lys Asp Gln Val Glu Ala Ala Leu Asp Thr Met Leu
   500 505 510
   Asn Ser Asp Gly Pro Tyr Leu Leu His Val Ser Ile Asp Glu Leu Glu
   515 520 525
   Asn Val Trp Pro Leu Val Pro Pro Gly Ala Ser Asn Ser Glu Met Leu
   530 535 540
   Glu Lys Leu Ser
   545
<210> 48
   <211> 87
   <212> PRT
   <213> Escherichia coli
<400> 48
   Met Met Gln His Gln Val Asn Val Ser Ala Arg Phe Asn Pro Glu Thr
   1               5                   10                  15
   Leu Glu Arg Val Leu Arg Val Val Arg His Arg Gly Phe His Val Cys
               20                  25                  30
   Ser Met Asn Met Ala Ala Ala Ser Asp Ala Gln Asn Ile Asn Ile Glu
           35                  40                  45
   Leu Thr Val Ala Ser Pro Arg Ser Val Asp Leu Leu Phe Ser Gln Leu
       50                  55                  60
   Asn Lys Leu Val Asp Val Ala His Val Ala Ile Cys Gln Ser Thr Thr
   65                  70                  75                  80
   Thr Ser Gln Gln Ile Arg Ala
                   85
<210> 49
   <211> 1725
   <212> DNA
   <213> Bacillus subtilis
<400> 49
<210> 50
   <211> 519
   <212> DNA
   <213> Bacillus subtilis
<400> 50
   ttgaaaagaa ttatcacatt gactgtggtg aaccgctccg gggtgttaaa ccggatcacc      60
   ggtctattca caaaaaggca ttacaacatt gaaagcatta cagttggaca cacagaaaca     120
   gccggcgttt ccagaatcac cttcgtcgtt catgttgaag gtgaaaatga tgttgaacag     180
   ttaacgaaac agctcaacaa acagattgat gtgctgaaag tcacagacat cacaaatcaa     240
   tcgattgtcc agagggagct ggccttaatc aaggttgtct ccgcaccttc aacaagaaca     300
   gagattaatg gaatcataga accgtttaga gcctctgtcg ttgatgtcag cagagacagc     360
   atcgttgttc aggtgacagg tgaatctaac aaaattgaag cgcttattga gttattaaaa     420
   ccttatggca ttaaagaaat cgcgagaaca ggtacaacgg cttttgcgag gggaacccag     480
   caaaaggcgt catccaataa aacaatatct attgtataa                            519
<210> 51
   <211> 2270
   <212> DNA
   <213> Bacillus subtilis
<400> 51
   gcggccgcaa aaggaggaac taaaatgggg actaatgtac aggtggattc agcatctgcc      60
   gaatgtacac agacgatgag cggagcatta atgctgattg aatcattaaa aaaagagaaa     120
   gtagaaatga tcttcggtta tccgggcggg gctgtgcttc cgatttacga taagctatac     180
   aattcagggt tggtacatat ccttccccgt cacgaacaag gagcaattca tgcagcggag     240
   ggatacgcaa gggtctccgg aaaaccgggt gtcgtcattg ccacgtcagg gccgggagcg     300
   acaaaccttg ttacaggcct tgctgatgcc atgattgatt cattgccgtt agtcgtcttt     360
   acagggcagg tagcaacctc tgtaatcggg agcgatgcat ttcaggaagc agacatttta     420
   gggattacga tgccagtaac aaaacacagc taccaggttc gccagccgga agatctgccg     480
   cgcatcatta aagaagcgtt ccatattgca acaactggaa gacccggacc tgtattgatt      540
   gatattccga aagatgtagc aacaattgaa ggagagttca gctacgatca tgagatgaat      600
   ctcccgggat accagccgac aacagagccg aattatttgc agatccgcaa gcttgtggaa      660
   gccgtgagca gtgcgaaaaa accggtgatc ctggcgggtg cgggcgtact gcacggaaaa      720
   gcgtcagaag aattaaaaaa ttatgctgaa cagcagcaaa tccctgtggc acacaccctt      780
   ttggggctcg gaggcttccc ggctgaccat ccgcttttcc tagggatggc gggaatgcac      840
   ggtacttata cagccaatat ggcccttcat gaatgtgatc tattaatcag tatcggcgcc      900
   cgttttgatg accgtgtcac aggaaacctg aaacactttg ccagaaacgc aaagatagcc      960
   cacatcgata ttgatccagc tgaaatcgga aaaatcatga aaacacagat tcctgtagtc     1020
   ggagacagca aaattgtcct gcaggagctg atcaaacaag acggcaaaca aagcgattca     1080
   agcgaatgga aaaaacagct cgcagaatgg aaagaagagt atccgctctg gtatgtagat     1140
   aatgaagaag aaggttttaa acctcagaaa ttgattgaat atattcatca atttacaaaa     1200
   ggagaggcca ttgtcgcaac ggatgtaggc cagcatcaaa tgtggtcagc gcaattttat     1260
   ccgttccaaa aagcagataa atgggtcacg tcaggcggac ttggaacgat gggattcggt     1320
   cttccggcgg cgatcggcgc acagctggcc gaaaaagatg ctactgttgt cgcggttgtc     1380
   ggagacggcg gattccaaat gacgcttcaa gaactcgatg ttattcgcga attaaatctt     1440
   ccggtcaagg tagtgatttt aaataacgct tgtctcggaa tggtcagaca gtggcaggaa     1500
   attttctatg aagaacgtta ttcagaatct aaattcgctt ctcagcctga cttcgtcaaa     1560
   ttgtccgaag catacggcat taaaggcatc agaatttcat cagaagcgga agcaaaggaa     1620
   aagctggaag aggcattaac atcaagagaa cctgttgtca ttgacgtgcg ggttgccagc     1680
   gaagaaaaag tattcccgat ggtggctccg gggaaagggc tgcatgaaat ggtgggggtg     1740
   aaaccttgaa aagaattatc acattgactg tggtgaaccg ctccggggtg ttaaaccgga     1800
   tcaccggtct attcacaaaa aggcattaca acattgaaag cattacagtt ggacacacag     1860
   aaacagccgg cgtttccaga atcaccttcg tcgttcatgt tgaaggtgaa aatgatgttg     1920
   aacagttaac gaaacagctc aacaaacaga ttgatgtgct gaaagtcaca gacatcacaa     1980
   atcaatcgat tgtccagagg gagctggcct taatcaaggt tgtctccgca ccttcaacaa     2040
   gaacagagat taatggaatc atagaaccgt ttagagcctc tgtcgttgat gtcagcagag     2100
   acagcatcgt tgttcaggtg acaggtgaat ctaacaaaat tgaagcgctt attgagttat     2160
   taaaacctta tggcattaaa gaaatcgcga gaacaggtac aacggctttt gcgaggggaa     2220
   cccagcaaaa ggcgtcatcc aataaaacaa tatctattgt ataagaattc                2270
<210> 52
   <211> 574
   <212> PRT
   <213> Bacillus subtilis
<400> 52
   Met Gly Thr Asn Val Gln Val Asp Ser Ala Ser Ala Glu Cys Thr Gln
   1               5                   10                  15
   Thr Met Ser Gly Ala Leu Met Leu Ile Glu Ser Leu Lys Lys Glu Lys
               20                  25                  30
   Val Glu Met Ile Phe Gly Tyr Pro Gly Gly Ala Val Leu Pro Ile Tyr
           35                  40                  45
   Asp Lys Leu Tyr Asn Ser Gly Leu Val His Ile Leu Pro Arg His Glu
       50                  55                  60
   Gln Gly Ala Ile His Ala Ala Glu Gly Tyr Ala Arg Val Ser Gly Lys
   65                  70                  75                  80
   Pro Gly Val Val Ile Ala Thr Ser Gly Pro Gly Ala Thr Asn Leu Val
                   85                  90                  95
   Thr Gly Leu Ala Asp Ala Met Ile Asp Ser Leu Pro Leu Val Val Phe
               100                 105                 110
   Thr Gly Gln Val Ala Thr Ser Val Ile Gly Ser Asp Ala Phe Gln Glu
           115                 120                 125
   Ala Asp Ile Leu Gly Ile Thr Met Pro Val Thr Lys His Ser Tyr Gln
       130                 135                 140
   Val Arg Gln Pro Glu Asp Leu Pro Arg Ile Ile Lys Glu Ala Phe His
   145                 150                 155                 160
   Ile Ala Thr Thr Gly Arg Pro Gly Pro Val Leu Ile Asp Ile Pro Lys
                   165                 170                 175
   Asp Val Ala Thr Ile Glu Gly Glu Phe Ser Tyr Asp His Glu Met Asn
               180                 185                 190
   Leu Pro Gly Tyr Gln Pro Thr Thr Glu Pro Asn Tyr Leu Gln Ile Arg
           195                 200                 205
   Lys Leu Val Glu Ala Val Ser Ser Ala Lys Lys Pro Val Ile Leu Ala
       210                 215                 220
   Gly Ala Gly Val Leu His Gly Lys Ala Ser Glu Glu Leu Lys Asn Tyr
   225                 230                 235                 240
   Ala Glu Gln Gln Gln Ile Pro Val Ala His Thr Leu Leu Gly Leu Gly
                   245                 250                 255
   Gly Phe Pro Ala Asp His Pro Leu Phe Leu Gly Met Ala Gly Met His
               260                 265                 270
   Gly Thr Tyr Thr Ala Asn Met Ala Leu His Glu Cys Asp Leu Leu Ile
           275                 280                 285
   Ser Ile Gly Ala Arg Phe Asp Asp Arg Val Thr Gly Asn Leu Lys His
       290                 295                 300
   Phe Ala Arg Asn Ala Lys Ile Ala His Ile Asp Ile Asp Pro Ala Glu
   305                 310                 315                 320
   Ile Gly Lys Ile Met Lys Thr Gln Ile Pro Val Val Gly Asp Ser Lys
                   325                 330                 335
   Ile Val Leu Gln Glu Leu Ile Lys Gln Asp Gly Lys Gln Ser Asp Ser
               340                 345                 350
   Ser Glu Trp Lys Lys Gln Leu Ala Glu Trp Lys Glu Glu Tyr Pro Leu
           355                 360                 365
   Trp Tyr Val Asp Asn Glu Glu Glu Gly Phe Lys Pro Gln Lys Leu Ile
       370                 375                 380
   Glu Tyr Ile His Gln Phe Thr Lys Gly Glu Ala Ile Val Ala Thr Asp
   385                 390                 395                 400
   Val Gly Gln His Gln Met Trp Ser Ala Gln Phe Tyr Pro Phe Gln Lys
                   405                 410                 415
   Ala Asp Lys Trp Val Thr Ser Gly Gly Leu Gly Thr Met Gly Phe Gly
               420                 425                 430
   Leu Pro Ala Ala Ile Gly Ala Gln Leu Ala Glu Lys Asp Ala Thr Val
           435                 440                 445
   Val Ala Val Val Gly Asp Gly Gly Phe Gln Met Thr Leu Gln Glu Leu
       450                 455                 460
   Asp Val Ile Arg Glu Leu Asn Leu Pro Val Lys Val Val Ile Leu Asn
   465                 470                 475                 480
   Asn Ala Cys Leu Gly Met Val Arg Gln Trp Gln Glu Ile Phe Tyr Glu
                   485                 490                 495
   Glu Arg Tyr Ser Glu Ser Lys Phe Ala Ser Gln Pro Asp Phe Val Lys
               500                 505                 510
   Leu Ser Glu Ala Tyr Gly Ile Lys Gly Ile Arg Ile Ser Ser Glu Ala
           515                 520                 525
   Glu Ala Lys Glu Lys Leu Glu Glu Ala Leu Thr Ser Arg Glu Pro Val
       530                 535                 540
   Val Ile Asp Val Arg Val Ala Ser Glu Glu Lys Val Phe Pro Met Val
   545                 550                 555                 560
   Ala Pro Gly Lys Gly Leu His Glu Met Val Gly Val Lys Pro
                   565                 570
<210> 53
   <211> 172
   <212> PRT
   <213> Bacillus subtilis
<400> 53
   Met Lys Arg Ile Ile Thr Leu Thr Val Val Asn Arg Ser Gly Val Leu
   1               5                   10                  15
   Asn Arg Ile Thr Gly Leu Phe Thr Lys Arg His Tyr Asn Ile Glu Ser
               20                  25                  30
   Ile Thr Val Gly His Thr Glu Thr Ala Gly Val Ser Arg Ile Thr Phe
           35                  40                  45
   Val Val His Val Glu Gly Glu Asn Asp Val Glu Gln Leu Thr Lys Gln
       50                  55                  60
   Leu Asn Lys Gln Ile Asp Val Leu Lys Val Thr Asp Ile Thr Asn Gln
   65                  70                  75                  80
   Ser Ile Val Gln Arg Glu Leu Ala Leu Ile Lys Val Val Ser Ala Pro
                   85                  90                  95
   Ser Thr Arg Thr Glu Ile Asn Gly Ile Ile Glu Pro Phe Arg Ala Ser
               100                 105                 110
   Val Val Asp Val Ser Arg Asp Ser Ile Val Val Gln Val Thr Gly Glu
           115                 120                 125
   Ser Asn Lys Ile Glu Ala Leu Ile Glu Leu Leu Lys Pro Tyr Gly Ile
       130                 135                 140
   Lys Glu Ile Ala Arg Thr Gly Thr Thr Ala Phe Ala Arg Gly Thr Gln
   145                 150                 155                 160
   Gln Lys Ala Ser Ser Asn Lys Thr Ile Ser Ile Val
                       165                 170
<210> 54
   <211> 2393
   <212> DNA
   <213> Artificial Sequence
<220>
   <223> Synthetic nucleotide
<400> 54
   atgcataatg tgcctgtcaa atggacgaag cagggattct gcaaacccta tgctactccg 60
   tcaagccgtc aattgtctga ttcgttacca attatgacaa cttgacggct acatcattca 120
   ctttttcttc acaaccggca cggaactcgc tcgggctggc cccggtgcat tttttaaata 180
   cccgcgagaa atagagttga tcgtcaaaac caacattgcg accgacggtg gcgataggca 240
   tccgggtggt gctcaaaagc agcttcgcct ggctgatacg ttggtcctcg cgccagctta 300
   agacgctaat ccctaactgc tggcggaaaa gatgtgacag acgcgacggc gacaagcaaa 360
   catgctgtgc gacgctggcg atatcaaaat tgctgtctgc caggtgatcg ctgatgtact 420
   gacaagcctc gcgtacccga ttatccatcg gtggatggag cgactcgtta atcgcttcca 480
   tgcgccgcag taacaattgc tcaagcagat ttatcgccag cagctccgaa tagcgccctt 540
   ccccttgccc ggcgttaatg atttgcccaa acaggtcgct gaaatgcggc tggtgcgctt 600
   catccgggcg aaagaacccc gtattggcaa atattgacgg ccagttaagc cattcatgcc 660
   agtaggcgcg cggacgaaag taaacccact ggtgatacca ttcgcgagcc tccggatgac 720
   gaccgtagtg atgaatctct cctggcggga acagcaaaat atcacccggt cggcaaacaa 780
   attctcgtcc ctgatttttc accaccccct gaccgcgaat ggtgagattg agaatataac 840
   ctttcattcc cagcggtcgg tcgataaaaa aatcgagata accgttggcc tcaatcggcg 900
   ttaaacccgc caccagatgg gcattaaacg agtatcccgg cagcagggga tcattttgcg 960
   cttcagccat acttttcata ctcccgccat tcagagaaga aaccaattgt ccatattgca 1020
   tcagacattg ccgtcactgc gtcttttact ggctcttctc gctaaccaaa ccggtaaccc 1080
   cgcttattaa aagcattctg taacaaagcg ggaccaaagc catgacaaaa acgcgtaaca 1140
   aaagtgtcta taatcacggc agaaaagtcc acattgatta tttgcacggc gtcacacttt 1200
   gctatgccat agcattttta tccataagat tagcggatcc tacctgacgc tttttatcgc 1260
   aactctctac tgtttctcca tacccgtttt ttgggctaac aggaggaatt aaccatgggg 1320
   ggttctcatc atcatcatca tcatggtatg gctagcatga ctggtggaca gcaaatgggt 1380
   cgggatctgt acgacgatga cgataaggat cgatggggat ccgagctcga gatgaaagct 1440
   ggaatacttg gtgttggacg ttacattcct gagaaggttt taacaaatca tgatcttgaa 1500
   aaaatggttg aaacttctga cgagtggatt cgtacaagaa caggaataga agaaagaaga 1560
   atcgcagcag atgatgtgtt ttcatcacat atggctgttg cagcagcgaa aaatgcgctg 1620
   gaacaagctg aagtggctgc tgaggatctg gatatgatct tggttgcaac tgttacacct 1680
   gatcagtcat tccctacggt ctcttgtatg attcaagaac aactcggcgc gaagaaagcg 1740
   tgtgctatgg atatcagcgc ggcttgtgcg ggcttcatgt acggggttgt aaccggtaaa 1800
   caatttattg aatccggaac ctacaagcat gttctagttg ttggtgtaga gaagctctca 1860
   agcattaccg actgggaaga ccgcaataca gccgttctgt ttggagacgg agcaggcgct 1920
   gcggtagtcg ggccagtcag tgatgacaga ggaatccttt catttgaact aggagccgac 1980
   ggcacaggcg gtcagcactt gtatctgaat gaaaaacgac atacaatcat gaatggacga 2040
   gaagttttca aatttgcagt ccgccaaatg ggagaatcat gcgtaaatgt cattgaaaaa 2100
   gccggacttt caaaagagga tgtcgacttt ttgattccgc atcaggcgaa catccgtatc 2160
   atggaagctg ctcgcgagcg tttagagctt cctgtcgaaa agatgtctaa aactgttcat 2220
   aaatatggaa atacttctgc cgcatccatt ccgatctctc ttgtagaaga attggaagcc 2280
   ggtaaaatca aagacggcga tgtggtcgtt atggtagggt tcggcggagg actaacatgg 2340
   ggcgccattg caatccgctg gggccgataa aaaaaggtga ggtgcacaca aga 2393
<210> 55
   <211> 48
   <212> DNA
   <213> Artificial Sequence
<220>
   <223> Synthetic primer
<400> 55
   cgtattttca tttcgacgcg tatgcataat gtgcctgtca aatggacg 48
<210> 56
   <211> 51
   <212> DNA
   <213> Artificial Sequence
<220>
   <223> Synthetic primer
<400> 56
   atttgatgcc tctagcacgc gttcttgtgt gcacctcacc tttttttatc g 51
<210> 57
   <211> 1209
   <212> DNA
   <213> Artificial Sequence
<220>
   <223> Synthetic primer
<400> 57
   tcatgatggt gcgcattttt gataccacgc tgcgtgacgg tgaacagacg ccgggcgtta 60
   gcctgacgcc gaacgataaa ctggaaattg ccaaaaaact ggatgaactg ggcgttgacg 120
   tcatcgaagc cggtagcgca gtgacctcta aaggcgaacg cgaaggtatt aaactgatca 180
   cgaaagaagg cctgaatgcc gaaatttgct ctttcgttcg tgcactgccg gtcgatattg 240
   acgcggccct ggaatgtgat gttgacagcg tccatctggt ggttccgacc tctccgatcc 300
   acatgaaata taaactgcgt aaaaccgaag atgaagtgct ggttacggct ctgaaagcgg 360
   ttgaatacgc caaagaacag ggtctgattg tcgaactgtc agccgaagat gcaacgcgct 420
   cggacgtgaa ctttctgatc aaactgttca atgaaggcga aaaagttggt gcagatcgtg 480
   tctgcgtgtg tgacaccgtt ggcgtcctga cgccgcagaa atcacaagaa ctgttcaaga 540
   aaattaccga aaacgtgaat ctgccggtgt cggttcattg ccacaacgat ttcggtatgg 600
   cgaccgcaaa tgcgtgcagc gcggtgctgg gcggtgcggt tcaatgtcat gtcacggtga 660
   acggcatcgg tgaacgcgct ggcaatgcga gtctggaaga agtcgtggca gcttccaaaa 720
   ttctgtatgg ttacgatacc aaaatcaaaa tggaaaaact gtacgaagtc agtcgcattg 780
   tgtcccgtct gatgaaactg ccggtcccgc cgaacaaagc tatcgtgggc gataatgctt 840
   ttgcgcatga agcgggcatt cacgtggacg gtctgatcaa aaacaccgaa acgtatgaac 900
   cgattaaacc ggaaatggtt ggcaatcgtc gccgtattat cctgggcaaa cactctggtc 960
   gtaaagcgct gaaatacaaa ctggatctga tgggtattaa cgttagtgac gaacaactga 1020
   acaaaatcta tgaacgtgtg aaagaatttg gcgatctggg taaatacatt agcgatgccg 1080
   acctgctggc aatcgtgcgt gaagttaccg gtaaactgtg atgtcgaaga attaccatat 1140
   tgccgtattg ccgggggacg gtattggtcc ggagcggccg cttaattaag tttaaactct 1200
   agagaattc 1209
<210> 58
   <211> 372
   <212> PRT
   <213> Artificial Sequence
<220>
   <223> Synthetic polypeptide
<400> 58
   Met Met Val Arg Ile Phe Asp Thr Thr Leu Arg Asp Gly Glu Gln Thr
   1               5                   10                  15
   Pro Gly Val Ser Leu Thr Pro Asn Asp Lys Leu Glu Ile Ala Lys Lys
               20                  25                  30
   Leu Asp Glu Leu Gly Val Asp Val Ile Glu Ala Gly Ser Ala Val Thr
           35                  40                  45
   Ser Lys Gly Glu Arg Glu Gly Ile Lys Leu Ile Thr Lys Glu Gly Leu
       50                  55                  60
   Asn Ala Glu Ile Cys Ser Phe Val Arg Ala Leu Pro Val Asp Ile Asp
   65                  70                  75                  80
   Ala Ala Leu Glu Cys Asp Val Asp Ser Val His Leu Val Val Pro Thr
                   85                  90                  95
   Ser Pro Ile His Met Lys Tyr Lys Leu Arg Lys Thr Glu Asp Glu Val
               100                 105                 110
   Leu Val Thr Ala Leu Lys Ala Val Glu Tyr Ala Lys Glu Gln Gly Leu
           115                 120                 125
   Ile Val Glu Leu Ser Ala Glu Asp Ala Thr Arg Ser Asp Val Asn Phe
       130                 135                 140
   Leu Ile Lys Leu Phe Asn Glu Gly Glu Lys Val Gly Ala Asp Arg Val
   145                 150                 155                 160
   Cys Val Cys Asp Thr Val Gly Val Leu Thr Pro Gln Lys Ser Gln Glu
                   165                 170                 175
   Leu Phe Lys Lys Ile Thr Glu Asn Val Asn Leu Pro Val Ser Val His
               180                 185                 190
   Cys His Asn Asp Phe Gly Met Ala Thr Ala Asn Ala Cys Ser Ala Val
           195                 200                 205
   Leu Gly Gly Ala Val Gln Cys His Val Thr Val Asn Gly Ile Gly Glu
       210                 215                 220
   Arg Ala Gly Asn Ala Ser Leu Glu Glu Val Val Ala Ala Ser Lys Ile
   225                 230                 235                 240
   Leu Tyr Gly Tyr Asp Thr Lys Ile Lys Met Glu Lys Leu Tyr Glu Val
                   245                 250                 255
   Ser Arg Ile Val Ser Arg Leu Met Lys Leu Pro Val Pro Pro Asn Lys
               260                 265                 270
   Ala Ile Val Gly Asp Asn Ala Phe Ala His Glu Ala Gly Ile His Val
           275                 280                 285
   Asp Gly Leu Ile Lys Asn Thr Glu Thr Tyr Glu Pro Ile Lys Pro Glu
       290                 295                 300
   Met Val Gly Asn Arg Arg Arg Ile Ile Leu Gly Lys His Ser Gly Arg
   305                 310                 315                 320
   Lys Ala Leu Lys Tyr Lys Leu Asp Leu Met Gly Ile Asn Val Ser Asp
                   325                 330                 335
   Glu Gln Leu Asn Lys Ile Tyr Glu Arg Val Lys Glu Phe Gly Asp Leu
               340                 345                 350
   Gly Lys Tyr Ile Ser Asp Ala Asp Leu Leu Ala Ile Val Arg Glu Val
           355                 360                 365
   Thr Gly Lys Leu
       370
<210> 59
   <211> 1092
   <212> DNA
   <213> Escherichia coli
<400> 59
   atgtcgaaga attaccatat tgccgtattg ccgggggacg gtattggtcc ggaagtgatg        60
   acccaggcgc tgaaagtgct ggatgccgtg cgcaaccgct ttgcgatgcg catcaccacc       120
   agccattacg atgtaggcgg cgcagccatt gataaccacg ggcaaccact gccgcctgcg       180
   acggttgaag gttgtgagca agccgatgcc gtgctgtttg gctcggtagg cggcccgaag       240
   tgggaacatt taccaccaga ccagcaacca gaacgcggcg cgctgctgcc tctgcgtaag       300
   cacttcaaat tattcagcaa cctgcgcccg gcaaaactgt atcaggggct ggaagcattc       360
   tgtccgctgc gtgcagacat tgccgcaaac ggcttcgaca tcctgtgtgt gcgcgaactg       420
   accggcggca tctatttcgg tcagccaaaa ggccgcgaag gtagcggaca atatgaaaaa       480
   gcctttgata ccgaggtgta tcaccgtttt gagatcgaac gtatcgcccg catcgcgttt       540
   gaatctgctc gcaagcgtcg ccacaaagtg acgtcgatcg ataaagccaa cgtgctgcaa       600
   tcctctattt tatggcggga gatcgttaac gagatcgcca cggaataccc ggatgtcgaa       660
   ctggcgcata tgtacatcga caacgccacc atgcagctga ttaaagatcc atcacagttt       720
   gacgttctgc tgtgctccaa cctgtttggc gacattctgt ctgacgagtg cgcaatgatc       780
   actggctcga tggggatgtt gccttccgcc agcctgaacg agcaaggttt tggactgtat       840
   gaaccggcgg gcggctcggc accagatatc gcaggcaaaa acatcgccaa cccgattgca       900
   caaatccttt cgctggcact gctgctgcgt tacagcctgg atgccgatga tgcggcttgc       960
   gccattgaac gcgccattaa ccgcgcatta gaagaaggca ttcgcaccgg ggatttagcc      1020
   cgtggcgctg ccgccgttag taccgatgaa atgggcgata tcattgcccg ctatgtagca      1080
   gaaggggtgt aa                                                          1092
<210> 60
   <211> 1401
   <212> DNA
   <213> Escherichia coli
<400> 60
   atggctaaga cgttatacga aaaattgttc gacgctcacg ttgtgtacga agccgaaaac      60
   gaaaccccac tgttatatat cgaccgccac ctggtgcatg aagtgacctc accgcaggcg     120
   ttcgatggtc tgcgcgccca cggtcgcccg gtacgtcagc cgggcaaaac cttcgctacc     180
   atggatcaca acgtctctac ccagaccaaa gacattaatg cctgcggtga aatggcgcgt     240
   atccagatgc aggaactgat caaaaactgc aaagaatttg gcgtcgaact gtatgacctg     300
   aatcacccgt atcaggggat cgtccacgta atggggccgg aacagggcgt caccttgccg     360
   gggatgacca ttgtctgcgg cgactcgcat accgccaccc acggcgcgtt tggcgcactg     420
   gcctttggta tcggcacttc cgaagttgaa cacgtactgg caacgcaaac cctgaaacag     480
   ggccgcgcaa aaaccatgaa aattgaagtc cagggcaaag ccgcgccggg cattaccgca     540
   aaagatatcg tgctggcaat tatcggtaaa accggtagcg caggcggcac cgggcatgtg     600
   gtggagtttt gcggcgaagc aatccgtgat ttaagcatgg aaggtcgtat gaccctgtgc     660
   aatatggcaa tcgaaatggg cgcaaaagcc ggtctggttg caccggacga aaccaccttt     720
   aactatgtca aaggccgtct gcatgcgccg aaaggcaaag atttcgacga cgccgttgcc     780
   tactggaaaa ccctgcaaac cgacgaaggc gcaactttcg ataccgttgt cactctgcaa     840
   gcagaagaaa tttcaccgca ggtcacctgg ggcaccaatc ccggccaggt gatttccgtg     900
   aacgacaata ttcccgatcc ggcttcgttt gccgatccgg ttgaacgcgc gtcggcagaa     960
   aaagcgctgg cctatatggg gctgaaaccg ggtattccgc tgaccgaagt ggctatcgac    1020
   aaagtgttta tcggttcctg taccaactcg cgcattgaag atttacgcgc ggcagcggag    1080
   atcgccaaag ggcgaaaagt cgcgccaggc gtgcaggcac tggtggttcc cggctctggc    1140
   ccggtaaaag cccaggcgga agcggaaggt ctggataaaa tctttattga agccggtttt    1200
   gaatggcgct tgcctggctg ctcaatgtgt ctggcgatga acaacgaccg tctgaatccg    1260
   ggcgaacgtt gtgcctccac cagcaaccgt aactttgaag gccgccaggg gcgcggcggg    1320
   cgcacgcatc tggtcagccc ggcaatggct gccgctgctg ctgtgaccgg acatttcgcc    1380
   gacattcgca acattaaata a                                              1401
<210> 61
   <211> 606
   <212> DNA
   <213> Escherichia coli
<400> 61
   atggcagaga aatttatcaa acacacaggc ctggtggttc cgctggatgc cgccaatgtc        60
   gataccgatg caatcatccc gaaacagttt ttgcagaaag tgacccgtac gggttttggc       120
   gcgcatctgt ttaacgactg gcgttttctg gatgaaaaag gccaacagcc aaacccggac       180
   ttcgtgctga acttcccgca gtatcagggc gcttccattt tgctggcacg agaaaacttc       240
   ggctgtggct cttcgcgtga gcacgcgccc tgggcattga ccgactacgg ttttaaagtg       300
   gtgattgcgc cgagttttgc tgacatcttc tacggcaata gctttaacaa ccagctgctg       360
   ccggtgaaat taagcgatgc agaagtggac gaactgtttg cgctggtgaa agctaatccg       420
   gggatccatt tcgacgtgga tctggaagcg caagaggtga aagcgggaga gaaaacctat       480
   cgctttacca tcgatgcctt ccgccgccac tgcatgatga acggtctgga cagtattggg       540
   cttaccttgc agcacgacga cgccattgcc gcttatgaag caaaacaacc tgcgtttatg       600
   aattaa                                                                 606
<210> 62
   <211> 3119
   <212> DNA
   <213> Escherichia coli
<400> 62
   atgtcgaaga attaccatat tgccgtattg ccgggggacg gtattggtcc ggaagtgatg      60
   acccaggcgc tgaaagtgct ggatgccgtg cgcaaccgct ttgcgatgcg catcaccacc     120
   agccattacg atgtaggcgg cgcagccatt gataaccacg ggcaaccact gccgcctgcg     180
   acggttgaag gttgtgagca agccgatgcc gtgctgtttg gctcggtagg cggcccgaag     240
   tgggaacatt taccaccaga ccagcaacca gaacgcggcg cgctgctgcc tctgcgtaag     300
   cacttcaaat tattcagcaa cctgcgcccg gcaaaactgt atcaggggct ggaagcattc     360
   tgtccgctgc gtgcagacat tgccgcaaac ggcttcgaca tcctgtgtgt gcgcgaactg     420
   accggcggca tctatttcgg tcagccaaaa ggccgcgaag gtagcggaca atatgaaaaa     480
   gcctttgata ccgaggtgta tcaccgtttt gagatcgaac gtatcgcccg catcgcgttt     540
   gaatctgctc gcaagcgtcg ccacaaagtg acgtcgatcg ataaagccaa cgtgctgcaa     600
   tcctctattt tatggcggga gatcgttaac gagatcgcca cggaataccc ggatgtcgaa     660
   ctggcgcata tgtacatcga caacgccacc atgcagctga ttaaagatcc atcacagttt     720
   gacgttctgc tgtgctccaa cctgtttggc gacattctgt ctgacgagtg cgcaatgatc     780
   actggctcga tggggatgtt gccttccgcc agcctgaacg agcaaggttt tggactgtat     840
   gaaccggcgg gcggctcggc accagatatc gcaggcaaaa acatcgccaa cccgattgca     900
   caaatccttt cgctggcact gctgctgcgt tacagcctgg atgccgatga tgcggcttgc     960
   gccattgaac gcgccattaa ccgcgcatta gaagaaggca ttcgcaccgg ggatttagcc    1020
   cgtggcgctg ccgccgttag taccgatgaa atgggcgata tcattgcccg ctatgtagca    1080
   gaaggggtgt aatcatggct aagacgttat acgaaaaatt gttcgacgct cacgttgtgt    1140
   acgaagccga aaacgaaacc ccactgttat atatcgaccg ccacctggtg catgaagtga    1200
   cctcaccgca ggcgttcgat ggtctgcgcg cccacggtcg cccggtacgt cagccgggca    1260
   aaaccttcgc taccatggat cacaacgtct ctacccagac caaagacatt aatgcctgcg    1320
   gtgaaatggc gcgtatccag atgcaggaac tgatcaaaaa ctgcaaagaa tttggcgtcg    1380
   aactgtatga cctgaatcac ccgtatcagg ggatcgtcca cgtaatgggg ccggaacagg 1440
   gcgtcacctt gccggggatg accattgtct gcggcgactc gcataccgcc acccacggcg 1500
   cgtttggcgc actggccttt ggtatcggca cttccgaagt tgaacacgta ctggcaacgc 1560
   aaaccctgaa acagggccgc gcaaaaacca tgaaaattga agtccagggc aaagccgcgc 1620
   cgggcattac cgcaaaagat atcgtgctgg caattatcgg taaaaccggt agcgcaggcg 1680
   gcaccgggca tgtggtggag ttttgcggcg aagcaatccg tgatttaagc atggaaggtc 1740
   gtatgaccct gtgcaatatg gcaatcgaaa tgggcgcaaa agccggtctg gttgcaccgg 1800
   acgaaaccac ctttaactat gtcaaaggcc gtctgcatgc gccgaaaggc aaagatttcg 1860
   acgacgccgt tgcctactgg aaaaccctgc aaaccgacga aggcgcaact ttcgataccg 1920
   ttgtcactct gcaagcagaa gaaatttcac cgcaggtcac ctggggcacc aatcccggcc 1980
   aggtgatttc cgtgaacgac aatattcccg atccggcttc gtttgccgat ccggttgaac 2040
   gcgcgtcggc agaaaaagcg ctggcctata tggggctgaa accgggtatt ccgctgaccg 2100
   aagtggctat cgacaaagtg tttatcggtt cctgtaccaa ctcgcgcatt gaagatttac 2160
   gcgcggcagc ggagatcgcc aaagggcgaa aagtcgcgcc aggcgtgcag gcactggtgg 2220
   ttcccggctc tggcccggta aaagcccagg cggaagcgga aggtctggat aaaatcttta 2280
   ttgaagccgg ttttgaatgg cgcttgcctg gctgctcaat gtgtctggcg atgaacaacg 2340
   accgtctgaa tccgggcgaa cgttgtgcct ccaccagcaa ccgtaacttt gaaggccgcc 2400
   aggggcgcgg cgggcgcacg catctggtca gcccggcaat ggctgccgct gctgctgtga 2460
   ccggacattt cgccgacatt cgcaacatta aataaggagc acaccatggc agagaaattt 2520
   atcaaacaca caggcctggt ggttccgctg gatgccgcca atgtcgatac cgatgcaatc 2580
   atcccgaaac agtttttgca gaaagtgacc cgtacgggtt ttggcgcgca tctgtttaac 2640
   gactggcgtt ttctggatga aaaaggccaa cagccaaacc cggacttcgt gctgaacttc 2700
   ccgcagtatc agggcgcttc cattttgctg gcacgagaaa acttcggctg tggctcttcg 2760
   cgtgagcacg cgccctgggc attgaccgac tacggtttta aagtggtgat tgcgccgagt 2820
   tttgctgaca tcttctacgg caatagcttt aacaaccagc tgctgccggt gaaattaagc 2880
   gatgcagaag tggacgaact gtttgcgctg gtgaaagcta atccggggat ccatttcgac 2940
   gtggatctgg aagcgcaaga ggtgaaagcg ggagagaaaa cctatcgctt taccatcgat 3000
   gccttccgcc gccactgcat gatgaacggt ctggacagta ttgggcttac cttgcagcac 3060
   gacgacgcca ttgccgctta tgaagcaaaa caacctgcgt ttatgaatta agcggccgc 3119
<210> 63
   <211> 25
   <212> DNA
   <213> Artificial Sequence
<220>
   <223> Synthetic primer
<400> 63
   ttggtccgga agtgatgacc caggc 25
<210> 64
   <211> 43
   <212> DNA
   <213> Artificial Sequence
<220>
   <223> Synthetic primer
<400> 64
   tatgtgcggc cgcttaattc ataaacgcag gttgttttgc ttc 43
<210> 65
   <211> 363
   <212> PRT
   <213> Escherichia coli
<400> 65
   Met Ser Lys Asn Tyr His Ile Ala Val Leu Pro Gly Asp Gly Ile Gly
   1 5 10 15
   Pro Glu Val Met Thr Gln Ala Leu Lys Val Leu Asp Ala Val Arg Asn
   20 25 30
   Arg Phe Ala Met Arg Ile Thr Thr Ser His Tyr Asp Val Gly Gly Ala
   35 40 45
   Ala Ile Asp Asn His Gly Gln Pro Leu Pro Pro Ala Thr Val Glu Gly
   50 55 60
   Cys Glu Gln Ala Asp Ala Val Leu Phe Gly Ser Val Gly Gly Pro Lys
   65 70 75 80
   Trp Glu His Leu Pro Pro Asp Gln Gln Pro Glu Arg Gly Ala Leu Leu
   85 90 95
   Pro Leu Arg Lys His Phe Lys Leu Phe Ser Asn Leu Arg Pro Ala Lys
   100 105 110
   Leu Tyr Gln Gly Leu Glu Ala Phe Cys Pro Leu Arg Ala Asp Ile Ala
   115 120 125
   Ala Asn Gly Phe Asp Ile Leu Cys Val Arg Glu Leu Thr Gly Gly Ile
   130 135 140
   Tyr Phe Gly Gln Pro Lys Gly Arg Glu Gly Ser Gly Gln Tyr Glu Lys
   145 150 155 160
   Ala Phe Asp Thr Glu Val Tyr His Arg Phe Glu Ile Glu Arg Ile Ala
   165 170 175
   Arg Ile Ala Phe Glu Ser Ala Arg Lys Arg Arg His Lys Val Thr Ser
   180 185 190
   Ile Asp Lys Ala Asn Val Leu Gln Ser Ser Ile Leu Trp Arg Glu Ile
   195 200 205
   Val Asn Glu Ile Ala Thr Glu Tyr Pro Asp Val Glu Leu Ala His Met
   210 215 220
   Tyr Ile Asp Asn Ala Thr Met Gln Leu Ile Lys Asp Pro Ser Gln Phe
   225 230 235 240
   Asp Val Leu Leu Cys Ser Asn Leu Phe Gly Asp Ile Leu Ser Asp Glu
   245 250 255
   Cys Ala Met Ile Thr Gly Ser Met Gly Met Leu Pro Ser Ala Ser Leu
   260 265 270
   Asn Glu Gln Gly Phe Gly Leu Tyr Glu Pro Ala Gly Gly Ser Ala Pro
   275 280 285
   Asp Ile Ala Gly Lys Asn Ile Ala Asn Pro Ile Ala Gln Ile Leu Ser
   290 295 300
   Leu Ala Leu Leu Leu Arg Tyr Ser Leu Asp Ala Asp Asp Ala Ala Cys
   305 310 315 320
   Ala Ile Glu Arg Ala Ile Asn Arg Ala Leu Glu Glu Gly Ile Arg Thr
   325 330 335
   Gly Asp Leu Ala Arg Gly Ala Ala Ala Val Ser Thr Asp Glu Met Gly
   340 345 350
   Asp Ile Ile Ala Arg Tyr Val Ala Glu Gly Val
   355 360
<210> 66
   <211> 466
   <212> PRT
   <213> Escherichia coli
<400> 66
   Met Ala Lys Thr Leu Tyr Glu Lys Leu Phe Asp Ala His Val Val Tyr
   1               5                   10                  15
   Glu Ala Glu Asn Glu Thr Pro Leu Leu Tyr Ile Asp Arg His Leu Val
   20 25 30
   His Glu Val Thr Ser Pro Gln Ala Phe Asp Gly Leu Arg Ala His Gly
   35 40 45
   Arg Pro Val Arg Gln Pro Gly Lys Thr Phe Ala Thr Met Asp His Asn
   50 55 60
   Val Ser Thr Gln Thr Lys Asp Ile Asn Ala Cys Gly Glu Met Ala Arg
   65 70 75 80
   Ile Gln Met Gln Glu Leu Ile Lys Asn Cys Lys Glu Phe Gly Val Glu
   85 90 95
   Leu Tyr Asp Leu Asn His Pro Tyr Gln Gly Ile Val His Val Met Gly
   100 105 110
   Pro Glu Gln Gly Val Thr Leu Pro Gly Met Thr Ile Val Cys Gly Asp
   115 120 125
   Ser His Thr Ala Thr His Gly Ala Phe Gly Ala Leu Ala Phe Gly Ile
   130 135 140
   Gly Thr Ser Glu Val Glu His Val Leu Ala Thr Gln Thr Leu Lys Gln
   145 150 155 160
   Gly Arg Ala Lys Thr Met Lys Ile Glu Val Gln Gly Lys Ala Ala Pro
   165 170 175
   Gly Ile Thr Ala Lys Asp Ile Val Leu Ala Ile Ile Gly Lys Thr Gly
   180 185 190
   Ser Ala Gly Gly Thr Gly His Val Val Glu Phe Cys Gly Glu Ala Ile
   195 200 205
   Arg Asp Leu Ser Met Glu Gly Arg Met Thr Leu Cys Asn Met Ala Ile
   210 215 220
   Glu Met Gly Ala Lys Ala Gly Leu Val Ala Pro Asp Glu Thr Thr Phe
   225 230 235 240
   Asn Tyr Val Lys Gly Arg Leu His Ala Pro Lys Gly Lys Asp Phe Asp
   245 250 255
   Asp Ala Val Ala Tyr Trp Lys Thr Leu Gln Thr Asp Glu Gly Ala Thr
   260 265 270
   Phe Asp Thr Val Val Thr Leu Gln Ala Glu Glu Ile Ser Pro Gln Val
   275 280 285
   Thr Trp Gly Thr Asn Pro Gly Gln Val Ile Ser Val Asn Asp Asn Ile
   290 295 300
   Pro Asp Pro Ala Ser Phe Ala Asp Pro Val Glu Arg Ala Ser Ala Glu
   305 310 315 320
   Lys Ala Leu Ala Tyr Met Gly Leu Lys Pro Gly Ile Pro Leu Thr Glu
   325 330 335
   Val Ala Ile Asp Lys Val Phe Ile Gly Ser Cys Thr Asn Ser Arg Ile
   340 345 350
   Glu Asp Leu Arg Ala Ala Ala Glu Ile Ala Lys Gly Arg Lys Val Ala
   355 360 365
   Pro Gly Val Gln Ala Leu Val Val Pro Gly Ser Gly Pro Val Lys Ala
   370 375 380
   Gln Ala Glu Ala Glu Gly Leu Asp Lys Ile Phe Ile Glu Ala Gly Phe
   385 390 395 400
   Glu Trp Arg Leu Pro Gly Cys Ser Met Cys Leu Ala Met Asn Asn Asp
   405 410 415
   Arg Leu Asn Pro Gly Glu Arg Cys Ala Ser Thr Ser Asn Arg Asn Phe
   420 425 430
   Glu Gly Arg Gln Gly Arg Gly Gly Arg Thr His Leu Val Ser Pro Ala
   435 440 445
   Met Ala Ala Ala Ala Ala Val Thr Gly His Phe Ala Asp Ile Arg Asn
   450 455 460
   Ile Lys
   465
<210> 67
   <211> 201
   <212> PRT
   <213> Escherichia coli
<400> 67
   Met Ala Glu Lys Phe Ile Lys His Thr Gly Leu Val Val Pro Leu Asp
   1 5 10 15
   Ala Ala Asn Val Asp Thr Asp Ala Ile Ile Pro Lys Gln Phe Leu Gln
   20 25 30
   Lys Val Thr Arg Thr Gly Phe Gly Ala His Leu Phe Asn Asp Trp Arg
   35 40 45
   Phe Leu Asp Glu Lys Gly Gln Gln Pro Asn Pro Asp Phe Val Leu Asn
   50 55 60
   Phe Pro Gln Tyr Gln Gly Ala Ser Ile Leu Leu Ala Arg Glu Asn Phe
   65 70 75 80
   Gly Cys Gly Ser Ser Arg Glu His Ala Pro Trp Ala Leu Thr Asp Tyr
   85 90 95
   Gly Phe Lys Val Val Ile Ala Pro Ser Phe Ala Asp Ile Phe Tyr Gly
   100 105 110
   Asn Ser Phe Asn Asn Gln Leu Leu Pro Val Lys Leu Ser Asp Ala Glu
   115 120 125
   Val Asp Glu Leu Phe Ala Leu Val Lys Ala Asn Pro Gly Ile His Phe
   130 135 140
   Asp Val Asp Leu Glu Ala Gln Glu Val Lys Ala Gly Glu Lys Thr Tyr
   145 150 155 160
   Arg Phe Thr Ile Asp Ala Phe Arg Arg His Cys Met Met Asn Gly Leu
   165 170 175
   Asp Ser Ile Gly Leu Thr Leu Gln His Asp Asp Ala Ile Ala Ala Tyr
   180 185 190
   Glu Ala Lys Gln Pro Ala Phe Met Asn
   195 200
<210> 68
   <211> 566
   <212> DNA
   <213> Escherichia coli
<400> 68
   catgccatgg cggacacgtt attgattctg ggtgatagcc tgagcgccgg gtatcgaatg 60
   tctgccagcg cggcctggcc tgccttgttg aatgataagt ggcagagtaa aacgtcggta 120
   gttaatgcca gcatcagcgg cgacacctcg caacaaggac tggcgcgcct tccggctctg 180
   ctgaaacagc atcagccgcg ttgggtgctg gttgaactgg gcggcaatga cggtttgcgt 240
   ggttttcagc cacagcaaac cgagcaaacg ctgcgccaga ttttgcagga tgtcaaagcc 300
   gccaacgctg aaccattgtt aatgcaaata cgtctgcctg caaactatgg tcgccgttat 360
   aatgaagcct ttagcgccat ttaccccaaa ctcgccaaag agtttgatgt tccgctgctg 420
   ccctttttta tggaagaggt ctacctcaag ccacaatgga tgcaggatga cggtattcat 480
   cccaaccgcg acgcccagcc gtttattgcc gactggatgg cgaagcagtt gcagccttta 540
   gtaaatcatg actcataagg atccgc 566
<210> 69
   <211> 32
   <212> DNA
   <213> Artificial Sequence
<220>
   <223> Synthetic primer
<400> 69
   catgccatgg cggacacgtt attgattctg gg 32
<210> 70
   <211> 9
   <212> PRT
   <213> Artificial Sequence
<220>
   <223> Synthetic peptide
<400> 70
   Met Ala Asp Thr Leu Leu Ile Leu Gly
   1 5
<210> 71
   <211> 36
   <212> DNA
   <213> Artificial Sequence
<220>
   <223> Synthetic primer
<400> 71
   gcggatcctt atgagtcatg atttactaaa ggctgc 36
<210> 72
   <211> 9
   <212> PRT
   <213> Artificial Sequence
<220>
   <223> Synthetic peptide
<400> 72
   Ser Asp His Asn Val Leu Pro Gln Leu
   1               5
<210> 73
   <211> 183
   <212> PRT
   <213> Escherichia coli
<400> 73
   Met Ala Asp Thr Leu Leu Ile Leu Gly Asp Ser Leu Ser Ala Gly Tyr
   1               5                   10                  15
   Arg Met Ser Ala Ser Ala Ala Trp Pro Ala Leu Leu Asn Asp Lys Trp
               20                  25                  30
   Gln Ser Lys Thr Ser Val Val Asn Ala Ser Ile Ser Gly Asp Thr Ser
           35                  40                  45
   Gln Gln Gly Leu Ala Arg Leu Pro Ala Leu Leu Lys Gln His Gln Pro
       50                  55                  60
   Arg Trp Val Leu Val Glu Leu Gly Gly Asn Asp Gly Leu Arg Gly Phe
   65                  70                  75                  80
   Gln Pro Gln Gln Thr Glu Gln Thr Leu Arg Gln Ile Leu Gln Asp Val
                   85                  90                  95
   Lys Ala Ala Asn Ala Glu Pro Leu Leu Met Gln Ile Arg Leu Pro Ala
               100                 105                 110
   Asn Tyr Gly Arg Arg Tyr Asn Glu Ala Phe Ser Ala Ile Tyr Pro Lys
           115                 120                 125
   Leu Ala Lys Glu Phe Asp Val Pro Leu Leu Pro Phe Phe Met Glu Glu
       130                 135                 140
   Val Tyr Leu Lys Pro Gln Trp Met Gln Asp Asp Gly Ile His Pro Asn
   145                 150                 155                 160
   Arg Asp Ala Gln Pro Phe Ile Ala Asp Trp Met Ala Lys Gln Leu Gln
                   165                 170                 175
   Pro Leu Val Asn His Asp Ser
               180
<210> 74
   <211> 33
   <212> DNA
   <213> Artificial Sequence
<220>
   <223> Synthetic primer
<400> 74
   cattactcga gcgcactccc gttctggata atg 33
<210> 75
   <211> 34
   <212> DNA
   <213> Artificial Sequence
<220>
   <223> Synthetic primer
<400> 75
   gggaagctta tgagtcatga tttactaaag gctg 34
<210> 76
   <211> 9
   <212> PRT
   <213> Artificial Sequence
<220>
   <223> Synthetic peptide
<400> 76
   Ser Asp His Asn Val Leu Pro Gln Leu
   1               5
<210> 77
   <211> 939
   <212> DNA
   <213> Listeria monocytogenes
<400> 77
   atgaacgcag gaattttagg agtaggtaaa tacgtacctg aaaaaatagt aactaacttt 60
   gatttagaaa aaattatgga tacatctgat gagtggattc gaactcgaac tggtatcgaa 120
   gaaagaagaa ttgctcgtga tgacgaatat acgcatgatt tagcatatga agcagcaaag 180
   gtagccatta agaatgctgg tcttacacca gatgatatcg acttgtttat cgttgcaact 240
   gttacacaag aagcgacatt tccttcagtt gctaatatta tccaagaccg tttaggagcg 300
   aaaaatgctg ccggtatgga cgttgaggca gcttgtgctg gttttacttt tggagtagtg 360
   actgcagcac aattcattaa aacaggtgca tataaaaata tcgtagttgt cggtgcggat 420
   aaattatcta aaatcactaa ctgggatgat cgcacaacag ccgtattatt cggtgatgga 480
   gcaggtgctg tagttatggg gccagtttct gatgatcatg gtttactttc atttgactta 540
   ggttcagatg gatcaggtgg taaatacttg aatttagatg aaaataaaaa gatttatatg 600
   aatggtcgtg aagtgttccg ttttgcagtt cgccaaatgg gagaagcttc actacgagta 660
   cttgaacgtg ctggtcttga gaaagaagac ttggacttac taattcctca ccaagcaaat 720
   attcgtatca tggaagcttc tcgtgagcgt ttgaatttac cagaagaaaa actgatgaaa 780
   acagtacaca aatatggtaa tacttcatcc tcttcaatcg cacttgcgct agttgatgca 840
   gtggaagaag gacgtattaa agataatgat aacgttctgc ttgtaggctt tggcggcgga 900
   ctaacatggg gcgccctaat cattcgttgg ggtaagtaa 939
<210> 78
   <211> 951
   <212> DNA
   <213> Listeria monocytogenes
<400> 78
   ctcgagatga atgcaggtat tctgggtgtt ggtaaatatg tgccggaaaa aatcgtgacc      60
   aacttcgatc tggaaaaaat tatggatacc agcgacgaat ggattcgtac ccgtaccggt     120
   attgaagaac gtcgtattgc acgtgatgat gaatataccc atgatctggc atatgaagca     180
   gcaaaagtgg caattaaaaa tgcaggtctg acaccggatg atattgacct gtttattgtt     240
   gcaaccgtta cccaagaagc aacctttccg agcgttgcaa atattattca ggatcgtctg     300
   ggtgcaaaaa atgcagcagg tatggatgtt gaagcagcat gtgcaggttt tacctttggt     360
   gttgttaccg cagcccagtt tattaaaacc ggtgcctata aaaacatcgt tgttgttggt     420
   gcagataaac tgagcaaaat taccaattgg gatgatcgta ccaccgcagt tctgtttggt     480
   gatggtgccg gtgcagttgt tatgggtccg gttagtgatg atcatggtct gctgtcattt     540
   gatctgggta gtgatggtag cggtggtaaa tatctgaatc tggacgagaa taaaaaaatt     600
   tatatgaatg gtcgcgaagt gtttcgtttt gcagttcgtc agatgggtga agcaagcctg     660
   cgtgttctgg aacgtgcagg tctggaaaaa gaggatctgg atctgctgat tccgcatcag     720
   gcaaatattc gtattatgga agcaagccgt gaacgtctga atctgccgga agaaaaactg     780
   atgaaaaccg tgcataaata tggcaatacc agcagcagca gcattgcact ggcactggtt     840
   gatgcagttg aagaaggtcg cattaaagat aatgataacg ttctgctggt tggttttggt     900
   ggtggtctga cctggggtgc actgattatt cgttggggta aataactgca g              951
<210> 79
   <211> 20
   <212> DNA
   <213> Artificial Sequence
<220>
   <223> Synthetic primer
<400> 79
   atgccatagc atttttatcc 20
<210> 80
   <211> 21
   <212> DNA
   <213> Artificial Sequence
<220>
   <223> Synthetic primer
<400> 80
   tctgatttaa tctgtatcag g 21
<210> 81
   <211> 1476
   <212> DNA
   <213> Escherichia coli
<400> 81
   atggctaact acttcaatac actgaatctg cgccagcagc tggcacagct gggcaaatgt      60
   cgctttatgg gccgcgatga attcgccgat ggcgcgagct accttcaggg taaaaaagta     120
   gtcatcgtcg gctgtggcgc acagggtctg aaccagggcc tgaacatgcg tgattctggt     180
   ctcgatatct cctacgctct gcgtaaagaa gcgattgccg agaagcgcgc gtcctggcgt     240
   aaagcgaccg aaaatggttt taaagtgggt acttacgaag aactgatccc acaggcggat     300
   ctggtgatta acctgacgcc ggacaagcag cactctgatg tagtgcgcac cgtacagcca     360
   ctgatgaaag acggcgcggc gctgggctac tcgcacggtt tcaacatcgt cgaagtgggc     420
   gagcagatcc gtaaagatat caccgtagtg atggttgcgc cgaaatgccc aggcaccgaa     480
   gtgcgtgaag agtacaaacg tgggttcggc gtaccgacgc tgattgccgt tcacccggaa     540
   aacgatccga aaggcgaagg catggcgatt gccaaagcct gggcggctgc aaccggtggt     600
   caccgtgcgg gtgtgctgga atcgtccttc gttgcggaag tgaaatctga cctgatgggc     660
   gagcaaacca tcctgtgcgg tatgttgcag gctggctctc tgctgtgctt cgacaagctg     720
   gtggaagaag gtaccgatcc agcatacgca gaaaaactga ttcagttcgg ttgggaaacc     780
   atcaccgaag cactgaaaca gggcggcatc accctgatga tggaccgtct ctctaacccg     840
   gcgaaactgc gtgcttatgc gctttctgaa cagctgaaag agatcatggc acccctgttc     900
   cagaaacata tggacgacat catctccggc gaattctctt ccggtatgat ggcggactgg     960
   gccaacgatg ataagaaact gctgacctgg cgtgaagaga ccggcaaaac cgcgtttgaa    1020
   accgcgccgc agtatgaagg caaaatcggc gagcaggagt acttcgataa aggcgtactg    1080
   atgattgcga tggtgaaagc gggcgttgaa ctggcgttcg aaaccatggt cgattccggc    1140
   atcattgaag agtctgcata ttatgaatca ctgcacgagc tgccgctgat tgccaacacc    1200
   atcgcccgta agcgtctgta cgaaatgaac gtggttatct ctgataccgc tgagtacggt    1260
   aactatctgt tctcttacgc ttgtgtgccg ttgctgaaac cgtttatggc agagctgcaa    1320
   ccgggcgacc tgggtaaagc tattccggaa ggcgcggtag ataacggcca actgcgtgat    1380
   gtgaacgaag cgattcgcag ccatgcgatt gagcaggtag gtaagaaact gcgcggctat    1440
   atgacagata tgaaacgtat tgctgttgcg ggttaa                              1476
<210> 82
   <211> 1851
   <212> DNA
   <213> Escherichia coli
<400> 82
   atgcctaagt accgttccgc caccaccact catggtcgta atatggcggg tgctcgtgcg      60
   ctgtggcgcg ccaccggaat gaccgacgcc gatttcggta agccgattat cgcggttgtg     120
   aactcgttca cccaatttgt accgggtcac gtccatctgc gcgatctcgg taaactggtc     180
   gccgaacaaa ttgaagcggc tggcggcgtt gccaaagagt tcaacaccat tgcggtggat     240
   gatgggattg ccatgggcca cggggggatg ctttattcac tgccatctcg cgaactgatc     300
   gctgattccg ttgagtatat ggtcaacgcc cactgcgccg acgccatggt ctgcatctct 360
   aactgcgaca aaatcacccc ggggatgctg atggcttccc tgcgcctgaa tattccggtg 420
   atctttgttt ccggcggccc gatggaggcc gggaaaacca aactttccga tcagatcatc 480
   aagctcgatc tggttgatgc gatgatccag ggcgcagacc cgaaagtatc tgactcccag 540
   agcgatcagg ttgaacgttc cgcgtgtccg acctgcggtt cctgctccgg gatgtttacc 600
   gctaactcaa tgaactgcct gaccgaagcg ctgggcctgt cgcagccggg caacggctcg 660
   ctgctggcaa cccacgccga ccgtaagcag ctgttcctta atgctggtaa acgcattgtt 720
   gaattgacca aacgttatta cgagcaaaac gacgaaagtg cactgccgcg taatatcgcc 780
   agtaaggcgg cgtttgaaaa cgccatgacg ctggatatcg cgatgggtgg atcgactaac 840
   accgtacttc acctgctggc ggcggcgcag gaagcggaaa tcgacttcac catgagtgat 900
   atcgataagc tttcccgcaa ggttccacag ctgtgtaaag ttgcgccgag cacccagaaa 960
   taccatatgg aagatgttca ccgtgctggt ggtgttatcg gtattctcgg cgaactggat 1020
   cgcgcggggt tactgaaccg tgatgtgaaa aacgtacttg gcctgacgtt gccgcaaacg 1080
   ctggaacaat acgacgttat gctgacccag gatgacgcgg taaaaaatat gttccgcgca 1140
   ggtcctgcag gcattcgtac cacacaggca ttctcgcaag attgccgttg ggatacgctg 1200
   gacgacgatc gcgccaatgg ctgtatccgc tcgctggaac acgcctacag caaagacggc 1260
   ggcctggcgg tgctctacgg taactttgcg gaaaacggct gcatcgtgaa aacggcaggc 1320
   gtcgatgaca gcatcctcaa attcaccggc ccggcgaaag tgtacgaaag ccaggacgat 1380
   gcggtagaag cgattctcgg cggtaaagtt gtcgccggag atgtggtagt aattcgctat 1440
   gaaggcccga aaggcggtcc ggggatgcag gaaatgctct acccaaccag cttcctgaaa 1500
   tcaatgggtc tcggcaaagc ctgtgcgctg atcaccgacg gtcgtttctc tggtggcacc 1560
   tctggtcttt ccatcggcca cgtctcaccg gaagcggcaa gcggcggcag cattggcctg 1620
   attgaagatg gtgacctgat cgctatcgac atcccgaacc gtggcattca gttacaggta 1680
   agcgatgccg aactggcggc gcgtcgtgaa gcgcaggacg ctcgaggtga caaagcctgg 1740
   acgccgaaaa atcgtgaacg tcaggtctcc tttgccctgc gtgcttatgc cagcctggca 1800
   accagcgccg acaaaggcgc ggtgcgcgat aaatcgaaac tggggggtta a 1851
<210> 83
   <211> 3682
   <212> DNA
   <213> Escherichia coli
<400> 83
   ctcgagcgca ctcccgttct ggataatgtt ttttgcgccg acatcataac ggttctggca     60
   aatattctga aatgagctgt tgacaattaa tcatccggct cgtataatgt gtggaattgt    120
   gagcggataa caatttcaca caggaaacag cgccgctgag aaaaagcgaa gcggcactgc    180
   tctttaacaa tttatcagac aatctgtgtg ggcactcgac cggaattatc gtttaacttt 240
   attattaaaa attaaagagg tatatattaa tgtatcgatt aaataaggag gaataaacca 300
   tggctaacta ctttaacacc ctgaacctgc gtcagcaact ggctcaactg ggcaaatgtc 360
   gttttatggg ccgtgatgaa tttgcggatg gtgcttcata tctgcaaggc aaaaaagtgg 420
   ttattgtggg ttgcggtgcg cagggcctga accaaggtct gaatatgcgt gattcaggtc 480
   tggacatttc gtatgcactg cgcaaagaag ccatcgcaga aaaacgtgca tcgtggcgca 540
   aagctaccga aaatggcttt aaagtgggta cgtatgaaga actgattccg caggcggatc 600
   tggtgatcaa cctgaccccg gataaacagc atagtgacgt cgtgcgtacg gttcaaccgc 660
   tgatgaaaga tggcgcggcc ctgggttatt cccacggctt taatattgtc gaagtgggtg 720
   aacagattcg caaagacatc accgttgtca tggtggcgcc gaaatgtccg ggcacggaag 780
   ttcgtgaaga atacaaacgc ggcttcggtg tcccgaccct gattgccgtg catccggaaa 840
   acgatccgaa aggcgagggt atggctatcg caaaagcctg ggcagctgcg accggcggtc 900
   accgtgcagg tgtgctggaa agctctttcg ttgccgaagt caaaagcgat ctgatgggcg 960
   aacagaccat tctgtgcggt atgctgcaag ccggctctct gctgtgtttt gataaactgg 1020
   ttgaagaagg tacggacccg gcctatgctg aaaaactgat tcagttcggc tgggaaacca 1080
   tcacggaagc gctgaaacaa ggcggtatta ccctgatgat ggatcgtctg agtaatccgg 1140
   caaaactgcg tgcatacgcc ctgtccgaac agctgaaaga aatcatggca ccgctgttcc 1200
   aaaaacatat ggatgacatc atctccggtg aatttagcag cggcatgatg gcagattggg 1260
   ctaacgatga caaaaaactg ctgacctggc gcgaagaaac cggcaaaacg gcgtttgaaa 1320
   cggccccgca gtatgaaggc aaaatcggtg aacaagaata cttcgataaa ggtgttctga 1380
   tgatcgcaat ggtgaaagct ggcgttgaac tggcctttga aaccatggtg gatagcggta 1440
   ttatcgaaga aagcgcatat tacgaatctc tgcatgaact gccgctgatt gcgaacacca 1500
   tcgcccgtaa acgcctgtac gaaatgaacg tggttatctc agatacggca gaatatggca 1560
   actacctgtt ttcgtacgct tgcgtgccgc tgctgaaacc gttcatggcg gaactgcaac 1620
   cgggcgacct gggtaaagcg atcccggaag gtgccgttga taacggccag ctgcgtgacg 1680
   tcaatgaagc aattcgcagc cacgctatcg aacaagtggg taaaaaactg cgtggctaca 1740
   tgacggatat gaaacgtatt gcggttgcgg gctgatcatg ccgaaatatc gctctgctac 1800
   gacgacgcat ggccgcaata tggctggtgc tcgcgctctg tggcgtgcta cgggtatgac 1860
   ggatgctgat tttggcaaac cgattatcgc ggtggtgaac agctttaccc agttcgtccc 1920
   gggccatgtg cacctgcgtg atctgggtaa actggtggcc gaacagatcg aagcggccgg 1980
   cggtgtggca aaagaattta ataccatcgc tgttgatgac ggcattgcga tgggtcatgg 2040
   cggtatgctg tattcgctgc cgagccgtga actgattgcc gattcggttg aatacatggt 2100
   gaacgcacac tgtgctgacg cgatggtttg tatcagcaat tgcgataaaa ttaccccggg 2160
   tatgctgatg gcctccctgc gcctgaacat cccggttatt tttgtctcag gcggtccgat 2220
   ggaagcaggc aaaacgaaac tgagcgacca gattatcaaa ctggacctgg tggatgccat 2280
   gatccaaggt gcagatccga aagtgagcga ctctcagagt gatcaagttg aacgttccgc 2340
   atgcccgacc tgtggctcct gctcaggcat gtttaccgct aactcaatga attgcctgac 2400
   ggaagcgctg ggtctgtctc agccgggtaa cggtagtctg ctggccaccc atgcagatcg 2460
   taaacaactg ttcctgaatg cgggtaaacg tattgttgaa ctgacgaaac gctattacga 2520
   acagaacgac gaatctgccc tgccgcgcaa tatcgcaagt aaagcagctt ttgaaaacgc 2580
   tatgaccctg gatattgcga tgggcggttc caccaatacg gtgctgcacc tgctggcggc 2640
   ggcacaggaa gccgaaattg atttcaccat gtccgacatt gataaactgt cacgtaaagt 2700
   cccgcagctg tgtaaagtgg cgccgagcac ccaaaaatat catatggaag atgttcaccg 2760
   tgccggcggt gtcattggca tcctgggtga actggaccgt gcaggcctgc tgaaccgcga 2820
   tgtgaaaaat gttctgggtc tgaccctgcc gcagacgctg gaacaatacg atgtcatgct 2880
   gacccaggat gacgcagtga aaaacatgtt ccgtgctggc ccggcgggta tccgcaccac 2940
   gcaggcgttc tctcaagact gtcgttggga tacgctggat gacgatcgtg ctaatggctg 3000
   cattcgctcg ctggaacatg cgtatagcaa agatggcggt ctggccgttc tgtacggcaa 3060
   ctttgcagaa aatggttgta tcgtgaaaac cgctggcgtg gatgattcta ttctgaaatt 3120
   cacgggtccg gcgaaagttt atgaaagtca ggacgatgcc gtcgaagcaa tcctgggcgg 3180
   taaagtcgtg gcgggcgatg ttgtcgtgat tcgttatgaa ggcccgaaag gcggtccggg 3240
   tatgcaggaa atgctgtacc cgacctcgtt tctgaaaagc atgggcctgg gtaaagcctg 3300
   cgcactgatc accgatggtc gcttctcggg cggtacgtct ggcctgagta ttggtcacgt 3360
   gtcaccggaa gctgcgtccg gcggttcaat cggcctgatt gaagacggtg atctgattgc 3420
   gatcgacatt ccgaaccgcg gcattcagct gcaagtctct gatgccgaac tggcagcacg 3480
   tcgcgaagct caggacgcgc gtggtgataa agcctggacc ccgaaaaatc gtgaacgcca 3540
   agtgagtttt gcactgcgcg cttatgcgag tctggcgacg agtgccgaca aaggtgctgt 3600
   gcgtgataaa tccaaactgg gtggctaaga tctgcagctg gtaccgcggc cgcgtttaaa 3660
   cgaattctag aagcttacgc gt 3682
<210> 84
   <211> 491
   <212> PRT
   <213> Escherichia coli
<400> 84
   Met Ala Asn Tyr Phe Asn Thr Leu Asn Leu Arg Gln Gln Leu Ala Gln
   1               5                   10                  15
   Leu Gly Lys Cys Arg Phe Met Gly Arg Asp Glu Phe Ala Asp Gly Ala
               20                  25                  30
   Ser Tyr Leu Gln Gly Lys Lys Val Val Ile Val Gly Cys Gly Ala Gln
           35                  40                  45
   Gly Leu Asn Gln Gly Leu Asn Met Arg Asp Ser Gly Leu Asp Ile Ser
       50                  55                  60
   Tyr Ala Leu Arg Lys Glu Ala Ile Ala Glu Lys Arg Ala Ser Trp Arg
   65                  70                  75                  80
   Lys Ala Thr Glu Asn Gly Phe Lys Val Gly Thr Tyr Glu Glu Leu Ile
                   85                  90                  95
   Pro Gln Ala Asp Leu Val Ile Asn Leu Thr Pro Asp Lys Gln His Ser
               100                 105                 110
   Asp Val Val Arg Thr Val Gln Pro Leu Met Lys Asp Gly Ala Ala Leu
           115                 120                 125
   Gly Tyr Ser His Gly Phe Asn Ile Val Glu Val Gly Glu Gln Ile Arg
       130                 135                 140
   Lys Asp Ile Thr Val Val Met Val Ala Pro Lys Cys Pro Gly Thr Glu
   145                 150                 155                 160
   Val Arg Glu Glu Tyr Lys Arg Gly Phe Gly Val Pro Thr Leu Ile Ala
                   165                 170                 175
   Val His Pro Glu Asn Asp Pro Lys Gly Glu Gly Met Ala Ile Ala Lys
               180                 185                 190
   Ala Trp Ala Ala Ala Thr Gly Gly His Arg Ala Gly Val Leu Glu Ser
           195                 200                 205
   Ser Phe Val Ala Glu Val Lys Ser Asp Leu Met Gly Glu Gln Thr Ile
       210                 215                 220
   Leu Cys Gly Met Leu Gln Ala Gly Ser Leu Leu Cys Phe Asp Lys Leu
   225                 230                 235                 240
   Val Glu Glu Gly Thr Asp Pro Ala Tyr Ala Glu Lys Leu Ile Gln Phe
                   245                 250                 255
   Gly Trp Glu Thr Ile Thr Glu Ala Leu Lys Gln Gly Gly Ile Thr Leu
               260                 265                 270
   Met Met Asp Arg Leu Ser Asn Pro Ala Lys Leu Arg Ala Tyr Ala Leu
           275                 280                 285
   Ser Glu Gln Leu Lys Glu Ile Met Ala Pro Leu Phe Gln Lys His Met
       290                 295                 300
   Asp Asp Ile Ile Ser Gly Glu Phe Ser Ser Gly Met Met Ala Asp Trp
   305                 310                 315                 320
   Ala Asn Asp Asp Lys Lys Leu Leu Thr Trp Arg Glu Glu Thr Gly Lys
                   325                 330                 335
   Thr Ala Phe Glu Thr Ala Pro Gln Tyr Glu Gly Lys Ile Gly Glu Gln
               340                 345                 350
   Glu Tyr Phe Asp Lys Gly Val Leu Met Ile Ala Met Val Lys Ala Gly
           355                 360                 365
   Val Glu Leu Ala Phe Glu Thr Met Val Asp Ser Gly Ile Ile Glu Glu
       370                 375                 380
   Ser Ala Tyr Tyr Glu Ser Leu His Glu Leu Pro Leu Ile Ala Asn Thr
   385                 390                 395                 400
   Ile Ala Arg Lys Arg Leu Tyr Glu Met Asn Val Val Ile Ser Asp Thr
                   405                 410                 415
   Ala Glu Tyr Gly Asn Tyr Leu Phe Ser Tyr Ala Cys Val Pro Leu Leu
               420                 425                 430
   Lys Pro Phe Met Ala Glu Leu Gln Pro Gly Asp Leu Gly Lys Ala Ile
           435                 440                 445
   Pro Glu Gly Ala Val Asp Asn Gly Gln Leu Arg Asp Val Asn Glu Ala
       450                 455                 460
   Ile Arg Ser His Ala Ile Glu Gln Val Gly Lys Lys Leu Arg Gly Tyr
   465                 470                 475                 480
   Met Thr Asp Met Lys Arg Ile Ala Val Ala Gly
                   485                 490
<210> 85
   <211> 616
   <212> PRT
   <213> Escherichia coli
<400> 85
   Met Pro Lys Tyr Arg Ser Ala Thr Thr Thr His Gly Arg Asn Met Ala
   1               5                   10                  15
   Gly Ala Arg Ala Leu Trp Arg Ala Thr Gly Met Thr Asp Ala Asp Phe
   20 25 30
   Gly Lys Pro Ile Ile Ala Val Val Asn Ser Phe Thr Gln Phe Val Pro
   35 40 45
   Gly His Val His Leu Arg Asp Leu Gly Lys Leu Val Ala Glu Gln Ile
   50 55 60
   Glu Ala Ala Gly Gly Val Ala Lys Glu Phe Asn Thr Ile Ala Val Asp
   65 70 75 80
   Asp Gly Ile Ala Met Gly His Gly Gly Met Leu Tyr Ser Leu Pro Ser
   85 90 95
   Arg Glu Leu Ile Ala Asp Ser Val Glu Tyr Met Val Asn Ala His Cys
   100 105 110
   Ala Asp Ala Met Val Cys Ile Ser Asn Cys Asp Lys Ile Thr Pro Gly
   115 120 125
   Met Leu Met Ala Ser Leu Arg Leu Asn Ile Pro Val Ile Phe Val Ser
   130 135 140
   Gly Gly Pro Met Glu Ala Gly Lys Thr Lys Leu Ser Asp Gln Ile Ile
   145 150 155 160
   Lys Leu Asp Leu Val Asp Ala Met Ile Gln Gly Ala Asp Pro Lys Val
   165 170 175
   Ser Asp Ser Gln Ser Asp Gln Val Glu Arg Ser Ala Cys Pro Thr Cys
   180 185 190
   Gly Ser Cys Ser Gly Met Phe Thr Ala Asn Ser Met Asn Cys Leu Thr
   195 200 205
   Glu Ala Leu Gly Leu Ser Gln Pro Gly Asn Gly Ser Leu Leu Ala Thr
   210 215 220
   His Ala Asp Arg Lys Gln Leu Phe Leu Asn Ala Gly Lys Arg Ile Val
   225 230 235 240
   Glu Leu Thr Lys Arg Tyr Tyr Glu Gln Asn Asp Glu Ser Ala Leu Pro
   245 250 255
   Arg Asn Ile Ala Ser Lys Ala Ala Phe Glu Asn Ala Met Thr Leu Asp
   260 265 270
   Ile Ala Met Gly Gly Ser Thr Asn Thr Val Leu His Leu Leu Ala Ala
           275                 280                 285
   Ala Gln Glu Ala Glu Ile Asp Phe Thr Met Ser Asp Ile Asp Lys Leu
       290                 295                 300
   Ser Arg Lys Val Pro Gln Leu Cys Lys Val Ala Pro Ser Thr Gln Lys
   305                 310                 315                 320
   Tyr His Met Glu Asp Val His Arg Ala Gly Gly Val Ile Gly Ile Leu
                   325                 330                 335
   Gly Glu Leu Asp Arg Ala Gly Leu Leu Asn Arg Asp Val Lys Asn Val
               340                 345                 350
   Leu Gly Leu Thr Leu Pro Gln Thr Leu Glu Gln Tyr Asp Val Met Leu
           355                 360                 365
   Thr Gln Asp Asp Ala Val Lys Asn Met Phe Arg Ala Gly Pro Ala Gly
       370                 375                 380
   Ile Arg Thr Thr Gln Ala Phe Ser Gln Asp Cys Arg Trp Asp Thr Leu
   385                 390                 395                 400
   Asp Asp Asp Arg Ala Asn Gly Cys Ile Arg Ser Leu Glu His Ala Tyr
                   405                 410                 415
   Ser Lys Asp Gly Gly Leu Ala Val Leu Tyr Gly Asn Phe Ala Glu Asn
               420                 425                 430
   Gly Cys Ile Val Lys Thr Ala Gly Val Asp Asp Ser Ile Leu Lys Phe
           435                 440                 445
   Thr Gly Pro Ala Lys Val Tyr Glu Ser Gln Asp Asp Ala Val Glu Ala
       450                 455                 460
   Ile Leu Gly Gly Lys Val Val Ala Gly Asp Val Val Val Ile Arg Tyr
   465                 470                 475                 480
   Glu Gly Pro Lys Gly Gly Pro Gly Met Gln Glu Met Leu Tyr Pro Thr
                   485                 490                 495
   Ser Phe Leu Lys Ser Met Gly Leu Gly Lys Ala Cys Ala Leu Ile Thr
               500                 505                 510
   Asp Gly Arg Phe Ser Gly Gly Thr Ser Gly Leu Ser Ile Gly His Val
           515                 520                 525
   Ser Pro Glu Ala Ala Ser Gly Gly Ser Ile Gly Leu Ile Glu Asp Gly
       530                 535                 540
   Asp Leu Ile Ala Ile Asp Ile Pro Asn Arg Gly Ile Gln Leu Gln Val
   545                 550                 555                 560
   Ser Asp Ala Glu Leu Ala Ala Arg Arg Glu Ala Gln Asp Ala Arg Gly
                   565                 570                 575
   Asp Lys Ala Trp Thr Pro Lys Asn Arg Glu Arg Gln Val Ser Phe Ala
               580                 585                 590
   Leu Arg Ala Tyr Ala Ser Leu Ala Thr Ser Ala Asp Lys Gly Ala Val
           595                 600                 605
   Arg Asp Lys Ser Lys Leu Gly Gly
       610                 615
<210> 86
   <211> 258
   <212> PRT
   <213> Bacillus subtilis
<400> 86
   Met Asn Phe Ser Leu Glu Gly Arg Asn Ile Val Val Met Gly Val Ala
   1               5                   10                  15
   Asn Lys Arg Ser Ile Ala Trp Gly Ile Ala Arg Ser Leu His Glu Ala
               20                  25                  30
   Gly Ala Arg Leu Ile Phe Thr Tyr Ala Gly Glu Arg Leu Glu Lys Ser
           35                  40                  45
   Val His Glu Leu Ala Gly Thr Leu Asp Arg Asn Asp Ser Ile Ile Leu
       50                  55                  60
   Pro Cys Asp Val Thr Asn Asp Ala Glu Ile Glu Thr Cys Phe Ala Ser
   65                  70                  75                  80
   Ile Lys Glu Gln Val Gly Val Ile His Gly Ile Ala His Cys Ile Ala
                   85                  90                  95
   Phe Ala Asn Lys Glu Glu Leu Val Gly Glu Tyr Leu Asn Thr Asn Arg
               100                 105                 110
   Asp Gly Phe Leu Leu Ala His Asn Ile Ser Ser Tyr Ser Leu Thr Ala
           115                 120                 125
   Val Val Lys Ala Ala Arg Pro Met Met Thr Glu Gly Gly Ser Ile Val
       130                 135                 140
   Thr Leu Thr Tyr Leu Gly Gly Glu Leu Val Met Pro Asn Tyr Asn Val
   145                 150                 155                 160
   Met Gly Val Ala Lys Ala Ser Leu Asp Ala Ser Val Lys Tyr Leu Ala
                   165                 170                 175
   Ala Asp Leu Gly Lys Glu Asn Ile Arg Val Asn Ser Ile Ser Ala Gly
               180                 185                 190
   Pro Ile Arg Thr Leu Ser Ala Lys Gly Ile Ser Asp Phe Asn Ser Ile
           195                 200                 205
   Leu Lys Asp Ile Glu Glu Arg Ala Pro Leu Arg Arg Thr Thr Thr Pro
       210                 215                 220
   Glu Glu Val Gly Asp Thr Ala Ala Phe Leu Phe Ser Asp Met Ser Arg
   225                 230                 235                 240
   Gly Ile Thr Gly Glu Asn Leu His Val Asp Ser Gly Phe His Ile Thr
                   245                 250                 255
   Ala Arg
<210> 87
   <211> 37
   <212> DNA
   <213> Artificial Sequence
<220>
   <223> Synthetic primer
<400> 87
   gagaccatgg atgaattttt cacttgaagg ccgtaac 37
<210> 88
   <211> 46
   <212> DNA
   <213> Artificial Sequence
<220>
   <223> Synthetic primer
<400> 88
   gagacgctgc agttagcggg cagtgatatg gaaaccagaa tcaacg 46
<210> 89
   <211> 789
   <212> DNA
   <213> Listeria monocytogenes
<400> 89
   ccatggatga atttttcact tgaaggccgt aacattgttg tgatgggggt agccaacaaa        60
   cgcagcatcg cctggggcat tgcgcgttct ttacatgaag cgggtgcacg tttgattttc       120
   acatacgctg gtgaacgcct ggagaaatcc gttcacgagc ttgccggaac attagaccgc       180
   aacgattcca tcatcctccc ttgcgatgtt acaaacgacg cagaaatcga aacttgcttc       240
   gcaagcatta aggagcaggt cggtgtaatc cacggtatcg cgcattgtat cgcgtttgcc       300
   aacaaagaag agcttgtcgg cgagtactta aacacaaatc gtgacggctt ccttttggct       360
   cataacatca gctcatattc tctgactgct gttgtcaaag cggcacgtcc gatgatgact       420
   gaaggcggaa gcattgtcac tttgacgtac cttggcggag agcttgtgat gccaaactac       480
   aacgtcatgg gtgtagcaaa agcttctctt gatgcaagtg tgaaatattt agctgctgac       540
   ttaggaaaag aaaatatccg cgtcaacagc atttctgccg gcccgatcag aacattatct       600
   gctaaaggca tcagcgattt caactctatc ttaaaagaca tcgaagagcg tgcaccgctt       660
   cgccgcacga caacacctga agaagtgggc gatacagctg cgttcttgtt cagcgatatg       720
   tcccgcggga ttacaggtga aaatcttcac gttgattctg gtttccatat cactgcccgc       780
   taactgcag                                                               789
<210> 90
   <211> 789
   <212> DNA
   <213> Listeria monocytogenes
<400> 90
   ctcgagatga atttttcact tgaaggccgt aacattgttg tgatgggggt agccaacaaa 60
   cgcagcatcg cctggggcat tgcgcgttct ttacatgaag cgggtgcacg tttgattttc 120
   acatacgctg gtgaacgcct ggagaaatcc gttcacgagc ttgccggaac attagaccgc 180
   aacgattcca tcatcctccc ttgcgatgtt acaaacgacg cagaaatcga aacttgcttc 240
   gcaagcatta aggagcaggt cggtgtaatc cacggtatcg cgcattgtat cgcgtttgcc 300
   aacaaagaag agcttgtcgg cgagtactta aacacaaatc gtgacggctt ccttttggct 360
   cataacatca gctcatattc tctgactgct gttgtcaaag cggcacgtcc gatgatgact 420
   gaaggcggaa gcattgtcac tttgacgtac cttggcggag agcttgtgat gccaaactac 480
   aacgtcatgg gtgtagcaaa agcttctctt gatgcaagtg tgaaatattt agctgctgac 540
   ttaggaaaag aaaatatccg cgtcaacagc atttctgccg gcccgatcag aacattatct 600
   gctaaaggca tcagcgattt caactctatc ttaaaagaca tcgaagagcg tgcaccgctt 660
   cgccgcacga caacacctga agaagtgggc gatacagctg cgttcttgtt cagcgatatg 720
   tcccgcggga ttacaggtga aaatcttcac gttgattctg gtttccatat cactgcccgc 780
   taactgcag 789
<210> 91
   <211> 39
   <212> DNA
   <213> Artificial Sequence
<220>
   <223> Synthetic primer
<400> 91
   gagacgctcg agatgaattt ttcacttgaa ggccgtaac 39
<210> 92
   <211> 258
   <212> PRT
   <213> Escherichia coli
<400> 92
   Met Asn Phe Ser Leu Glu Gly Arg Asn Ile Val Val Met Gly Val Ala
   1               5                   10                  15
   Asn Lys Arg Ser Ile Ala Trp Gly Ile Ala Arg Ser Leu His Glu Ala
               20                  25                  30
   Gly Ala Arg Leu Ile Phe Thr Tyr Ala Gly Glu Arg Leu Glu Lys Ser
           35                  40                  45
   Val His Glu Leu Ala Gly Thr Leu Asp Arg Asn Asp Ser Ile Ile Leu
       50                  55                  60
   Pro Cys Asp Val Thr Asn Asp Ala Glu Ile Glu Thr Cys Phe Ala Ser
   65                  70                  75                  80
   Ile Lys Glu Gln Val Gly Val Ile His Gly Ile Ala His Cys Ile Ala
                   85                  90                  95
   Phe Ala Asn Lys Glu Glu Leu Val Gly Glu Tyr Leu Asn Thr Asn Arg
               100                 105                 110
   Asp Gly Phe Leu Leu Ala His Asn Ile Ser Ser Tyr Ser Leu Thr Ala
           115                 120                 125
   Val Val Lys Ala Ala Arg Pro Met Met Thr Glu Gly Gly Ser Ile Val
       130                 135                 140
   Thr Leu Thr Tyr Leu Gly Gly Glu Leu Val Met Pro Asn Tyr Asn Val
   145                 150                 155                 160
   Met Gly Val Ala Lys Ala Ser Leu Asp Ala Ser Val Lys Tyr Leu Ala
                   165                 170                 175
   Ala Asp Leu Gly Lys Glu Asn Ile Arg Val Asn Ser Ile Ser Ala Gly
               180                 185                 190
   Pro Ile Arg Thr Leu Ser Ala Lys Gly Ile Ser Asp Phe Asn Ser Ile
           195                 200                 205
   Leu Lys Asp Ile Glu Glu Arg Ala Pro Leu Arg Arg Thr Thr Thr Pro
       210                 215                 220
   Glu Glu Val Gly Asp Thr Ala Ala Phe Leu Phe Ser Asp Met Ser Arg
   225                 230                 235                 240
   Gly Ile Thr Gly Glu Asn Leu His Val Asp Ser Gly Phe His Ile Thr
                   245                 250                 255
   Ala Arg

## Description

### FIELD OF THE INVENTION

The invention relates to cells and methods for producing fatty acids, and more particularly relates to cells and methods for producing anteiso and/or iso branched-chain fatty acids.

### BACKGROUND OF THE INVENTION

Anteiso and iso branched-chain fatty acids are carboxylic acids with a methyl branch on the n-2 and n-1 carbon, respectively. Similar to other fatty acids, anteiso and iso branched-chain fatty acids are useful in manufacturing, such as, e.g., food, detergents, pesticides, and personal care products such as shampoos, soaps, and cosmetics.

Anteiso and iso branched-chain fatty acids can be chemically synthesized or can be isolated from certain animals and bacteria. While certain bacteria, such as *Escherichia coli,* do not naturally produce anteiso or iso branched-chain fatty acids, some bacteria, such as members of the genera *Bacillus* and *Streptomyces,* do naturally produce anteiso and iso branched-chain fatty acids. For example, *Streptomyces avermitilis* and *Bacillus subtilis* both produce anteiso fatty acids with 15 and 17 total carbons and iso branched fatty acids with 15, 16 and 17 total carbons (Cropp et al., Can. J. Microbiology 46: 506-14 (2000); De Mendoza et al., Biosynthesis and Function of Membrane Lipids, in Bacillus subtilis and Other Gram-Positive Bacteria, Sonenshein and Losick, eds., American Society for Microbiology, (1993)). In FEMS MICROLIOLOGY LETTERS, Vol. 301, No. 2, dated October 26, 2019, pages 188-192, a method for producing anteiso fatty acids in E.coli is discussed using FabH from Listeria. However, these organisms do not produce anteiso and/or iso branched-chain fatty acids in amounts that are commercially useful. Another limitation of these natural organisms is that they apparently do not produce medium-chain anteiso and/or iso branched-chain fatty acids, such as those with 11 or 13 carbons.

As such, there remains a need for commercially useful biosynthetically-produced anteiso and/or iso branched-chain fatty acids. In addition, there remains a need for a method of producing such anteiso and/or iso branched-chain fatty acids.

### SUMMARY OF THE INVENTION

Cells and methods for producing anteiso and/or iso branched-chain fatty acids (also referred to herein as anteiso and/or iso fatty acids) are provided. The polynucleotide comprises a nucleic acid sequence encoding a polypeptide that catalyzes a reaction associated with branched-chain fatty acid production in the cell.

In one aspect, the invention provides a method for producing anteiso fatty acid. The method comprises culturing a cell comprising at least one exogenous or overexpressed polynucleotide comprising a nucleic acid sequence encoding a polypeptide that catalyzes at least one of the following reactions: (aa) conversion of pyruvate to citramalate; (bb) conversion of citramalate to citraconate; (cc) conversion of citraconate to β-methyl-D-malate; (dd) conversion of β-methyl-D-malate to 2-oxobutanoate; or (ee) conversion of threonine to 2-oxobutanoate, under conditions allowing expression of the polynucleotide(s) and production of anteiso fatty acid. The cell produces more anteiso fatty acids than an otherwise similar cell that does not comprise the polynucleotide(s). In some embodiments, the cell further comprises at least one exogenous or overexpressed polynucleotide comprising a nucleic acid sequence encoding a polypeptide that catalyzes at least one of the following reactions: (ff) conversion of 2-oxobutanoate to 2-aceto-2-hydroxy-butyrate, (gg) conversion of 2-aceto-2-hydroxy-butyrate to 2,3-dihydroxy-3-methylvalerate, or (hh) conversion of 2,3-dihydroxy-3-methylvalerate to 2-keto-3-methylvalerate. Optionally, the method further comprises extracting anteiso fatty acid from the culture or extracting from the culture a product derived from anteiso fatty acid.

The invention also provides a cell comprising an exogenous or overexpressed polynucleotide comprising a nucleic acid sequence encoding a threonine deaminase, an exogenous or overexpressed polynucleotide comprising a nucleic acid sequence encoding a branched-chain α-keto acid dehydrogenase, and an exogenous or overexpressed polynucleotide comprising a nucleic acid sequence encoding a 3-ketoacyl-ACP synthase, wherein the polynucleotides are expressed in the cell. Additionally, the invention provides a cell comprising an exogenous or overexpressed polynucleotide comprising a nucleic acid sequence encoding a citramalate synthase, an exogenous or overexpressed polynucleotide comprising a nucleic acid sequence encoding a branched-chain α-keto acid dehydrogenase, and an exogenous or overexpressed polynucleotide comprising a nucleic acid sequence encoding a 3-ketoacyl-ACP synthase, wherein the polynucleotides are expressed in the cell. Optionally, the cell further comprises an exogenous or overexpressed polynucleotide comprising a nucleic acid sequence encoding an acetohydroxy acid synthase and/or an exogenous or overexpressed polynucleotide comprising a nucleic acid sequence encoding a thioesterase. A method of producing anteiso fatty acid by culturing the cell also is provided.

Disclosed herein is a cell having at least one exogenous polynucleotide. Alternatively or in addition, the cell comprises a polynucleotide that is overexpressed. The polynucleotide has a nucleic acid sequence encoding a polypeptide that catalyzes one of the following reactions: conversion of isoleucine to 2-keto, 3-methylvalerate; conversion of 2-keto, 3-methylvalerate to 2-methylbutyryl-CoA; conversion of 2-methylbutyryl-CoA to 2-methylbutyryl-ACP; conversion of 2-methylbutyryl-ACP to 4-methyl 3-ketohexanoyl-ACP; conversion of 2-methylbutyryl-CoA to 4-methyl 3-ketohexanoyl-ACP; or conversion of acyl-ACP to anteiso fatty acids. The cell comprising the exogenous polynucleotide produces more anteiso fatty acids than an otherwise similar cell that does not comprise the exogenous polynucleotide.

A method of increasing anteiso fatty acids in a bacterial cell is also disclosed. The method includes expressing in a bacterial cell a polynucleotide encoding a polypeptide that catalyzes one of the following reactions: conversion of 2-keto, 3-methylvalerate to 2-methylbutyryl-CoA; conversion of 2-methylbutyryl-CoA to 2-methylbutyryl-ACP; conversion of 2-methylbutyryl-ACP to 4-methyl 3-ketohexanoyl-ACP; conversion of 2-methylbutyryl-CoA to 4-methyl 3-ketohexanoyl-ACP; or conversion of acyl-ACP to anteiso fatty acids, and culturing the bacterial cell under conditions that allow the cell to produce the polypeptide such that anteiso fatty acids are produced.

Further disclosed is an *Escherichia coli* cell that produces anteiso fatty acids.

Also disclosed is a method of increasing anteiso fatty acids in a cell. The method includes expressing in a cell a polynucleotide encoding an exogenous branched-chain amino acid aminotransferase, an exogenous branched-chain α-keto acid dehydrogenase (BCDH), and an exogenous 3-ketoacyl-ACP synthase; and culturing the cell under conditions such that anteiso fatty acids are produced.

Also disclosed is a method for making anteiso fatty acids. The method includes culturing at least one cell comprising at least one exogenous polynucleotide that encodes at least one polypeptide that is capable of producing anteiso fatty acids from isoleucine under conditions such that anteiso fatty acids are produced.

In addition, a cell comprising at least two exogenous polynucleotides is also disclosed. The exogenous polynucleotides comprise nucleic acid sequences encoding polypeptides that catalyze at least two of the following reactions: conversion of leucine to 2-keto, 4-methylvalerate; conversion of valine to 2-keto 3-methybutyrate; conversion of 2-keto, 4-methylvalerate to 3-methylbutyryl-CoA; conversion of 3-methylbutyryl-CoA to 3-methylbutyryl-ACP; conversion of 3-methylbutyryl-ACP to 5-methyl 3-ketohexanoyl-ACP; conversion of 2-keto 3-methylbutyrate to 2-methylpropionyl-CoA; conversion of 2-methylpropionyl-CoA to 2-methylpropionyl-ACP; conversion of 2-methylpropionyl-ACP to 4-methylvaleroyl-ACP; conversion of 3-methylbutyryl-CoA to 5-methyl 3-ketohexanoyl-ACP; conversion of 2-methylpropionyl-CoA to 4-methyl 3-ketovaleroyl-ACP; or conversion of acyl-ACP to iso fatty acids, and wherein the cell comprising the exogenous polynucleotides produces more iso fatty acids than an otherwise similar cell that does not comprise the exogenous polynucleotides.

Also disclosed is a method for increasing iso fatty acids in a bacterial cell. The method includes expressing in a bacterial cell polynucleotides encoding at least two polypeptides, the polypeptides catalyzing at least two of the following reactions: conversion of leucine to 2-keto, 4-methylvalerate; conversion of valine to 2-keto 3-methybutyrate; conversion of 2-keto, 4-methylvalerate to 3-methylbutyryl-CoA; conversion of 3-methylbutyryl-CoA to 3-methylbutyryl-ACP; conversion of 3-methylbutyryl-ACP to 5-methyl 3-ketohexanoyl-ACP; conversion of 2-keto 3-methylbutyrate to 2-methylpropionyl-CoA; conversion of 2-methylpropionyl-CoA to 2-methylpropionyl-ACP; conversion of 2-methylpropionyl-ACP to 4-methylvaleroyl-ACP; conversion of 3-methylbutyryl-CoA to 5-methyl 3-ketohexanoyl-ACP; conversion of 2-methylpropionyl-CoA to 4-methyl 3-ketovaleroyl-ACP; or conversion of acyl-ACP to iso fatty acids, and culturing the bacterial cell under conditions that allow the cell to produce the polypeptides, such that iso fatty acids are produced.

The following numbered paragraphs each succinctly define one or more exemplary variations of the disclosure
1. A method for producing anteiso fatty acid, the method comprising culturing a cell comprising at least one exogenous or overexpressed polynucleotide comprising a nucleic acid sequence encoding a polypeptide that catalyzes at least one of the following reactions: (aa) conversion of pyruvate to citramalate; (bb) conversion of citramalate to citraconate; (cc) conversion of citraconate to β-methyl-D-malate; (dd) conversion of β-methyl-D-malate to 2-oxobutanoate; or (ee) conversion of threonine to 2-oxobutanoate under conditions allowing expression of the polynucleotide(s) and production of anteiso fatty acid, wherein the cell produces more anteiso fatty acids than an otherwise similar cell that does not comprise the polynucleotide(s).
2. The method of paragraph 1, further comprising exposing the cell to thiamine.
3. The method of paragraph 1, further comprising extracting anteiso fatty acid from the culture.
4. The method of paragraph 1, further comprising extracting from the culture a product derived from anteiso fatty acid.
5. The method of paragraph 1, wherein the cell further comprises at least one exogenous or overexpressed polynucleotide comprising a nucleic acid sequence encoding a polypeptide that catalyzes at least one of the following reactions: (ff) conversion of 2-oxobutanoate to 2-aceto-2-hydroxy-butyrate, (gg) conversion of 2-aceto-2-hydroxy-butyrate to 2,3-dihydroxy-3-methylvalerate, or (hh) conversion of 2,3-dihydroxy-3-methylvalerate to 2-keto-3-methylvalerate.
6. The method of paragraph 5, wherein the cell comprises exogenous or overexpressed polynucleotides encoding polypeptides that catalyze 3, 4, 5, 6, 7, or all of the reactions.
7. The method of paragraph 5, wherein the cell comprises exogenous or overexpressed polynucleotides encoding polypeptides that catalyze reactions (aa), (bb), (cc), and (ff).
8. The method of paragraph 5, wherein the cell comprises an exogenous or overexpressed polynucleotide encoding a citramalate synthase, an exogenous or overexpressed polynucleotide comprising a nucleic acid sequence encoding an acetohydroxy acid synthase, an exogenous or overexpressed polynucleotide comprising a nucleic acid sequence encoding an isopropylmalate isomerase, an exogenous or overexpressed polynucleotide comprising a nucleic acid sequence encoding an isopropylmalate dehydrogenase, or a combination thereof.
9. The method of paragraph 8, wherein the cell comprises an exogenous polynucleotide encoding a citramalate synthase, an exogenous or overexpressed polynucleotide encoding an acetohydroxy acid synthase, an exogenous or overexpressed polynucleotide encoding an isopropylmalate isomerase, and an exogenous or overexpressed polynucleotide encoding an isopropylmalate dehydrogenase.
10. The method of paragraph 8, wherein the citramalate synthase is CimA derived from *M*. *jannaschii.*
11. The method of paragraph 8, wherein the isopropylmalate isomerase is *E. coli* LeuCD.
12. The method of paragraph 8, wherein the isopropylmalate dehydrogenase is *E. coli* LeuB.
13. The method of paragraph 8, wherein the acetohydroxy acid synthase is *E. coli* IlvIH, *E. coli* IlvIH (G14D), *E. coli* IlvGM, or *B. subtilis* IlvBH.
14. The method of paragraph 5, wherein the cell comprises exogenous or overexpressed polynucleotides encoding polypeptides that catalyze reactions (ee) and (ff).
15. The method of paragraph 5, wherein the cell comprises an exogenous or overexpressed polynucleotide encoding a threonine deaminase and an exogenous or overexpressed polynucleotide encoding an acetohydroxy acid synthase.
16. The method of paragraph 15, wherein the threonine deaminase is *E. coli* TdcB.
17. The method of paragraph 15, wherein the acetohydroxy acid synthase is *E. coli* IlvIH, *E. coli* IlvIH (G14D), *E. coli* IlvGM, or *B. subtilis* IlvBH.
18. The method of paragraph 5, wherein the one or more of the exogenous or overexpressed polynucleotides (i) comprise a nucleic acid sequence having at least about 90 percent identity to the nucleic acid sequence set forth in SEQ ID NO: 32, 36, 42, 43, 46, 51, 57, 62, 68, or 83, or (ii) encode a polypeptide comprising an amino acid sequence having at least about 90 percent identity to the amino acid sequence set forth in SEQ ID NO: 33, 39, 40, 41, 47, 48, 52, 53, 58, 65, 66, 67, 84, or 85.
19. The method of paragraph 5, wherein the cell is modified to attenuate branched-chain amino acid aminotransferase activity.
20. The method of paragraph 5, wherein the cell further comprises an exogenous or overexpressed polynucleotide comprising a nucleic acid sequence encoding a branched-chain amino acid aminotransferase, an exogenous or overexpressed polynucleotide comprising a nucleic acid sequence encoding a branched-chain α-keto acid dehydrogenase, an exogenous or overexpressed polynucleotide comprising a nucleic acid sequence encoding an acyl transferase, an exogenous or overexpressed polynucleotide comprising a nucleic acid sequence encoding a 3-ketoacyl-ACP synthase, an exogenous or overexpressed polynucleotide comprising a nucleic acid sequence encoding an enoyl-ACP reductase, an exogenous or overexpressed polynucleotide comprising a nucleic acid sequence encoding a thioesterase, or a combination thereof.
21. The method of paragraph 20, wherein one or more of the exogenous or overexpressed polynucleotides comprise a nucleic acid sequence (i) having at least 90 percent identity to the nucleic acid sequence set forth in SEQ ID NO: 1, 4, 7, 13, 17, 18, 19, 20, 21, 22, 23, 68, 77, or 78 or (ii) encoding a polypeptide having an amino acid sequence having at least 90 percent identity to the amino acid sequence set forth in SEQ ID NO: 10, 16, 24, 25, 26, 27, 28, 29, or 73.
22. The method of paragraph 20, wherein the cell comprises an exogenous or overexpressed polynucleotide comprising a nucleic acid sequence encoding a branched-chain α-keto acid dehydrogenase and an exogenous or overexpressed polynucleotide comprising a nucleic acid sequence encoding a 3-ketoacyl-ACP synthase.
23. The method of paragraph 22, wherein the cell is an *Escherichia* cell.
24. The method of paragraph 22, wherein the branched-chain α-keto acid dehydrogenase is *B. subtilis* Bkd.
25. The method of paragraph 22, wherein the 3-ketoacyl-ACP synthase is *B. subtilis* FabH.
26. The method of paragraph 22, wherein the cell further comprises an exogenous or overexpressed polynucleotide comprising a nucleic acid sequence encoding a thioesterase.
27. A cell comprising: an exogenous or overexpressed polynucleotide comprising a nucleic acid sequence encoding a threonine deaminase, an exogenous or overexpressed polynucleotide comprising a nucleic acid sequence encoding a branched-chain α-keto acid dehydrogenase, and an exogenous or overexpressed polynucleotide comprising a nucleic acid sequence encoding a 3-ketoacyl-ACP synthase, wherein the polynucleotides are expressed and the cell produces more anteiso fatty acid than an otherwise similar cell that does not comprise the polynucleotide(s).
28. The cell of paragraph 27, wherein the branched-chain α-keto acid dehydrogenase is *B. subtilis* Bkd and the 3-ketoacyl-ACP synthase is *B. subtilis* FabH.
29. The cell of paragraph 27 further comprising an exogenous or overexpressed polynucleotide comprising a nucleic acid sequence encoding a thioesterase.
30. The cell of paragraph 27 further comprising an exogenous or overexpressed polynucleotide comprising a nucleic acid sequence encoding an acetohydroxy acid synthase.
31. The cell of paragraph 30, wherein the acetohydroxy acid synthase is *E. coli* IlvIH, *E. coli* IlvIH (G14D), *E. coli* IlvGM, or *B. subtilis* IlvBH.
32. The cell of paragraph 27, wherein the cell is a bacterial cell that does not naturally produce anteiso fatty acids
33. The cell of paragraph 32, wherein the cell is an *Escherichia* cell.
34. A method of producing anteiso fatty acid, the method comprising culturing the bacterial cell of paragraph 32 under conditions that allow expression of the polynucleotides and production of anteiso fatty acid.
35. A cell comprising: an exogenous or overexpressed polynucleotide comprising a nucleic acid sequence encoding a citramalate synthase, an exogenous or overexpressed polynucleotide comprising a nucleic acid sequence encoding a branched-chain α-keto acid dehydrogenase, and an exogenous or overexpressed polynucleotide comprising a nucleic acid sequence encoding a 3-ketoacyl-ACP synthase, wherein the polynucleotides are expressed and the cell produces more anteiso fatty acid than an otherwise similar cell that does not comprise the polynucleotide(s).
36. The cell of paragraph 35, wherein the citramalate synthase is CimA derived from *M. jannaschii,* the branched-chain α-keto acid dehydrogenase is *B. subtilis* Bkd, and the 3-ketoacyl-ACP synthase is *B. subtilis* FabH.
37. The cell of paragraph 35 further comprising an exogenous or overexpressed polynucleotide comprising a nucleic acid sequence encoding an isopropylmalate isomerase, an exogenous or overexpressed polynucleotide comprising a nucleic acid sequence encoding an isopropylmalate dehydrogenase, an exogenous or overexpressed polynucleotide comprising a nucleic acid sequence encoding an acetohydroxy acid synthase, an exogenous or overexpressed polynucleotide comprising a nucleic acid sequence encoding an enoyl-ACP synthase, an exogenous or overexpressed polynucleotide comprising a nucleic acid sequence encoding a thioesterase, or a combination thereof.
38. The method of paragraph 37, wherein the isopropylmalate isomerase is *E. coli* LeuCD.
39. The method of paragraph 37, wherein the isopropylmalate dehydrogenase is *E*. *coli* LeuB.
40. The cell of paragraph 37, wherein the acetohydroxy acid synthase is *E. coli* IlvIH, *E. coli* IlvIH (G14D), *E. coli* IlvGM, or *B. subtilis* IlvBH.
41. The cell of paragraph 35, wherein the cell is a bacterial cell that does not naturally produce anteiso fatty acids.
42. The cell of paragraph 41, wherein the cell is an *Escherichia* cell.
43. A method of producing anteiso fatty acid, the method comprising culturing a bacterial cell of paragraph 41 under conditions that allow expression of the polynucleotides and production of anteiso fatty acid.
44. A cell comprising at least one exogenous polynucleotide, wherein the polynucleotide comprises a nucleic acid sequence encoding a polypeptide that catalyzes one of the following reactions: a. conversion of isoleucine to 2-keto, 3-methylvalerate; b. conversion of 2-keto, 3-methylvalerate to 2-methylbutyryl-CoA; c. conversion of 2-methylbutyryl-CoA to 2-methylbutyryl-ACP; d. conversion of 2-methylbutyryl-ACP to 4-methyl 3-ketohexanoyl-ACP; e. conversion of 2-methylbutyryl-CoA to 4-methyl 3-ketohexanoyl-ACP; or f. conversion of acyl-ACP to anteiso fatty acids, and wherein the cell comprising the exogenous polynucleotide produces more anteiso fatty acids than an otherwise similar cell that does not comprise the exogenous polynucleotide.
45. The cell of paragraph 44, wherein the polynucleotide encodes a branched-chain amino acid aminotransferase, a branched-chain α-keto acid dehydrogenase (BCDH), an acyl transferase, a 3-ketoacyl-ACP synthase, or a thioesterase.
46. The cell of paragraph 44, wherein the cell is an *Escherichia* cell.
47. The cell of paragraph 44, wherein the cell comprising polynucleotides encoding polypeptides that catalyze 2, 3, 4, 5, or all of the reactions.
48. The cell of paragraph 44, wherein the polynucleotide has at least 30 percent sequence identity to a sequence set forth in SEQ ID NO: 1, 4, 7, 13, 17, 18, 19, 20, 21, 22, or 23.
49. The cell of paragraph 44, wherein the polypeptide has at least 40 percent sequence identity to a sequence set forth in SEQ ID NO: 10, 16, 24, 25, 26, 27, 28, or 29.
50. The cell of paragraph 44, wherein the cell is an *Escherichia* coli cell and the polynucleotide has at least 65 percent sequence identity to a sequence set forth in SEQ ID NO: 1, 4, 7, 13, 17, 18, 19, 20, 21, 22, or 23.
51. The cell of paragraph 44, wherein the polypeptide is substantially identical to a polypeptide having the sequence set forth in SEQ ID NO: 10, 16, 24, 25, 26, 27, 28, or 29.
52. The cell of paragraph 44, wherein the anteiso fatty acids are medium-chain anteiso fatty acids.
53. The cell of paragraph 44, wherein the anteiso fatty acids are not naturally produced in the cell.
54. Anteiso fatty acids produced by the cell of paragraph 44.
55. The cell of paragraph 44, wherein the polynucleotide encodes a fatty acid synthase gene from a *Bacillus, a Streptomyces,* or a *Listeria.*
56. The cell of paragraph 44, wherein the polynucleotide encodes a fatty acid synthase gene from an organism that naturally produces branched-chain fatty acids.
57. A method of increasing anteiso fatty acids in a bacterial cell, comprising:
   a. expressing in a bacterial cell a polynucleotide encoding a polypeptide that catalyzes one of the following reactions: i. conversion of 2-keto, 3-methylvalerate to 2-methylbutyryl-CoA; ii. conversion of 2-methylbutyryl-CoA to 2-methylbutyryl-ACP; iii. conversion of 2-methylbutyryl-ACP to 4-methyl 3-ketohexanoyl-ACP; iv. conversion of 2-methylbutyryl-CoA to 4-methyl 3-ketohexanoyl-ACP; or v. conversion of acyl-ACP to anteiso fatty acids, and
   b. culturing the bacterial cell under conditions that allow the cell to produce the polypeptide, such that anteiso fatty acids are produced.
58. The method of paragraph 57, wherein the cell produces higher levels of anteiso fatty acids after expression of the polynucleotide than it did prior to expression of the polynucleotide.
59. The method of paragraph 57, wherein the polypeptide is a branched-chain amino acid aminotransferase, a branched-chain α-keto acid dehydrogenase (BCDH), a 3-ketoacyl-ACP synthase, or a thioesterase.
60. The method of paragraph 57, wherein the cell is an *Escherichia* cell.
61. The method of paragraph 57, wherein the method includes expressing in the bacterial cell polynucleotides encoding polypeptides that catalyze 2, 3, 4, 5, or all of the reactions.
62. The method of paragraph 57, wherein the polynucleotide has at least 30 percent sequence identity to a sequence set forth in SEQ ID NO: 1, 4, 7, 13, 17, 18, 19, 20, 21, 22, or 23.
63. The method of paragraph 57, wherein the polypeptide has at least 40 percent sequence identity to a sequence set forth in SEQ ID NO: 10, 16, 24, 25, 26, 27, 28, or 29.
64. The method of paragraph 57, wherein the cell is an *Escherichia coli* cell and the polynucleotide has at least 65 percent sequence identity to a sequence set forth in SEQ ID NO: 1, 4, 7, 13, 17, 18, 19, 20, 21, 22, or 23.
65. The method of paragraph 57, wherein the polypeptide is substantially identical to a polypeptide having the sequence set forth in SEQ ID NO: 10, 16, 24, 25, 26, 27, 28, or 29.
66. The method of paragraph 57, wherein the anteiso fatty acids are medium chain anteiso fatty acids.
67. The method of paragraph 57, wherein the anteiso fatty acids are not naturally produced in the cell.
68. Anteiso fatty acids produced by the method of paragraph 57.
69. An *Escherichia coli* cell that produces anteiso fatty acids.
70. The cell of paragraph 69, wherein the anteiso fatty acids are medium chain fatty acids.
71. The cell of paragraph 69, wherein the cell comprises a polynucleotide with at least 30 percent sequence identity to a sequence set forth in SEQ ID NO: 1, 4, 7, 13, 17, 18, 19, 20, 21, 22, or 23.
72. Anteiso fatty acids produced by the cell of paragraph 69.
73. A method of increasing anteiso fatty acids in a cell, comprising:
   a. expressing in a cell one or more polynucleotide encoding an exogenous branched-chain amino acid aminotransferase, an exogenous branched-chain α-keto acid dehydrogenase (BCDH), and an exogenous 3-ketoacyl-ACP synthase;
   b. culturing the cell under conditions such that anteiso fatty acids are produced.
74. The method of paragraph 73, wherein the method further includes expressing in the cell a polynucleotide encoding an exogenous thioesterase.
75. The method of paragraph 73, wherein the polynucleotide has at least 30 percent sequence identity to a sequence set forth in SEQ ID NO: 1, 4, 7, 13, 17, 18, 19, 20, 21, 22, or 23.
76. The method of paragraph 73, wherein the polynucleotide encodes a polypeptide having at least 40 percent sequence identity to a sequence set forth in SEQ ID NO: 10, 16, 24, 25, 26, 27, 28, or 29.
77. The method of paragraph 73, wherein the cell is an *Escherichia coli* cell and the polynucleotide has at least 65 percent sequence identity to a sequence set forth in SEQ ID NO: 1, 4, 7, 13, 17, 18, 19, 20, 21, 22, or 23.
78. The method of paragraph 76, wherein the polypeptide is substantially identical to a polypeptide having the sequence set forth in SEQ ID NO: 10, 16, 24, 25, 26, 27, 28, or 29.
79. The method of paragraph 73, wherein the anteiso fatty acids are medium chain anteiso fatty acids.
80. The method of paragraph 73, wherein the anteiso fatty acids are not naturally produced in the cell.
81. Anteiso fatty acids produced by the method of paragraph 73.
82. A method for making anteiso fatty acids, the method comprising culturing at least one cell comprising at least one exogenous polynucleotide that encodes at least one polypeptide that is capable of producing anteiso fatty acids from isoleucine under conditions such that anteiso fatty acids are produced.
83. The method of paragraph 82, wherein the cell is an *Escherichia coli* cell.
84. The method of paragraph 82, wherein the anteiso fatty acids are not naturally produced in the cell.
85. Anteiso fatty acids produced by the method of paragraph 82.
86. A cell comprising at least two exogenous polynucleotides, wherein the exogenous polynucleotides comprise nucleic acid sequences encoding polypeptides that catalyze at least two of the following reactions: a. conversion of leucine to 2-keto, 4-methylvalerate; b. conversion of valine to 2-keto 3-methybutyrate; c. conversion of 2-keto, 4-methylvalerate to 3-methylbutyryl-CoA; d. conversion of 3-methylbutyryl-CoA to 3-methylbutyryl-ACP; e. conversion of 3-methylbutyryl-ACP to 5-methyl 3-ketohexanoyl-ACP; f. conversion of 2-keto 3-methylbutyrate to 2-methylpropionyl-CoA; g. conversion of 2-methylpropionyl-CoA to 2-methylpropionyl-ACP; h. conversion of 2-methylpropionyl-ACP to 4-methylvaleroyl-ACP; i. conversion of 3-methylbutyryl-CoA to 5-methyl 3-ketohexanoyl-ACP; j. conversion of 2-methylpropionyl-CoA to 4-methyl 3-ketovaleroyl-ACP; or k. conversion of acyl-ACP to iso fatty acids, and wherein the cell comprising the exogenous polynucleotides produces more iso fatty acids than an otherwise similar cell that does not comprise the exogenous polynucleotides.
87. The cell of paragraph 86, wherein the polynucleotides encode a branched-chain amino acid aminotransferase, a branched-chain α-keto acid dehydrogenase (BCDH), an acyl transferase, a 3-ketoacyl-ACP synthase, or a thioesterase.
88. The cell of paragraph 86, wherein the cell is an *Escherichia* cell.
89. The cell of paragraph 86, wherein the polynucleotides comprise nucleic acid sequences encoding polypeptides that catalyze 3, 4, 5, 6, 7, 8, 9, 10, or all of the reactions.
90. The cell of paragraph 86, wherein the polynucleotide has at least 30 percent sequence identity to a sequence set forth in SEQ ID NO: 1, 4, 7, 13, 17, 18, 19, 20, 21, 22, or 23.
91. The cell of paragraph 86, wherein the polypeptide has at least 40 percent sequence identity to a sequence set forth in SEQ ID NO: 10, 16, 24, 25, 26, 27, 28, or 29.
92. The cell of paragraph 86, wherein the cell is an *Escherichia coli* cell and the polynucleotide has at least 65 percent sequence identity to a sequence set forth in SEQ ID NO: 1, 4, 7, 13, 17, 18, 19, 20, 21, 22, or 23.
93. The cell of paragraph 86, wherein the polypeptide is substantially identical to a polypeptide having the sequence set forth in SEQ ID NO: 10, 16, 24, 25, 26, 27, 28, or 29.
94. The cell of paragraph 86, wherein the iso fatty acids are medium-chain iso fatty acids.
95. The cell of paragraph 86, wherein the iso fatty acids are not naturally produced in the cell.
96. Iso fatty acids produced by the cell of paragraph 86.
97. The cell of paragraph 86, wherein the polynucleotide encodes a fatty acid synthase gene from a *Bacillus, a Streptomyces,* or a *Listeria.*
98. The cell of paragraph 86, wherein the polynucleotide encodes a fatty acid synthase gene from an organism that naturally produces branched-chain fatty acids.
99. A method of increasing iso fatty acids in a bacterial cell, comprising:
   a. expressing in a bacterial cell polynucleotides encoding at least two polypeptides, the polypeptides catalyzing at least two of the following reactions: i. conversion of leucine to 2-keto, 4-methylvalerate; ii. conversion of valine to 2-keto 3-methybutyrate; iii. conversion of 2-keto, 4-methylvalerate to 3-methylbutyryl-CoA; iv. conversion of 3-methylbutyryl-CoA to 3-methylbutyryl-ACP; v. conversion of 3-methylbutyryl-ACP to 5-methyl 3-ketohexanoyl-ACP; vi. conversion of 2-keto 3-methylbutyrate to 2-methylpropionyl-CoA; vii. conversion of 2-methylpropionyl-CoA to 2-methylpropionyl-ACP; viii. conversion of 2-methylpropionyl-ACP to 4-methylvaleroyl-ACP; ix. conversion of 3-methylbutyryl-CoA to 5-methyl 3-ketohexanoyl-ACP; x. conversion of 2-methylpropionyl-CoA to 4-methyl 3-ketovaleroyl-ACP; and xi. conversion of acyl-ACP to iso fatty acids, and
   b. culturing the bacterial cell under conditions that allow the cell to produce the polypeptides, such that iso fatty acids are produced.
100. A method of increasing accumulation of anteiso fatty acids in the culture medium by using a host strain unable to degrade fatty acids.
101. The host strain of paragraph 100, wherein the organism is *E. coli.*
102. The host strain of paragraph 100, wherein the organism is a *fadD* mutant of *E*. *coli.*
103. A method of increasing production of anteiso fatty acids in a cell, comprising: a. expressing in the cell a polynucleotide encoding a polypeptide having one of the following activities: citramalate synthase, isopropylmalate isomerase, and/or isopropylmalate dehydrogenase, and b. culturing the cell under conditions that allow the cell to produce the polypeptides, such that anteiso fatty acids are produced.
104. The method of paragraph 103, wherein the method includes expressing in the cell polynucleotides encoding polypeptides that have 2 or 3 of the activities.
105. A method for increasing production of anteiso fatty acids in a cell, comprising: a. expressing in the cell a polynucleotide encoding least one of *ilvA, tdcB, ilvI, ilvH, ilvC, and*/*or ilvD,* and b. culturing the cell under conditions that allow the cell to produce the polypeptides encoded by the polynucleotide, such that anteiso fatty acids are produced.
106. A method of increasing iso and/or anteiso fatty acid production in a cell, comprising: a. expressing in the cell a polynucleotide encoding a polypeptide having acetyl-CoA carboxylase activity, and b. culturing the cell under conditions that allow the cell to produce the polypeptides, such that iso and/or anteiso fatty acids are produced.

### BRIEF DESCRIPTION OF THE DRAWINGS

Figure 1 is a diagram for anteiso and iso branched-chain fatty acid biosynthesis pathway.
Figure 2 is a diagram for a threonine-dependent anteiso fatty acid biosynthesis pathway.

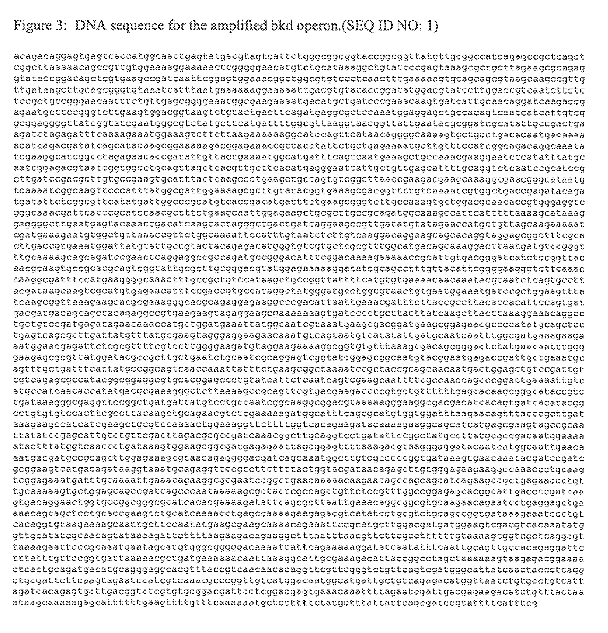

Figure 3 is the DNA sequence for the amplified *bkd* operon (SEQ ID NO: 1).

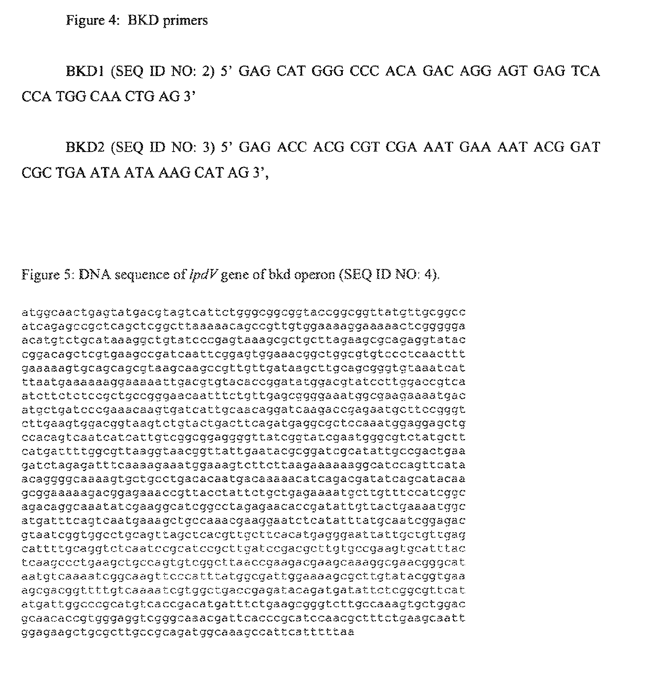

Figure 4 is the sequences for the *bkd* primers (SEQ ID NO: 2, 3).
Figure 5 is the DNA sequence of the *lpdV* gene of bkd operon (SEQ ID NO: 4).

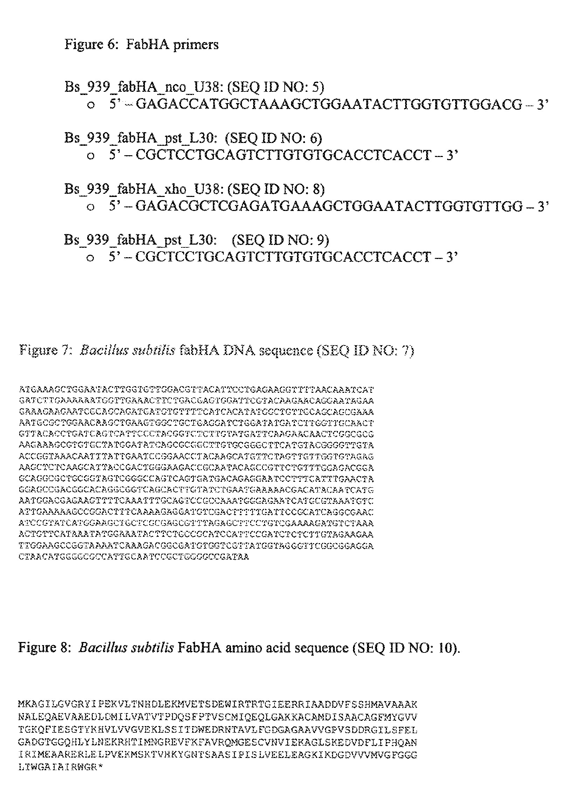

Figure 6 is the sequences for the *fabHA* primers (SEQ ID NO: 5, 6, 8, 9).
Figure 7 is the *Bacillus subtilis fabHA* DNA sequence (SEQ ID NO: 7).
Figure 8 is the *Bacillus subtilis* FabHA amino acid sequence (SEQ ID NO: 10).

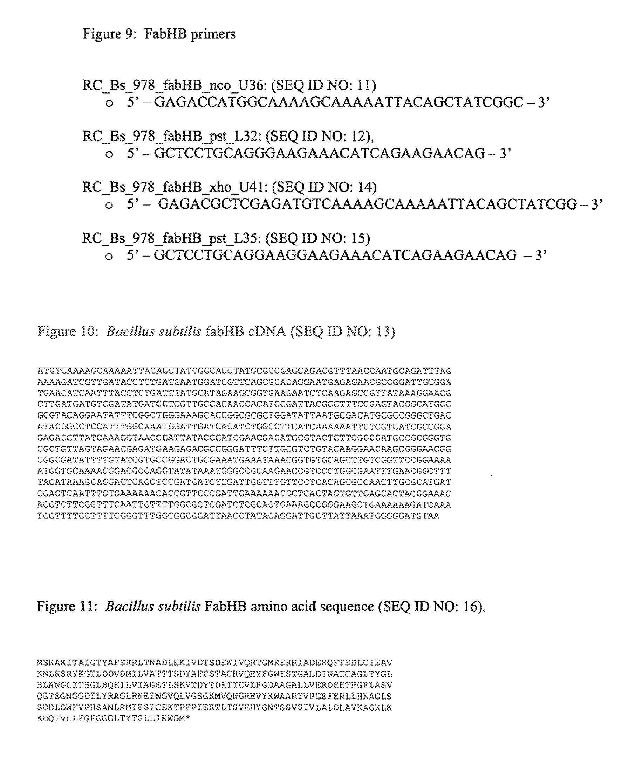

Figure 9 is the sequences for the *fabHB* primers (SEQ ID NO: 11, 12, 14, and 15).
Figure 10 is the sequence for the *Bacillus subtilis fabHB* DNA (SEQ ID NO: 13).
Figure 11 is the *Bacillus subtilis* FabHB amino acid sequence (SEQ ID NO: 16).

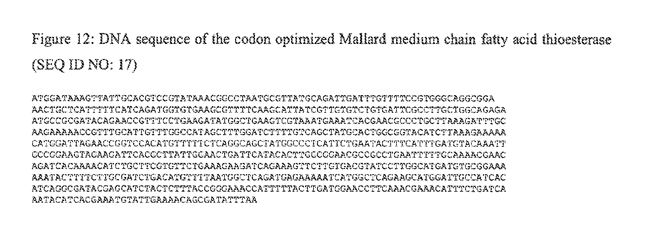

Figure 12 is the DNA sequence of the codon-optimized Mallard medium chain fatty acid thioesterase gene (SEQ ID NO: 17).
Figure 13 is an alignment of the optimized open reading frame (ORF) (SEQ ID NO: 17) with the original Mallard medium chain fatty acid thioesterase sequence (SEQ ID NO: 18).

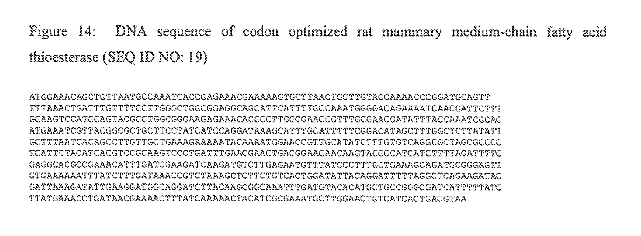

Figure 14 is the DNA sequence of a codon-optimized rat mammary medium-chain fatty acid thioesterase gene (SEQ ID NO: 19).
Figure 15 is an alignment of the optimized ORF (SEQ ID NO: 19) with the original rat mammary medium-chain fatty acid thioesterase (SEQ ID NO: 20).
Figure 16 is a graph showing the effect of isoleucine supplementation on anteiso fatty acid production.
Figure 17 is a diagram of a threonine-independent anteiso fatty acid synthesis pathway.

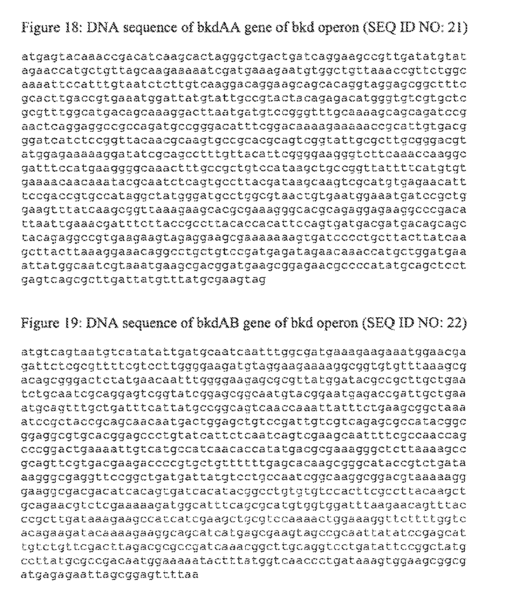

Figure 18 is the DNA sequence of *bkdAA* gene of bkd operon (SEQ ID NO: 21)
Figure 19 is the DNA sequence of *bkdAB* gene of bkd operon (SEQ ID NO: 22)

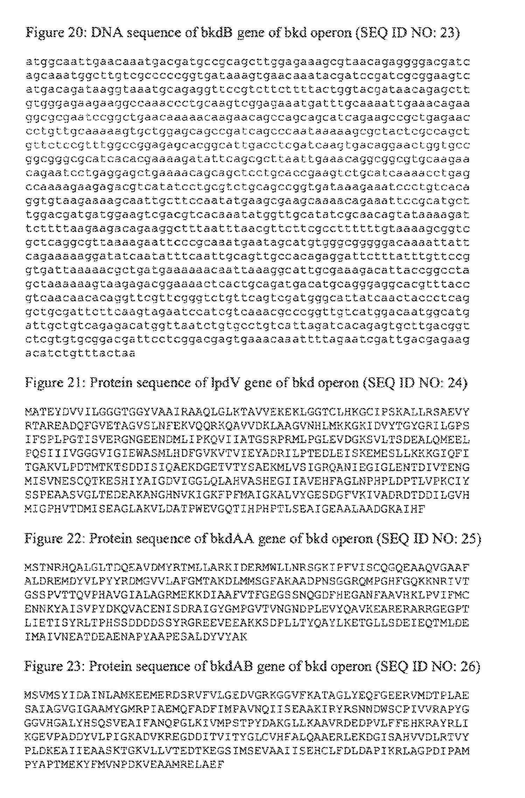

Figure 20 is the DNA sequence of *bkdB* gene of bkd operon (SEQ ID NO: 23)
Figure 21 is the protein sequence of *lpdV* gene of bkd operon (SEQ ID NO: 24)
Figure 22 is the protein sequence of *bkdAA* gene of bkd operon (SEQ ID NO: 25)
Figure 23 is the protein sequence of *bkdAB* gene of bkd operon (SEQ ID NO: 26)

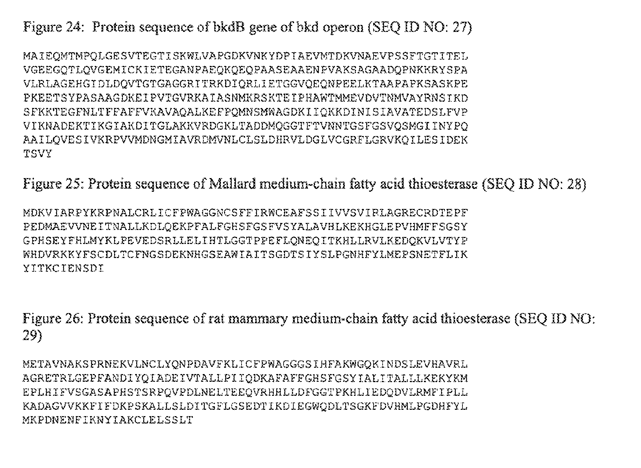

Figure 24 is the protein sequence of *bkdB* gene of bkd operon (SEQ ID NO: 27)
Figure 25 is the protein sequence of Mallard medium-chain fatty acid thioesterase (SEQ ID NO: 28)
Figure 26 is the protein sequence of rat mammary medium-chain fatty acid thioesterase

### (SEQ ID NO: 29)

Figure 27 is a bar graph illustrating C15 anteiso fatty acid production (fraction of a-C15 anteiso fatty acids in the total pool of synthesized fatty acids; y-axis) in *E*. *coli* strains K27-Z1 (parental strain), K27-Z1 (*Bs bkd Bs fabH*), K27-Z1 (*Bs bkd Bs fabH Ec tcdB*), and K27-Z1 (*Bs bkd Bs fabH Ec tdcB Ec ilvlH*(G14D)) (x-axis). Cultures were prepared in triplicate, with standard deviation of fatty acid measurements indicated by error bars.

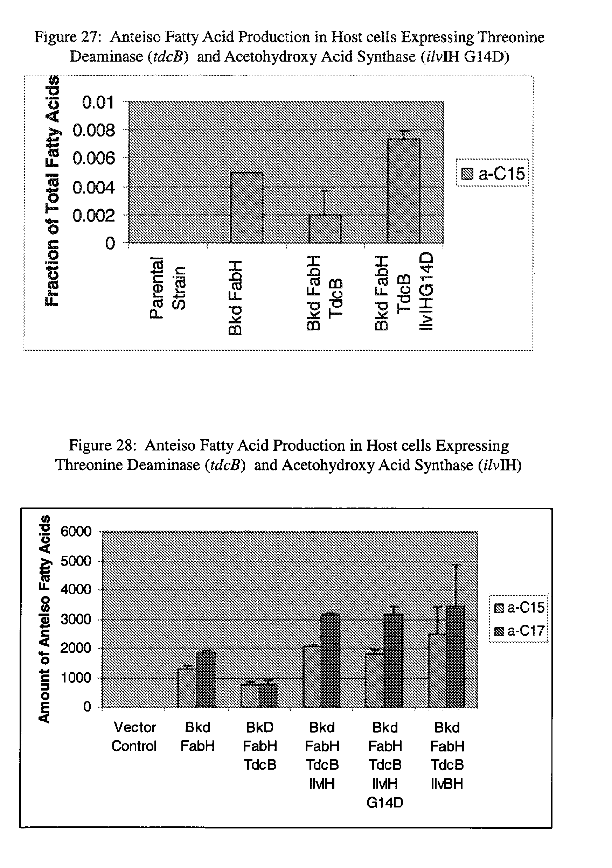

Figure 28 is a bar graph illustrating C15 and C17 anteiso fatty acid production in *E*. *coli* K27-Z1 derivative strains expressing different AHAS genes. The K27-Z1 derivative strains (x-axis) comprised the following plasmids and genes: (i) pTrcHisA and pZA31MCS (Vector Control); (ii) *Bs bkd Bs fabHA* pTrcHisA; (iii) *Bs bkd fabHA Ec tdcB;* (iv) *Bs bkd fabHA Ec tdcB Ec ilvIH;* (v) *Bs bkd fabHA Ec tdcB Ec ilvIH*(G14D); and (vi) *Bs bkd Bs fabHA Ec tdcB Bs ilvBH.* The peak area from gas chromatography analysis is represented on the y-axis. One biological replicate is represented with duplicate fatty acid analysis.

Figure 29 is a bar graph illustrating C15 anteiso fatty acid production in *E. coli* BL21 Star (DE3) derivatives.

Figure 30 is a bar graph illustrating C15 and C17 anteiso fatty acid production in the following *E. coli* derivative strains: K27-Z1 (pTrcHisA pZA31 MCS (vector control)), K27-Z1 (*Bs bkd Bs fabHA*), K27-Z1 (*Bs bkd Bs fabHA Ec tdcB*), and K27-Z1 (*Bs bkd Bs fabHA Ec tdcB Ec ilvGM*). One biological replicate was tested with duplicate fatty acid analysis; standard deviations are indicated by error bars.

Figure 31 is a bar graph illustrating C15 and C17 anteiso fatty acid production in *E*. *coli* K27-Z1 derivatives designed to produce the indicated recombinant proteins: Bkd-FabHA, Bkd-FabHA-CimA-LeuBCD, Bkd-FabHA-CimA-LeuBCD-IlvIH, Bkd-FabHA-CimA-LeuBCD-IlvIH(GI4D), Bkd-FabHA-CimA-LeuBCD-IlvBH, and Bkd-FabHA-CimA-LeuBCD-IlvGM.

Figure 32 is a bar graph illustrating C13, C15, and C17 anteiso fatty acid production in *E*. *coli* K27-Z1 (*Bs bkd Bs fabH*) and *E*. *coli* K27-Z1 (*Bs bkd Bs fabHA Ec 'tesA*).

Figure 33 is a bar graph illustrating C13, C15, and C17 anteiso fatty acid production in *E*. *coli* BL21 Star (DE3) (*Bs bkd Bs fabHA*) and BL21 Star (DE3) (*Bs bkd Bs fabHA Ec 'tesA*).

Figure 34 is a bar graph illustrating C15 and C17 anteiso fatty acid production in *E. coli* cultured in the presence and absence of thiamine. The *E*. *coli* derivatives were designed to produce the indicated recombinant proteins. Duplicate samples are indicated by "#2." The presence of thiamine in the culture medium improved anteiso fatty acid production.

Figure 35 is a bar graph illustrating C15 and C17 anteiso fatty acid production in an *E*. *coli ilvE* deletion strain (*Bs bkd Bs fabHA Ec tdcB Ec ilvIH*(G14D)) and a control *ilvE* deletion strain (pZA31 MCS pTrcHisA).

Figure 36 is a bar graph illustrating anteiso and iso branched-chain fatty acid production in *E*. *coli* BW25113 derivatives harboring polynucleotides encoding *Listeria monocytogenes* FabH and *B. subtilis* Bkd.

### DETAILED DESCRIPTION OF THE INVENTION

The invention relates to biologically produced anteiso and/or iso branched-chain fatty acids and improved biological production of such anteiso and/or iso branched-chain fatty acids. This improved biological production can, in certain embodiments, provide higher yields of anteiso and/or iso branched-chain fatty acids. In addition, or alternatively, the invention provides the ability to tailor the chain length of the anteiso and/or iso branched-chain fatty acids to a desired chain length.

As used herein, "amplify," "amplified," or "amplification" refers to any process or protocol for copying a polynucleotide sequence into a larger number of polynucleotide molecules, e.g., by reverse transcription, polymerase chain reaction, and ligase chain reaction.

As used herein, an "antisense sequence" refers to a sequence that specifically hybridizes with a second polynucleotide sequence. For instance, an antisense sequence is a DNA sequence that is inverted relative to its normal orientation for transcription. Antisense sequences can express an RNA transcript that is complementary to a target mRNA molecule expressed within the host cell (e.g., it can hybridize to target mRNA molecule through Watson-Crick base pairing).

As used herein, "cDNA" refers to a DNA that is complementary or identical to an mRNA, in either single stranded or double stranded form.

As used herein, "complementary" refers to a polynucleotide that base pairs with a second polynucleotide. Put another way, "complementary" describes the relationship between two single-stranded nucleic acid sequences that anneal by base-pairing. For example, a polynucleotide having the sequence 5'-GTCCGA-3' is complementary to a polynucleotide with the sequence 5'-TCGGAC-3'.

As used herein, a "conservative substitution" refers to the substitution in a polypeptide of an amino acid with a functionally similar amino acid. Put another way, a conservative substitution involves replacement of an amino acid residue with an amino acid residue having a similar side chain. Families of amino acid residues having similar side chains have been defined within the art, and include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), beta-branched side chains (e.g., threonine, valine, and isoleucine) and aromatic side chains (e.g., tyrosine, phenylalanine, tryptophan, and histidine).

As used herein, "encoding" refers to the inherent property of nucleotides to serve as templates for synthesis of other polymers and macromolecules. Unless otherwise specified, a "nucleotide sequence encoding an amino acid sequence" includes all nucleotide sequences that are degenerate versions of each other and that encode the same amino acid sequence.

As used herein, "endogenous" refers to polynucleotides, polypeptides, or other compounds that are expressed naturally or originate within an organism or cell. That is, endogenous polynucleotides, polypeptides, or other compounds are not exogenous. For instance, an "endogenous" polynucleotide or peptide is present in the cell when the cell was originally isolated from nature.

As used herein, "expression vector" refers to a vector comprising a recombinant polynucleotide comprising expression control sequences operatively linked to a nucleotide sequence to be expressed. For example, suitable expression vectors can be an autonomously replicating plasmid or integrated into the chromosome. An expression vector also can be a viral-based vector.

As used herein, "exogenous" refers to any polynucleotide or polypeptide that is not naturally expressed in the particular cell or organism where expression is desired. Exogenous polynucleotides, polypeptides, or other compounds are not endogenous.

As used herein, "hybridization" includes any process by which a strand of a nucleic acid joins with a complementary nucleic acid strand through base-pairing. Thus, the term refers to the ability of the complement of the target sequence to bind to a test (i.e., target) sequence, or vice-versa.

As used herein, "hybridization conditions" are typically classified by degree of "stringency" of the conditions under which hybridization is measured. The degree of stringency can be based, for example, on the melting temperature (Tₘ) of the nucleic acid binding complex or probe. For example, "maximum stringency" typically occurs at about Tₘ -5° C (5° below the Tₘ of the probe); "high stringency" at about 5-10° below the Tₘ; "intermediate stringency" at about 10-20° below the Tₘ of the probe; and "low stringency" at about 20-25° below the Tₘ. Alternatively, or in addition, hybridization conditions can be based upon the salt or ionic strength conditions of hybridization and/or one or more stringency washes. For example, 6xSSC=very low stringency; 3xSSC=low to medium stringency; lxSSC=medium stringency; and 0.5xSSC=high stringency. Functionally, maximum stringency conditions may be used to identify nucleic acid sequences having strict (i.e., about 100%) identity or near-strict identity with the hybridization probe; while high stringency conditions are used to identify nucleic acid sequences having about 80% or more sequence identity with the probe.

As used herein, "identical" or percent "identity," in the context of two or more polynucleotide or polypeptide sequences, refers to two or more sequences that are the same or have a specified percentage of nucleotides or amino acid residues that are the same, when compared and aligned for maximum correspondence, as measured using sequence comparison algorithms or by visual inspection.

As used herein, "long-chain fatty acids" refers to fatty acids with aliphatic tails longer than 14 carbons.

As used herein, "medium-chain fatty acids" refers to fatty acids with aliphatic tails between 6 and 14 carbons. In certain embodiments, the medium-chain fatty acids can have from 11 to 13 carbons.

As used herein, "naturally-occurring" refers to an object that can be found in nature. For example, a polypeptide or polynucleotide sequence that is present in an organism (including viruses) that can be isolated from a source in nature and which has not been intentionally modified by man in the laboratory is naturally-occurring.

As used herein, "operably linked," when describing the relationship between two DNA regions or two polypeptide regions, means that the regions are functionally related to each other. For example, a promoter is operably linked to a coding sequence if it controls the transcription of the sequence; a ribosome binding site is operably linked to a coding sequence if it is positioned so as to permit translation; and a sequence is operably linked to a peptide if it functions as a signal sequence, such as by participating in the secretion of the mature form of the protein.

As used herein, "overexpression" refers to expression of a polynucleotide to produce a product (e.g., a polypeptide or RNA) at a higher level than the polynucleotide is normally expressed in the host cell. An overexpressed polynucleotide is a polynucleotide native to the host cell, the product of which is generated in a greater amount than that normally found in the host cell. Overexpression is achieved by, for instance and without limitation, operably linking the polynucleotide to a different promoter than the polynucleotide's native promoter or introducing additional copies of the polynucleotide into the host cell.

As used herein, "polynucleotide" refers to a polymer composed of nucleotides. The polynucleotide may be in the form of a separate fragment or as a component of a larger nucleotide sequence construct, which has been derived from a nucleotide sequence isolated at least once in a quantity or concentration enabling identification, manipulation, and recovery of the sequence and its component nucleotide sequences by standard molecular biology methods, for example, using a cloning vector. When a nucleotide sequence is represented by a DNA sequence (i.e., A, T, G, C), this also includes an RNA sequence (i.e., A, U, G, C) in which "U" replaces "T." Put another way, "polynucleotide" refers to a polymer of nucleotides removed from other nucleotides (a separate fragment or entity) or can be a component or element of a larger nucleotide construct, such as an expression vector or a polycistronic sequence. Polynucleotides include DNA, RNA and cDNA sequences.

As used herein, "polypeptide" refers to a polymer composed of amino acid residues which may or may not contain modifications such as phosphates and formyl groups.

As used herein, "recombinant expression vector" refers to a DNA construct used to express a polynucleotide that, e.g., encodes a desired polypeptide. A recombinant expression vector can include, for example, a transcriptional subunit comprising (i) an assembly of genetic elements having a regulatory role in gene expression, for example, promoters and enhancers, (ii) a structural or coding sequence which is transcribed into mRNA and translated into protein, and (iii) appropriate transcription and translation initiation and termination sequences. Recombinant expression vectors are constructed in any suitable manner. The nature of the vector is not critical, and any vector may be used, including plasmid, virus, bacteriophage, and transposon. Possible vectors for use in the invention include, but are not limited to, chromosomal, nonchromosomal and synthetic DNA sequences, e.g., bacterial plasmids; phage DNA; yeast plasmids; and vectors derived from combinations of plasmids and phage DNA, DNA from viruses such as vaccinia, adenovirus, fowl pox, baculovirus, SV40, and pseudorabies.

As used herein, "primer" refers to a polynucleotide that is capable of specifically hybridizing to a designated polynucleotide template and providing a point of initiation for synthesis of a complementary polynucleotide when the polynucleotide primer is placed under conditions in which synthesis is induced. As used herein, "recombinant polynucleotide" refers to a polynucleotide having sequences that are not naturally joined together. A recombinant polynucleotide may be included in a suitable vector, and the vector can be used to transform a suitable host cell. A host cell that comprises the recombinant polynucleotide is referred to as a "recombinant host cell." The polynucleotide is then expressed in the recombinant host cell to produce, e.g., a "recombinant polypeptide."

As used herein, "specific hybridization" refers to the binding, duplexing, or hybridizing of a polynucleotide preferentially to a particular nucleotide sequence under stringent conditions.

As used herein, "stringent conditions" refers to conditions under which a probe will hybridize preferentially to its target subsequence, and to a lesser extent to, or not at all to, other sequences.

As used herein, "short chain fatty acids" refers to fatty acids having aliphatic tails with fewer than 6 carbons.

As used herein, "substantially homologous" or "substantially identical" in the context of two nucleic acids or polypeptides, generally refers to two or more sequences or subsequences that have at least 40%, 60%, 80%, 90%, 95%, 96%, 97%, 98% or 99% nucleotide or amino acid residue identity, when compared and aligned for maximum correspondence, as measured using sequence comparison algorithms or by visual inspection. The substantial identity can exist over any suitable region of the sequences, such as, for example, a region that is at least about 50 residues in length, a region that is at least about 100 residues, or a region that is at least about 150 residues. In certain embodiments, the sequences are substantially identical over the entire length of either or both comparison biopolymers.

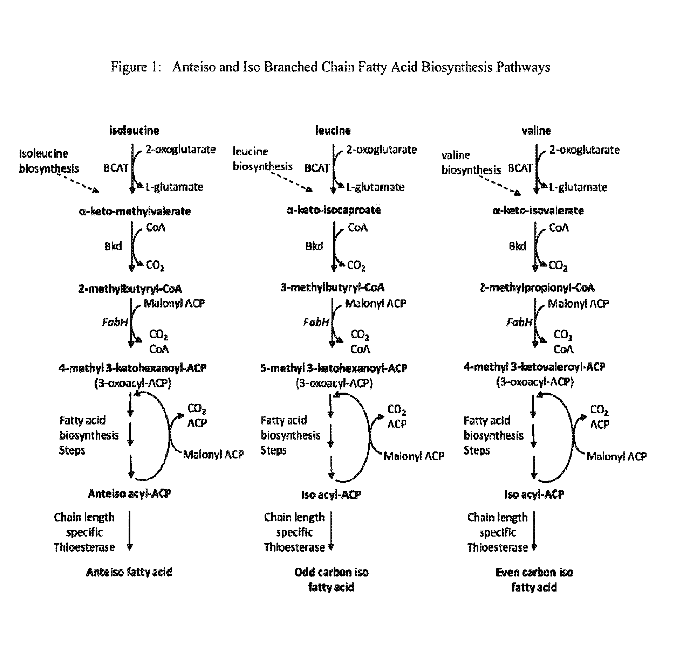

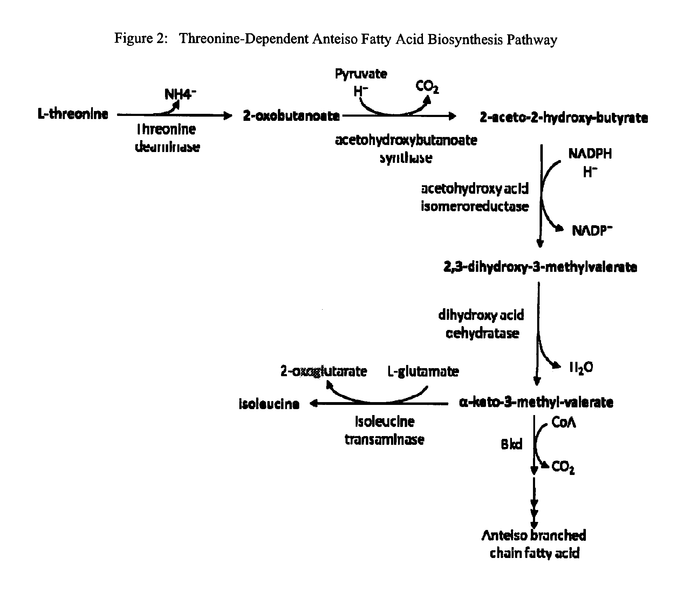

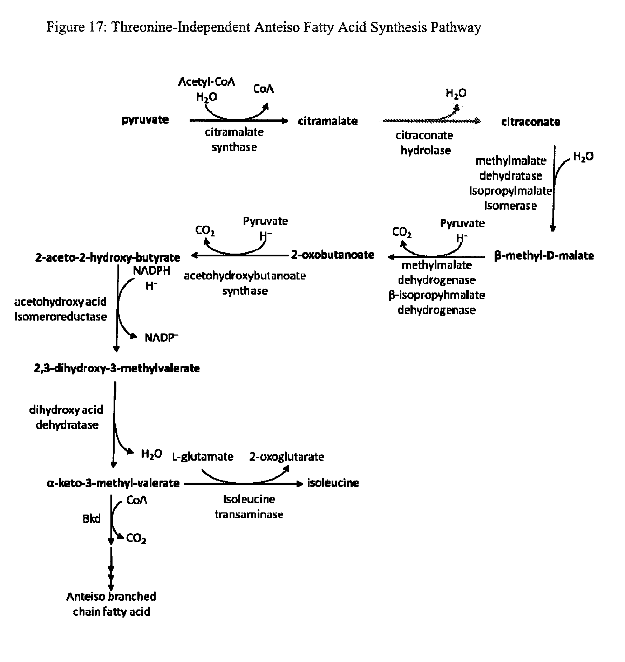

In one aspect, the invention relates to a novel method of producing anteiso and/or iso branched chain fatty acids (or products derived from anteiso and/or iso branched-chain fatty acids) using bacteria. In one aspect, the method features incorporating one or more exogenous polynucleotides that increase production of anteiso or iso fatty acid in a suitable cell, such as, for example, by transfecting or transforming the cell with the polynucleotide(s). Alternatively or in addition, the method comprises overexpressing one or more polynucleotides to increase production of anteiso or iso fatty acid within the host cell. Exemplary metabolic pathways for producing anteiso and iso fatty acid in a host cell are illustrated in Figures 1, 2, and 17. Figure 1 illustrates metabolic pathways for producing (1) anteiso fatty acid via a pathway that includes conversion of isoleucine to 2-keto 3-methylvalerate, (2) odd total carbon iso-branched-chain fatty acids via a pathway that includes conversion of leucine to 2-keto-isocaproate (also referred to as 2-keto, 4-methylvalerate), and (3) even numbered total carbon iso-branched-chain fatty acids via a pathway that includes conversion of valine to 2-keto-isovalerate (also referred to as 2-keto 3-methylbutyrate). In certain embodiments, driving the carbon flow to the branched 2-keto acid precursor results in increased production of the corresponding branched-chain fatty acid. For example, 1) increasing the carbon flow to the isoleucine pathway results in increased production of anteiso fatty acids; 2) increasing the carbon flow to the leucine pathway results in increased production of iso branched-chain fatty acid with an odd number of carbons; and/or 3) increasing the carbon flow to the valine pathway results in increased production of the iso branched-chain fatty acid with an even number of carbons. Figures 2 and 17 illustrate pathways for generating isoleucine and/or 2-keto 3-methylvalerate from threonine or pyruvate, respectively. Increasing carbon flow through the threonine and/or pyruvate pathways enhance the production of anteiso branched-chain fatty acid in a recombinant host cell.

In one aspect, the invention provides a method for producing anteiso fatty acid. The method comprises culturing a cell comprising at least one exogenous or overexpressed polynucleotide comprising a nucleic acid sequence encoding a polypeptide that catalyzes at least one of the following reactions: (aa) conversion of pyruvate to citramalate; (bb) conversion of citramalate to citraconate; (cc) conversion of citraconate to β-methyl-D-malate; (dd) conversion of β-methyl-D-malate to 2-oxobutanoate; or (ee) conversion of threonine to 2-oxobutanoate. Optionally, the cell further comprises at least one exogenous or overexpressed polynucleotide comprising a nucleic acid sequence encoding a polypeptide that catalyzes at least one of the following reactions: (ff) conversion of 2-oxobutanoate to 2-aceto-2-hydroxy-butyrate, (gg) conversion of 2-aceto-2-hydroxy-butyrate to 2,3-dihydroxy-3-methylvalerate, or (hh) conversion of 2,3-dihydroxy-3-methylvalerate to α-keto-3-methylvalerate. In some embodiments, the cell comprises exogenous or overexpressed polynucleotides encoding polypeptides that catalyze 3, 4, 5, 6, 7, or all of the reactions (aa)-(hh). The cell is cultured under conditions allowing expression of the polynucleotide(s) and production of anteiso fatty acid. The invention is predicated, at least in part, on the observation that host cells comprising the genetic modifications described herein produce more anteiso fatty acids than an otherwise similar cell that does not comprise the polynucleotide(s). Metabolic pathways and genetic modifications for increasing anteiso and iso fatty acid production in a cell are further described below.

One method for increasing carbon flow to the isoleucine pathway comprises upregulating production of 2-keto 3-methylvalerate through the threonine-dependent pathway of Figure 2. Threonine can be produced at high levels in, e.g., *E. coli* (Lee et al., Molecular Systems Biology 3: 149 (2007)) and, through a series of steps shown in Figure 2, isoleucine is produced from threonine via 2-keto 3-methylvalerate as an intermediate. As illustrated in Figure 2, the threonine-dependent pathway entails conversion of threonine to 2-oxobutanoate by, e.g., threonine deaminase; conversion of 2-oxobutanoate to 2-aceto2-hydroxy-butyrate by, e.g., acetohydroxy acid synthase (AHAS) (also known as acetohydroxybutanoate synthase); conversion of 2-aceto 2-hydroxy-butyrate to 2,3-dihydroxy-3-methylvalerate by, e.g., acetohydroxy acid isomeroreductase; and conversion of 2,3-dihydroxy-3-methylvalerate to 2-keto-3-methyl-valerate by, e.g., dihydroxy acid dehydratase. The pathway is optimized for carbon flow to 2-keto-3-methyl-valerate and ultimately to the anteiso fatty acid by expressing exogenous polynucleotides or overexpressing endogenous polynucleotides encoding any one or more of the activities described above. For example, the pathway is optimized for carbon flow to 2-keto 3-methyl-valerate by overexpressing *ilvA, tdcB, ilvI, ilvH, ilvC* and/or *ilvD.* IlvA and TdcB are threonine deaminases. IlvC is an acetohydroxy acid isomeroreductase (also known as ketol-acid reductoisomerase), and IlvD is a dihydroxy acid dehydratase. IlvI and IlvH are two subunits that form AHAS, which catalyzes the formation of 2-acetolactate from pyruvate for valine and leucine synthesis, or the formation of 2-aceto-2-hydroxybutyrate from 2-oxobutanoate and pyruvate for isoleucine biosynthesis (see Figure 1). The two AHAS reactions are irreversible and committed steps toward the synthesis of two different sets of branched-chain amino acids. In certain embodiments, for example, deletion of the AHAS I (*ilvBN*) and/or overproduction of AHAS II (*ilvGM*) and/or AHAS III (*ilvIH*) minimize production of iso fatty acid derived from leucine or valine. IlvBH also is an AHAS suitable for use in the context of the invention.

Thus, in one aspect, the cell of the invention comprises an exogenous or overexpressed polynucleotide encoding a polypeptide that catalyzes the conversion of threonine to 2-oxobutanoate (e.g., a threonine deaminase) and an exogenous or overexpressed polynucleotide encoding a polypeptide that catalyzes the conversion of 2-oxobutanoate to 2-aceto 2-hydroxy-butyrate (e.g., an AHAS).

In certain embodiments, cells or organisms of the invention are engineered to accumulate anteiso fatty acids under nitrogen-limiting conditions and to utilize a threonine-independent isoleucine synthesis pathway, such as the pyruvate pathway shown in Figure 17. As illustrated in Figure 17, pyruvate is combined with acetyl-CoA to produce citramalate by, e.g., citramalate synthase. An exemplary citramalate synthase is CimA, such as CimA derived from M. *jannaschii.* Citramalate is then converted to citraconate by, e.g., a citraconate hydrolase (also known as isopropylmalate or citramalate isomerase), an example of which is encoded by *leuCD.* Citraconate is converted to β-methyl-D-malate by, e.g., an isopropylmalate isomerase (such as LeuCD), and the resulting β-methyl-D-malate is converted to 2-oxobutanoate (also referred to as α-ketobutyrate) by, e.g., isopropylmalate dehydrogenase (such as LeuB). The pyruvate pathway converges with the threonine pathway of Figure 2, as 2-oxobutanoate is converted to 2-aceto-2-hydroxy-butyrate by, e.g., AHAS; 2-aceto 2-hydroxy-butyrate is converted to 2,3-dihydroxy-3-methylvalerate by, e.g., acetohydroxy acid isomeroreductase; and 2,3-dihydroxy-3-methylvalerate is converted to 2-keto 3-methyl-valerate by, e.g., dihydroxy acid dehydratase. The pathway is optimized for carbon flow to 2-keto 3-methyl-valerate and ultimately to the anteiso fatty acid by expressing exogenous polynucleotides or overexpressing endogenous polynucleotides encoding any one or more of the activities described above. For example, the pathway is optimized for carbon flow to 2-keto 3-methyl-valerate by overexpressing or expressing exogenous *cimA, leuCD, leuB, ilvI, ilvH, ilvC ilvG, ilvM,* and/or *ilvD.*

Thus, in one aspect, the cell of the invention comprises exogenous or overexpressed polynucleotides encoding polypeptides that catalyze the conversion of pyruvate to citramalate, the conversion of citramalate to citraconate, the conversion of citraconate to β-methyl-D-malate, and the conversion of 2-oxobutanoate to 2-aceto-2-hydroxy-butyrate. For example, in one embodiment, the cell comprises an exogenous or overexpressed polynucleotide comprising a nucleic acid sequence encoding a citramalate synthase, an exogenous or overexpressed polynucleotide comprising a nucleic acid sequence encoding an isopropylmalate isomerase, an exogenous or overexpressed polynucleotide comprising a nucleic acid sequence encoding an isopropylmalate dehydrogenase, an exogenous or overexpressed polynucleotide comprising a nucleic acid sequence encoding a AHAS, or a combination thereof, including a combination of polynucleotides encoding all four polypeptides.

In one aspect, the host cell is modified to express an exogenous polynucleotide or overexpress a native polynucleotide encoding one or more enzyme activities that mediate downstream reactions yielding anteiso or iso fatty acid from isoleucine, leucine, or valine. For example, as shown in Figure 1, in certain embodiments, cells are modified to produce anteiso fatty acids via a pathway that includes conversion of isoleucine to 2-keto 3-methylvalerate by a branched-chain amino acid aminotransferase (BCAT). Alternatively, the 2-keto 3-methylvalerate is introduced into isoleucine biosynthesis pathway without first being converted to isoleucine. The 2-keto 3-methylvalerate is then converted to 2-methylbutyryl-CoA by, e.g., a branched-chain α-keto acid dehydrogenase (BCDH), such as a BCDH encoded by *bkd.* The 2-methylbutyryl-CoA is condensed with a malonyl-ACP by, e.g., a 3-ketoacyl-ACP synthase, and the subsequent incorporation of malonyl-ACP is processed via fatty acid biosynthesis to anteiso acyl-ACP. Acyl-ACP is then converted to anteiso fatty acids via a thioesterase.

In certain embodiments, odd total carbon iso-branched-chain fatty acids are produced via a pathway that includes conversion of leucine to 2-keto-isocaproate (also referred to as 2-keto, 4-methylvalerate) by, e.g., a BCAT. If desired, 2-keto-isocaproate is introduced into the leucine biosynthesis pathway without first being converted to leucine. The 2-keto-isocaproate is then converted to isovaleryl-CoA (also referred to as 3-methylbutyryl-CoA) by, e.g., a BCDH, such as a BCDH encoded by *bkd.* The isovaleryl-CoA is condensed with a malonyl-ACP by, e.g., a 3-ketoacyl-ACP synthase, and the subsequent incorporation of malonyl-ACP is processed via fatty acid biosynthesis to iso acyl-ACP. Iso acyl-ACP is then converted to iso fatty acids via a thioesterase.

Furthermore, in certain embodiments, even numbered total carbon iso-branched-chain fatty acids are produced via a pathway that includes conversion of valine to 2-keto-isovalerate (also referred to as 2-keto 3-methylbutyrate) by a BCAT. Optionally, 2-keto-isovalerate is introduced into the valine biosynthesis pathway without first being converted to valine. The 2-keto-isovalerate is then converted to isobutyryl-CoA by, e.g., a BCDH, such as a BCDH encoded by *bkd.* The isobutyryl-CoA is condensed with a malonyl-ACP by, e.g., a 3-ketoacyl-ACP synthase, and the subsequent incorporation of malonyl-ACP is processed via fatty acid biosynthesis to iso acyl-ACP. Iso acyl-ACP can then be converted to iso fatty acids via a thioesterase.

Thus, in some embodiments, the host cell comprises an exogenous or overexpressed polynucleotide encoding a BCDH or a biologically active fragment or variant thereof. Alternatively or in addition, the cell comprises an exogenous or overexpressed polynucleotide encoding a BCAT and/or an exogenous or overexpressed polynucleotide encoding an acyl transferase. Alternatively or in addition, the cell comprises an exogenous or overexpressed polynucleotide encoding a 3-ketoacyl-ACP synthase that uses anteiso and/or iso branched-CoA primers as substrates into a suitable cell. In addition or alternatively, the cell comprises an exogenous or overexpressed polynucleotide comprising a nucleic acid sequence encoding an enoyl-ACP reductase. In addition, or alternatively, the cell comprises an exogenous or overexpressed polynucleotide encoding a thioesterase. Depending on the activity and substrate specificity of the thioesterase, such recombinant cells can produce anteiso and/or iso branched-chain fatty acids having a desired chain length. For example, in some embodiments, the host cell preferentially generates long chain fatty acid, medium-length chain fatty acid, or a desired combination thereof (e.g., 60%, 70%, 80%, 85%, 90%, 95% or more of the fatty acid comprises the desired number of carbons). Combinations of any of the enzymes described herein also is contemplated, such as, for example, a cell comprising exogenous or overexpressed polynucleotides encoding BCDH, 3-ketoacyl-ACP synthase, and thioesterase (such as TesA). Indeed, the invention contemplates a cell engineered to increase carbon flow through the threonine-dependent and/or threonine-independent pathways as described above and further comprising exogenous or overexpressed polynucleotides that augment carbon flow through the isoleucine, leucine, and/or valine pathways illustrated in Figure 1. Optionally, one or more of the exogenous or overexpressed polynucleotides comprise a nucleic acid sequence having at least 90 percent identity to the nucleic acid sequences set forth in SEQ ID NOs: 1, 4, 7, 13, 17-23, 68, 77, or 78.

In addition, or alternatively, in certain embodiments, production of anteiso and/or iso branched-chain fatty acids is enhanced by modifying cells to increase acetyl-CoA carboxylase activity. For example, production of one or more of the enzyme subunits is increased by, e.g., increasing the amount of available biotin or by increasing the activity or amount of the biotin-protein ligase, BirA. Upregulating thiamine levels in a host cell by, for instance, augmenting thiamine synthase production, also is contemplated herein to further enhance branched-chain fatty acid synthesis.

In some embodiments of the invention, the cell is engineered to express one or more exogenous polynucleotides encoding one or more of the enzyme activities described herein and/or is engineered to overexpress one or more endogenous polynucleotides encoding one or more of the enzyme activities described herein. Different organisms manufacture fatty acids using different pathways, and endogenous fatty acid synthesis reactions can leech resources away from branched-chain fatty acid synthesis. Thus, in certain embodiments, native enzyme activity is attenuated to enhance branched-chain fatty acid synthesis. For example, in *E. coli,* which does not naturally produce anteiso and/or iso branched-chain fatty acids, the first condensation reaction in fatty acid synthesis is the reaction of acetyl-CoA with malonyl-ACP, yielding 3-ketobutyryl-ACP (Smirnova and Reynolds, J. Industrial Microbiology & Biotechnology 27: 246-51 (2001)). This reaction is primarily catalyzed by the *fabH* product, a 3-ketoacyl-ACP synthase. The *E*. *coli* 3-ketoacyl-ACP synthase, however, shows specificity in that it prefers acetyl-CoA over branched acyl-CoA such as 2-methylbutyryl-CoA (Choi et al., J. Bacteriology 182: 365-70 (2000)). Reducing or removing endogenous FabH activity through chemical inhibitors such as cerulenin or through genetic engineering (e.g., by creating a *fabD* and *fabH* double mutant) reduces the amount of straight chain fatty acids produced. If desired, branched-chain fatty acid production also is increased by removing or reducing a host cell's (e.g., *E*. *coli'*s) capacity to make straight-chain fatty acids by, for example, incorporating fatty acid synthesis genes derived from *Exiguobacterium* to shorten the chain length and/or increase the amount of anteiso and/or iso branched-chain fatty acids.

Additionally or alternatively, gene knockouts or knockdowns that minimize the carbon flow to branch pathways not contributing to the anteiso or iso fatty acid formation may be used. For example, isoleucine transaminase activity is attenuated to redirect carbon flow from isoleucine synthesis to anteiso branched-chain fatty acid synthesis (see Figures 2 and 17). In this regard, the cell is genetically modified to reduce expression of *ilvE* or inhibit activity of the gene product. Alternatively, the cell is modified to generate a *fadD* mutant defective in converting a fatty acid to fatty acyl-CoA, the first step in fatty acid degradation. Fatty acid content is thereby increased. Alternatively or in addition, the cell is modified to attenuate branched-chain amino acid aminotransferase (BCAT) activity. Enzyme activity is attenuated (i.e., reduced or abolished) by, for example, mutating the coding sequence for the enzyme to create a non-function or reduced-function polypeptide, by removing the coding sequence for the enzyme from the cellular genome, by interfering with translation of an RNA transcript encoding the enzyme (e.g., using antisense oligonucleotides), or by manipulating the expression control sequences influencing expression of the enzyme.

Anteiso and/or iso branched-chain fatty acids are produced using any suitable cells or organisms, such as, for example, bacterial cells and other prokaryotic cells, yeast cells, or mammalian cells. In certain embodiments, the invention relates to cells, such as *Escherichia* cells (e.g., *E. coli*), which do not naturally produce anteiso and/or iso branched-chain fatty acids. These cells are engineered to produce anteiso and/or iso branched-chain fatty acids as described herein. In one aspect, the cells are modified to produce anteiso and/or iso branched-chain fatty acids at desired levels and with desired chain lengths. In addition, in certain embodiments, the engineered cells tolerate large amounts of anteiso and/or iso branched-chain fatty acids in the growth medium, plasma membrane, or lipid droplets, and/or produce anteiso and/or iso branched-chain fatty acids more economically than an unmodified cell by, e.g., using a less expensive feedstock, requiring less fermentation time, and the like.

In certain embodiments, cells or organisms that naturally produce anteiso and/or iso branched-chain fatty acids, such as *Bacillus subtilis* and *Streptomyces avermitilis,* are modified as described herein to produce higher levels of anteiso and/or iso branched-chain fatty acids compared to an unmodified cell or organism. Optimization is achieved, for example, by incorporating regulatory mutations that lead to higher levels of fatty acids in the cells and/or overexpressing enzyme activities for increased branched keto acid precursor and/or precursors for the fatty acid biosynthesis pathway. Optimization also may be achieved by attenuating enzyme activity that diverts carbon flow from branched-chain fatty acid production. Alternatively or in addition, the cells produce anteiso and/or iso branched-chain fatty acids with specified chain lengths. If desired, a thioesterase is selected with specificity for a particular chain length. For example, the thioesterases from Mallard uropygial gland and rat mammary gland preferentially generate medium-chain length fatty acids having C6-C 14 aliphatic tails.

Exemplary bacteria that naturally produce branched-chain fatty acids and are suitable for use in the invention include, but are not limited to, *Spirochaeta aurantia, Spirochaeta littoralis, Pseudomonas maltophilia, Pseudomonas putrefaciens, Xanthomonas campestris, Legionella anisa, Moraxella catarrhalis, Thermus aquaticus, Flavobacterium aquatile, Bacteroides asaccharolyticus, Bacteroides fragilis, Succinimonas amylolytica, Desulfovibrio africanus, Micrococcus agilis, Stomatococcus mucilaginosus, Planococcus citreus, Marinococcus albusb, Staphylococcus aureus, Peptostreptococcus auaerobius, Ruminococcus albus, Sarcina lutea, Bacillus anthracis, Sporolactobacillus inulinus, Clostridium thermocellum, Sporosarcina ureae, Desulfotomaculum nigrificans, Listeria monocytogenes, Brochothrix thermosphacta, Renibacterium salmoninarum, Kurthia zopfii, Corynebacterium aquaticum, Arthrobacter radiotolerans, Brevibacterium fermentans, Propionibacterium acidipropionici, Eubacterium lentum, Cytophaga aquatilis, Sphingobacteriuma multivorumb, Capnocytophaga gingivalis, Sporocytophaga myxococcoides, Flexibacter elegans, Myxococcus coralloides, Archangium gephyra, Stigmatella aurantiaca, Oerskovia turbata,* and *Saccharomonospora viridis.* Exemplary microorganisms that produce branched-chain fatty acids also are disclosed in, e.g., Kaneda, Microbiol. Rev. 55(2): 288-302 (1991) (see Table 3).

The polynucleotide(s) encoding one or more enzyme activities for producing anteiso and/or iso fatty acids may be derived from any source. Depending on the embodiment of the invention, the polynucleotide is isolated from a natural source such as bacteria, algae, fungi, plants, or animals; produced via a semi-synthetic route (e.g., the nucleic acid sequence of a polynucleotide is codon optimized for expression in a particular host cell, such as *E. coli);* or synthesized *de novo.* In certain embodiments, it is advantageous to select an enzyme from a particular source based on, e.g., the substrate specificity of the enzyme, the type of branched-chain fatty acid produced by the source, or the level of enzyme activity in a given host cell. In one aspect of the invention, the enzyme and corresponding polynucleotide are naturally found in the host cell and overexpression of the polynucleotide is desired. In this regard, in some instances, additional copies of the polynucleotide are introduced in the host cell to increase the amount of enzyme available for fatty acid production. Overexpression of a native polynucleotide also is achieved by upregulating endogenous promoter activity, or operably linking the polynucleotide to a more robust promoter.

Exogenous enzymes and their corresponding polynucleotides also are suitable for use in the context of the invention, and the features of the biosynthesis pathway or end product can be tailored depending on the particular enzyme used. If desired, the polynucleotide(s) is isolated or derived from the branched-chain fatty acid-producing organisms described herein. For example, *E. coli* FabH, a 3-ketoacyl-ACP synthase, preferentially uses acetyl-CoA as a substrate rather than branched acyl-CoA, while FabH from *B. subtilis* more efficiently drives branched fatty acid synthesis. Thus, in one aspect, the cell is an *E*. *coli* cell comprising a polynucleotide encoding *B. subtilis* FabH.

An exemplary citramalate synthase produced by the cell is derived from *M. jannaschii* CimA. Exemplary AHASs include *E*. *coli* IlvIH, *E*. *coli* IlvIH (G14D), *E*. *coli* IlvGM, and *B*. *subtilis* IlvBH. An exemplary BCDH is *B. subtilis* Bkd, and an exemplary 3-ketoacyl-ACP synthase is *B. subtilis* FabH. An exemplary threonine deaminase is *E. coli* TdcB. Exemplary thioesterases include, but are not limited to, *E. coli* TesA, thioesterase from Mallard uropygial gland, and thioesterase from rat mammary gland. An exemplary isopropylmalate isomerase is *E*. *coli* LeuCD, and an exemplary isopropylmalate dehydrogenase is *E*. *coli* LeuB. In one aspect, the cell comprises a nucleic acid sequence having at least about 90 percent identity to the nucleic acid sequence set forth in SEQ ID NO: 32, 36, 42, 43, 46, 51, 57, 62, 68, or 83, or encodes a polypeptide comprising an amino acid sequence having at least about 90 percent identity to the amino acid sequence set forth in SEQ ID NO: 33, 39, 40, 41, 47, 48, 52, 53, 58, 65, 66, 67, 84, or 85. Exemplary enzymes that mediate production of anteiso and/or iso fatty acids also are disclosed in Table A.

**TABLE A**

| **Activity** | **Gene Name** | **Organism** | **Accession** |
|---|---|---|---|
| Branched-chain amino acid transaminase | *ilvE* | *Salmonella enteric* | NP_457845 |
| | *ilvE* | *Yersinia pestis* | YP_002348774 |
| | *ilvE* | *Shigella flexneri* | NP_709575 |
| | *ilvE* | *Pectobacterium carotovorum* | YP_003018265 |
| | *ilvE* | *Ralstonia solanacearum* | YP_003753411 |
| Branched-chain 2-keto acid dehydrogenase, E1 component, alpha subunit | *bkdAA* | *Anoxybacillus flavithermus* | YP_002315323 |
| | *bfmBAA* | *Staphylococcus aureus* | NP_374631 |
| | *bkdA1* | *Sphingobium japonicum* | YP_003544745 |
| | *bkdA1* | *Brevibacillus brevis* | YP_002771850 |
| | *bkdA* | *Lactobacillus casei* | YP_001987607 |
| Branched-chain 2-keto acid dehydrogenase, E1 component, beta subunit | *bkdAB* | *Anoxybacillus flavithermus* | YP_002315324 |
| | *bfmBAB* | *Staphylococcus aureus* | NP_374630 |
| | *bkdA2* | *Sphingobium japonicum* | YP_003544746 |
| | *bkdA2* | *Brevibacillus brevis* | YP_002771851 |
| | *bkdB* | *Lactobacillus casei* | YP_001987606 |
| Branched-chain 2-keto acid dehydrogenase, E2 component | *bkdB* | *Anoxybacillus flavithermus* | YP_002315325 |
| | *bfmBB* | *Staphylococcus aureus* | NP_374629 |
| | *pdhC* | *Sphingobium japonicum* | YP_003544747 |
| | *bkdB* | *Brevibacillus brevis* | YP_002771852 |
| | *bkdC* | *Lactobacillus casei* | YP_001987605 |
| Branched-chain 2-keto acid dehydrogenase, E3 component | *lpdV* | *Anoxybacillus flavithermus* | YP_002315322 |
| | | *Staphylococcus aureus* | NP_374632 |
| | *pdhD* | *Sphingobium japonicum* | YP_003545508 |
| | *Lpd* | *Brevibacillus brevis* | YP_002771849 |
| | *bkdD* | *Lactobacillus casei* | YP_001987608 |
| 3-ketoacyl-ACP synthase III | *fabH* | *Geobacillus kaustophilus* | YP_146657.1 |
| | | *Bacillus megaierium* | YP_003561163.1 |
| | *fabH* | *Staphylococcus aureus* | ZP_05601460.1 |
| | *fabH1* | *Streptomyces coelicolor* | P72392 |
| | | *Beutenbergia cavernae* | YP_002881824 |
| citramalate synthase | *cimA* | *Methanobrevibacter ruminantium* | YP_003424156 |
| | *cimA* | *Leptospira interrogans* | ABK13754 |
| | | *Ignicoccus hospitalis* | ABU82163 |
| | | *Cyanothece 51142* | YP_001801665 |
| | | *Geobacter sulfurreducens* | NP_952848 |
| 3-isopropylmalate dehydrogenase | *leuB* | *Cronobacter turicensis* | YP_003209069.1 |
| | *leuB* | *Shigella boydii* | YP_406624 |
| | *leuB* | *Actinobacillus pleuropneumoniae* | YP_001651485 |
| | *leuB* | *Cronobacter turicensis* | YP_003209069 |
| | *leuB* | *Pantoea ananatis* | YP_003519000 |
| isopropylmalate isomerase large subunit | *leuC* | *Salmonella enteric* | NP_459116 |
| | *leuC* | *Serratia protearnaculans* | YP_001476977 |
| | *leuC* | *Photorhabdus asymbiotica* | YP_003039932 |
| | *leuC* | *Klebsiella pneumonia* | YP_002917788 |
| | *leuC* | *Haemophilus influenzae* | NP_439151 |
| isopropylmalate isomerase small subunit | *leuD* | *Shigella dysenteriae* | YP_401823 |
| | *leuD* | *Buchnera aphidicola* | YP_002477704 |
| | *leuD* | *Actinobacillus pleuropueumoniae* | YP_001967934 |
| | *leuD* | *Haemophilus somnus* | YP_718599 |
| | *leuD* | *Xanthomonas campestris* | YP_365316 |
| threonine deaminase | *Chal* | *Saccharomyces cerevisiae* | NP_001018030 |
| | *ilvA* | *Vibrio, fischeri* | YP_002157347 |
| | *ilvA* | *Shewanella violacea* | YP_003558613 |
| | *ilvA* | *Methylococcus capsulatus* | YP_112886 |
| | *ilvA* | *Dichelobacter nodosus* | YP_001209243 |
| threonine dehydratase | *tdcB* | *Pantoea ananatis* | YP_003519179 |
| | *tdcB* | *Klebsiella pneumonia* | YP_001335951 |
| | *tdcB* | *Shigella boydii* | YP_409322 |
| | *tdcB* | *Acinetobacter baumannii* | YP_001706275 |
| | *tdcB* | *Psychrobacter arcticum* | YP_264671 |
| acetolactate synthase (AHAS) III large subunit | *ilvI* | *Yersinia enterocolitica* | YP_001005008 |
| | *ilvI* | *Salmonella enterica* | YP_002113134 |
| | *ilvI* | *Buchnera aphidicola* | NP_240056 |
| | *ilvI* | *Xenorhabdus bovienii* | YP_003469370 |
| | *ilvI* | *Klebsiella pneumonia* | YP_001333772 |
| acetolactate synthase (AHAS) III small subunit | *ilvH* | *Laribacter hongkongensis* | YP_002794162 |
| | *ilvH* | *Burkholderia mallei* | YP_103450 |
| | *ilvH* | *Nitrosomonas europaea* | NP_841373 |
| | *ilvH* | *Campylobacter jejuni* | YP_178690 |
| | *ilvH* | *Desulfomicrobium baculatum* | YP_003156951 |
| acetolactate synthase (AHAS) II large subunit | *ilvG* | *Yersinia pestis* | YP_002348776 |
| | *ilvG* | *Ralstonia solanacearum* | YP_003752653 |
| | *ilvG* | *Bordetella bronchiseptica* | NP_887973 |
| | *ilvG* | *Aeromonas salmonicida* | YP_001140066 |
| | *ilvG* | *Stenotrophomonas maltophilia* | YP_001973605 |
| acetolactate synthase (AHAS) II small subunit | *ilvM* | *Shigella dysenteriae* | YP_405398 |
| | *ilvM* | *Dickeya dadantii* | YP_002989351 |
| | *ilvM* | *Xenorhabdus bovienii* | YP_003470064 |
| | *ilvM* | *Photorhabdus luminescent* | NP_931846 |
| | *ilvM* | *Xanthomonas oryzae* | YP_199583 |
| ketol-acid reductoisomerase | *ilvC* | *Buchnera aphidicola* | NP_240398 |
| | *ilvC* | *Pseudomonas aeruginosa* | YP_002442658 |
| | *ilvC* | *Francisella tularensis* | YP_001122021 |
| | *ilvC* | *Vibrio, fischeri* | YP_205911 |
| | *ilvC* | *Actinobacillus pleuropneumoniae* | YP_001652891 |
| dihydroxy-acid dehydratase | *ilvD* | *Citrobacter rodentium* | YP_003367413 |
| | *ilvD* | *Buchnera aphidicola* | YP_002468875 |
| | *ilvD* | *Xanthomonas campestris* | YP_001901776 |
| | *ilvD* | *Methylococcus capsulatus* | YP-114512 |
| | *ilvD* | *Chromobacterium violaceum* | NP_900947 |
| enoyl-ACP reductase | *fabI* | *Geobacillus thermodenitrificans* | YP_001124839 |
| | *fabI* | *Anoxybacillus flavithermus* | YP_002316451 |
| | *fabI* | *Listeria monocytogenes* | ZP_05298370 |
| | *fabI* | *Staphylococcus epidermidis* | ZP_04796766 |
| | *fabI* | *Clostridium thermocellum* | ABN54364 |

In certain embodiments, the recombinant cell produces an analog or variant of the polypeptide encoding an enzyme activity involved in fatty acid biosynthesis. Amino acid sequence variants of the polypeptide include substitution, insertion, or deletion variants, and variants may be substantially homologous or substantially identical to the unmodified polypeptides as set out above. In certain embodiments, the variants retain at least some of the biological activity, e.g., catalytic activity, of the polypeptide. Other variants include variants of the polypeptide that retain at least about 50%, preferably at least about 75%, more preferably at least about 90%, of the biological activity.

Substitutional variants typically exchange one amino acid for another at one or more sites within the protein. Substitutions of this kind can be conservative, that is, one amino acid is replaced with one of similar shape and charge. Conservative substitutions include, for example, the changes of: alanine to serine; arginine to lysine; asparagine to glutamine; aspartate to glutamate; cysteine to serine; glutamine to asparagine; glutamate to aspartate; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine.

In some instances, the recombinant cell comprises an analog or variant of the exogenous or overexpressed polynucleotide(s) described herein. Nucleic acid sequence variants include one or more substitutions, insertions, or deletions, and variants may be substantially homologous or substantially identical to the unmodified polynucleotide. Polynucleotide variants or analogs encode mutant enzymes having at least partial activity of the unmodified enzyme. Alternatively, polynucleotide variants or analogs encode the same amino acid sequence as the unmodified polynucleotide. Codon optimized sequences, for example, generally encode the same amino acid sequence as the parent/native sequence but contain codons that are preferentially expressed in a particular host organism.

A polypeptide or polynucleotide "derived from" an organism contains one or more modifications to the native amino acid sequence or nucleotide sequence and exhibits similar, if not better, activity compared to the native enzyme (e.g., at least 70%, at least 80%, at least 90%, at least 95%, at least 100%, or at least 110% the level of activity of the native enzyme). For example, enzyme activity is improved in some contexts by directed evolution of a parent/native sequence. Additionally or alternatively, an enzyme coding sequence is mutated to achieve feedback resistance. The citramalate synthase CimA3.7 derived from *M. jannaschii* and described in Example 14 is truncated and comprises substitutions compared to the native *M*. *jannaschii* citramalate synthase enzyme. The modifications confer feedback resistance to the CimA3.7 enzyme and improve its activity. Similarly, substitution of the glycine with aspartate at amino acid position 14 of the *E. coli* IlvIH AHAS sequence (designated IlvIH (G14D)) imparts resistance to 2-aceto-2-hydroxy-butyrate inhibition. Thus, in one or more embodiments of the invention, the polypeptide encoded by the exogenous polynucleotide is feedback resistant and/or is modified to alter the activity of the native enzyme.

Recombinant cells can be produced in any suitable manner to establish an expression vector within the cell. The expression vector can include the exogenous polynucleotide operably linked to expression elements, such as, for example, promoters, enhancers, ribosome binding sites, operators and activating sequences. Such expression elements may be regulatable, for example, inducible (via the addition of an inducer). Alternatively or in addition, the expression vector can include additional copies of a polynucleotide encoding a native gene product operably linked to expression elements. Representative examples of useful promoters include, but are not limited to: the LTR (long terminal 35 repeat from a retrovirus) or SV40 promoter, the *E. coli lac, tet,* or *trp* promoter, the phage Lambda P_{L} promoter, and other promoters known to control expression of genes in prokaryotic or eukaryotic cells or their viruses. In one aspect, the expression vector also includes appropriate sequences for amplifying expression. The expression vector can comprise elements to facilitate incorporation of polynucleotides into the cellular genome. Introduction of the expression vector or other polynucleotides into cells can be performed using any suitable method, such as, for example, transformation, electroporation, microinjection, microprojectile bombardment, calcium phosphate precipitation, modified calcium phosphate precipitation, cationic lipid treatment, photoporation, fusion methodologies, receptor mediated transfer, or polybrene precipitation. Alternatively, the expression vector or other polynucleotides can be introduced by infection with a viral vector, by conjugation, by transduction, or by other suitable methods.

Cells, such as bacterial cells or any other desired host cells, containing the polynucleotides encoding the exogenous or overexpressed proteins are cultured under conditions appropriate for growth of the cells and expression of the polynucleotide(s). Cells expressing the polypeptide(s) can be identified by any suitable methods, such as, for example, by PCR screening, screening by Southern blot analysis, or screening for the expression of the protein. In certain embodiments, cells that contain the polynucleotide can be selected by including a selectable marker in the DNA construct, with subsequent culturing of cells containing a selectable marker gene, under conditions appropriate for survival of only those cells that express the selectable marker gene. The introduced DNA construct can be further amplified by culturing genetically modified cells under appropriate conditions (e.g., culturing genetically modified cells containing an amplifiable marker gene in the presence of a concentration of a drug at which only cells containing multiple copies of the amplifiable marker gene can survive). Cells that contain and express polynucleotides encoding the exogenous or overexpressed proteins are referred to herein as genetically modified cells. Bacterial cells that contain and express polynucleotides encoding the exogenous or overexpressed protein can be referred to as genetically modified bacterial cells.

In certain embodiments, the genetically modified cells (such as genetically modified bacterial cells) have an optimal temperature for growth, such as, for example, a lower temperature than normally encountered for growth and/or fermentation. For example, in certain embodiments, incorporation of branched-chain fatty acids into the membrane increases membrane fluidity, a property normally associated with low growth temperatures. In addition, in certain embodiments, cells of the invention exhibit a decline in growth at higher temperatures as compared to normal growth and/or fermentation temperatures as typically found in cells of the type.

Any cell culture condition appropriate for growing a host cell and synthesizing anteiso and/or iso fatty acids is suitable for use in the inventive method. Addition of fatty acid synthesis intermediates, precursors, and/or co-factors for the enzymes associated with anteiso and/or iso branched-chain fatty acid synthesis to the culture is contemplated herein. In one embodiment, the method comprises exposing the host cell to thiamine, which enhances anteiso fatty acid synthesis. Isoleucine, leucine, and/or valine is added to the culture in some embodiments.

The inventive method optionally comprises extracting anteiso and/or iso fatty acid from the culture. Fatty acids can be extracted from the culture medium and measured in any suitable manner. Suitable extraction methods include, for example, methods as described in Bligh et al., "A rapid method for total lipid extraction and purification," Can. J. Biochem. Physiol. 37:911-917 (1959). In certain embodiments, production of fatty acids in the culture supernatant or in the membrane fraction of recombinant cells can be measured. In this embodiment, cultures are prepared in the standard manner, although nutrients (e.g. 2-methylbutyrate, isoleucine) that may provide a boost in substrate supply can be added to the culture. Cells are harvested by centrifugation, acidified with hydrochloric or perchloric acid, and extracted with chloroform and methanol, with the fatty acids entering the organic layer. The fatty acids are converted to methyl esters, using methanol at 100°C. The methyl esters are separated by gas chromatography (GC) and compared with known standards of straight-chain, iso and anteiso fatty acids (purchased from Larodan or Sigma). Confirmation of chemical identity is carried out by combined GC/mass spec, with further mass spec analysis of fragmented material carried out if necessary.

In one embodiment, the cell utilizes the branched-chain anteiso and/or iso fatty acid as a precursor to make or more other products. Products biosynthesized (i.e., derived) from anteiso or iso fatty acid include, but are not limited to, phospholipids, triglycerides, alkanes, olefins, wax esters, fatty alcohols, and fatty aldehydes. Some host cells naturally generate one or more products derived from anteiso or iso fatty acid; other host cells are genetically engineered to convert branched-chain fatty acid to, e.g., an alkane, olefin, wax ester, fatty alcohol, and/or fatty aldehyde. Organisms and genetic modifications thereof to synthesize products derived from branched-chain fatty acids are further described in, e.g., International Patent Publication Nos. WO 2007/136762, WO 2008/151149, and WO 2010/062480, and U.S. Patent Application Publication US 2010/0298612. In one aspect, the inventive method comprises extracting a product derived from anteiso fatty acid (phospholipid, triglyceride, alkane, olefin, wax ester, fatty alcohol, and/or fatty aldehyde synthesized in the cell from anteiso fatty acid) from the culture. Any extraction method is appropriate, including the extraction methods described in International Patent Publication Nos. WO 2007/136762, WO 2008/151149, and WO 2010/062480, and U.S. Patent Application Publication Nos. US 2010/0251601, US 20100242345, US 20100105963, and US 2010/0298612.

In one embodiment, the invention provides a cell comprising an exogenous or overexpressed polynucleotide comprising a nucleic acid sequence encoding a threonine deaminase, an exogenous or overexpressed polynucleotide comprising a nucleic acid sequence encoding an acetohydroxy acid synthase, an exogenous or overexpressed polynucleotide comprising a nucleic acid sequence encoding a branched-chain α-keto acid dehydrogenase, and an exogenous or overexpressed polynucleotide comprising a nucleic acid sequence encoding a 3-ketoacyl-ACP synthase. The cell optionally further comprises at least one exogenous or overexpressed polynucleotide comprising a nucleic acid sequence encoding a thioesterase. The polynucleotides are expressed in the cell. In one aspect, the cell is a bacterial cell that does not naturally produce anteiso fatty acid, such as *Escherichia coli.* The invention further provides a method of producing anteiso fatty acid, the method comprising culturing the bacterial cell under conditions that allow expression of the polynucleotides and production of anteiso fatty acid.

In another embodiment, the invention provides a cell comprising an exogenous or overexpressed polynucleotide comprising a nucleic acid sequence encoding a citramalate synthase (such as *M. jannaschii* CimA or a feedback resistant derivative thereof), an exogenous or overexpressed polynucleotide comprising a nucleic acid sequence encoding a branched-chain α-keto acid dehydrogenase (such as *B. subtilis* Bkd), and an exogenous or overexpressed polynucleotide comprising a nucleic acid sequence encoding a 3-ketoacyl-ACP synthase (such as *B. subtilis* FabH), wherein the polynucleotides are expressed in the cell. The cell optionally further comprises at least one exogenous or overexpressed polynucleotide comprising a nucleic acid sequence encoding an isopropylmalate isomerase, an isopropylmalate dehydrogenase, an acetohydroxy acid synthase (such as *E. coli* IlvIH, *E. coli* IlvIH (G14D), *E. coli* IlvGM, or *B. subtilis* IlvBH), a thioesterase, or a combination thereof.

The inventive cell preferably produces more anteiso fatty acid than an otherwise similar cell that does not comprise the polynucleotide(s). Methods of measuring fatty acid released into the fermentation broth or culture media are described herein. Anteiso fatty acid production is not limited to fatty acid accumulated in the culture, however, but also includes fatty acid used as a precursor for downstream reactions yielding products derived from anteiso fatty acid. Thus, products derived from anteiso (or iso) fatty acid (e.g., phospholipids, triglycerides, fatty alcohols, wax esters, fatty aldehydes, and alkanes) are, in some embodiments, surrogates for measuring branched-chain fatty acid production in a host cell. Methods of measuring fatty acid content in phospholipid in the cell membrane are described herein. Similarly, measurement of degradation products of branched-chain anteiso fatty acids also is instructive as to the amount of anteiso fatty acid is produced in a host cell. Depending on the particular embodiment of the invention, the inventive cell produces at least 3%, at least 5%, at least 10%, at least 20%, at least 25%, or at least 50% more anteiso fatty acid than an otherwise similar cell that does not comprise the polynucleotide(s).

The following examples further describe and demonstrate embodiments within the scope of the present invention. The examples are given solely for the purpose of illustration and are not to be construed as limitations of the present invention.

### Example 1. Construction of Bacillus subtilis bkd expression vectors.

This example demonstrates production of a recombinant expression vector for expression of *B. subtilis bkd* in, e.g., *E. coli.*

Genomic DNA was prepared from *B. subtilis* 168 (*Bacillus* Genetic Stock Center, Columbus, OH) by picking a colony from an agar plate, suspending the colony in 100 µl of 1 mM Tris pH 8.0, 0.1 mM EDTA, boiling the sample for five minutes, and removing the insoluble debris by centrifugation.

*B. subtilis bkd* cDNA (SEQ ID NO: 1) (including *lpdv, bkdAA, bkdAB,* and *bkdB* genes that are part of the larger *bkd* operon in *B. subtilis*), was amplified from the genomic DNA sample by polymerase chain reaction (PCR) using primers BKD1 (SEQ ID NO: 2) and BKD2 (SEQ ID NO: 3) 5', which incorporated flanking restriction sites for *Apa*I and *Mlu*I into the *bkd* cDNA during the PCR reaction.

The PCR was performed with 10 µl of *Pfu* Ultra II Hotstart 2X master mix (Agilent Technologies, Santa Clara, CA), 1 µl of a mix of the two primers (10 µmoles of each), 1µl of *B*. *subtilis* genomic DNA, and 8 µl of water. The PCR began with a two-minute incubation at 95°C, followed by 30 cycles of 20 seconds at 95°C for denaturation, 20 seconds for annealing at an optimized temperature of 62°C, and 90 seconds at 72°C for extension. The samples were incubated at 72°C for three minutes and then held at 4°C. The PCR product was purified using a QIAquick® PCR Purification Kit (Qiagen, Valencia, CA) and digested with *Apa*I and *Mlu*I restriction enzymes.

Bacterial expression vector pZA31-MCS (Expressys, Ruelzheim, Germany) was digested with *Apa*I, *Mlu*I, and *Hind*III, and the digested vector and insert were ligated together using Fast-Link (Epicentre Biotechnologies, Madison, WI). The ligation mix was then used to transform *E*. *coli* TOP10 cells (Invitrogen, Carlsbad, CA). Recombinant plasmids were isolated using a QIAPrep® Spin Miniprep Kit (Qiagen) spin plasmid miniprep kit and characterized by gel electrophoresis of restriction digests with *Eco*RV and with *PstI.* Plasmid DNA was isolated, and DNA sequencing confirmed that the *bkd* insert had been cloned and that the insert encoded the published amino acid sequence (Genbank # AL009126.3) (SEQ ID NO: 4). The resulting plasmid was designated *pZA31-Bs bkd.*

### Example 2. Construction of B. subtilis fabHA expression vectors.

This example demonstrates production of recombinant expression vectors for expression of *B. subtilis fabHA* in, e.g., *E. coli.*

To engineer *E. coli* for more efficient incorporation of the 2-methylbutyryl-CoA as a primer in fatty acid synthesis, *E. coli* was transformed with a vector containing *B. subtilis fabHA,* which encodes a 3-ketoacyl-ACP synthase that efficiently acts on 2-methylbutyryl-CoA. *B*. *subtilis* encodes two *fabH* genes whose products catalyze this reaction. Each *fabH* gene was separately cloned.

Genomic DNA was prepared from *B. subtilis* 168 (*Bacillus* Genetic Stock Center, Columbus, OH) by picking an isolated colony from a Luria agar plate, suspending the colony in 50 µL of sterile Milli-Q water (Millipore, Bedford, MA), boiling the sample at 100°C for five minutes, and removing the insoluble debris by centrifugation.

To generate an expression plasmid lacking a polyhistidine tag, *B. subtilis fabHA* cDNA was amplified from the genomic DNA sample by PCR using primers Bs_939_fabHA_nco_U38 (SEQ ID NO: 5) and Bs_939_fabHA_pst_L30 (SEQ ID NO: 6), which incorporated flanking restriction sites for *NcoI* and *PstI* into the amplified cDNA. Because of the use of an *NcoI* site in this cloning construct, an additional three base pairs was added to *fabHA* so that one would predict an extra alanine to be found in the FabHA protein.

To generate an expression plasmid where *fabHA* is fused to a polyhistidine tag, *B. subtilis fabHA* cDNA (SEQ ID NO: 7) was amplified from the genomic DNA sample by PCR using primers Bs_939_fabHA_xho_U38 (SEQ ID NO: 8) and Bs_939_fabHA_pst_E30 (SEQ ID NO: 9), which incorporated flanking restriction sites for *Xho*I and *PstI* into the amplified cDNA.

PCR was run on samples having 1 µl of *B. subtilis* 168 genomic DNA, 1.5 µl of a 10 µM stock of each primer, 5 µl of 10X *Pfx* reaction mix (Invitrogen Carlsbad, CA), 0.5 µl of *Pfx* DNA polymerase (1.25 units), and 41 µl of water. PCR conditions were as follows: the samples were initially incubated at 95°C for one minute, followed by 30 cycles at 95°C for 30 seconds (strand separation), 58°C for 30 seconds (primer annealing), and 68°C primer extension for 1.5 minutes. Following these cycles, there was a ten-minute incubation at 68°C, and the samples were then held at 4°C.

The PCR products were purified using a QIAquick® PCR Purification Kit (Qiagen), double digested with restriction enzymes *Xho*I/*Pst*I or *Nco*I/*Pst*I, and ligated (Fast-Link Epicentre Biotechnologies, Madison, WI) into *Xho*I/*Pst*I or *Nco*I/*Pst*I-digested pBAD/His A (Invitrogen, Carlsbad, CA). The ligation mix was used to transform *E*. *coli* DH5α™ (Invitrogen Carlsbad, CA). Isolated colonies were screened by PCR using a sterile pipette-tip stab as an inoculum into a reaction tube containing only water, followed by addition of the remaining PCR reaction cocktail (AccuPrime™ SuperMixII, Invitrogen Carlsbad, CA) and primers as described above.

Recombinant plasmids were isolated and purified using the QIAPrep® Spin Miniprep Kit (Qiagen) and characterized by restriction enzyme digestion (*Xho*I + *Pst*I, *Nco*I + *Pst*I, *DraI, Mfe*I, and *Hae*II from Invitrogen or New England Biolabs, Beverly, MA). The plasmids were subsequently used to transform *E*. *coli* strain BW25113 (*E. coli* Genetics Stock Center, New Haven, CT) made competent using the calcium chloride method. Transformants were selected on Luria agar plates containing 100 µg/ml ampicillin. Plasmid DNA was isolated and purified using the QIAfilter™ Plasmid Midi Kit (Qiagen). DNA sequencing confirmed that the *fabHA* inserts had been cloned and that the inserts encoded the published amino acid sequence (SEQ ID NO: 10). The resulting plasmid lacking a polyhistidine tag was designated *Bs fabHA*-His and the plasmid incorporating a polyhistidine tag was designated pBAD-Bs *fabHA*+His.

### Example 3. Construction of B. subtilis fabHB expression vectors.

This example demonstrates production of recombinant expression vectors for expression of *B. subtilis fabHB* in, e.g., *E*. *coli.*

Genomic DNA was prepared from *B. subtilis* 168 (*Bacillus* Genetic Stock Center, Columbus, OH) by picking an isolated colony from a Luria agar plate, suspending the colony in 50 µL of sterile Milli-Q water (Millipore, Bedford, MA), boiling the sample at 100°C for five minutes, and removing the insoluble debris by centrifugation.

To generate an expression plasmid lacking a polyhistidine tag, *B. subtilis fabHB* cDNA was amplified from the genomic DNA sample by PCR using primers RC_Bs_978_fabHB_nco_U36 (SEQ ID NO: 11) and RC_Bs-978_fabHB_pst_L32 (SEQ ID NO: 12), which incorporated flanking restriction sites for *Nco*I and *Pst*I into the amplified cDNA. Because of the use of an *Nco*I site in this cloning a predicted serine-to-alanine change was made in the FabHB protein.

To generate an expression plasmid where *fabHB* would be fused to a polyhistidine tag, *B*. *subtilis fabHB* cDNA (SEQ ID NO: 13) was amplified from the genomic DNA sample by PCR using primers RC_Bs_978_fabHB_xho_U41 (SEQ ID NO: 14) and RC_Bs_978_fabHB_pst_L35 (SEQ ID NO: 15), which incorporated flanking restriction sites for *Xho*I and *Pst*I into the amplified cDNA.

PCR was run on samples having 1 µl of *B. subtilis* 168 genomic DNA, 1.5 µl of a 10 µM stock of each primer, 5 µl of 10X *Pfx* reaction mix (Invitrogen Carlsbad, CA), 0.5 µl of *Pfx* DNA polymerase (1.25 units), and 41 µl of water. PCR conditions were as follows: the samples were initially incubated at 95°C for one minute, followed by 30 cycles at 95°C for 30 seconds (strand separation), 58°C for 30 seconds (primer annealing), and 68°C primer extension for 1.5 minutes. Following these cycles, there was a ten-minute incubation at 68°C, and the samples were then held at 4°C.

The PCR products were purified using a QIAquick® PCR Purification Kit (Qiagen), double digested with restriction enzymes *Xho*I/*Pst*I or *Nco*I/*Pst*I, and ligated (Fast-Link Epicentre Biotechnologies, Madison, WI) into *Xho*I/*Pst*I or *Nco*I/*Pst*I-digested pBAD/His A (Invitrogen, Carlsbad, CA). The ligation mix was used to transform *E*. *coli* DH5α™ (Invitrogen Carlsbad, CA). Isolated colonies were screened by PCR using a sterile pipette-tip stab as an inoculum into a reaction tube containing only water, followed by addition of the remaining PCR reaction cocktail (AccuPrime™ SuperMixII, Invitrogen Carlsbad, CA) and primers as described above.

Recombinant plasmids were isolated and purified using the QIAPrep® Spin Miniprep Kit (Qiagen) and characterized by restriction enzyme digestion (*Xho*I + *Pst*I, *Nco*I + *Pst*I, *Dra*I, *Mfe*I,i and *Hae*II from Invitrogen or New England Biolabs, Beverly, MA). The plasmids were subsequently used to transform *E*. *coli* strain BW25113 (*E. coli* Genetics Stock Center, New Haven, CT) made competent using the calcium chloride method. Transformants were selected on Luria agar plates containing 100 µg/ml ampicillin. Plasmid DNA was isolated and purified using the QIAfilter™ Plasmid Midi Kit (Qiagen). DNA sequencing confirmed that the *fabHB* inserts had been cloned and that the inserts encoded the FabHB amino acid sequence (SEQ ID NO: 16). The resulting plasmid lacking a polyhistidine tag was designated *Bs fabHB*-His and the plasmid incorporating a polyhistidine tag was designated pBAD-*Bs fabHB*+His.

### Example 4. Co-transformation of E. coli with B. subtilis fabHA, fabHB and bkd genes.

This example demonstrates the co-transformation of *E*. *coli* with *B. subtilis fabHA, fabHB* and *bkd* genes.

To produce anteiso fatty acids in *E. coli,* combinations of each of the *B. subtilis fabH* plasmid constructs (Examples 2 and 3), with and without the *B. subtilis bkd* plasmid construct (Example 1), were used to transform both parent BW25113 and knockout BW25113 *ΔfadD*730 strains (*E. coli* Genetic Stock Center, New Haven, CT). The *ΔfadD*730 strain has a deleted acyl-CoA synthase gene. Acyl-CoA synthase is an enzyme in the fatty acid degradation pathway, and deletion of the acyl-CoA synthase gene increases fatty acid content in the host cell by attenuating the cell's natural fatty acid degradation pathway.

The two *E*. *coli* strains were made chemically competent for plasmid DNA transformation by a calcium chloride method. Actively growing 50 ml *E. coli* cultures were grown to an optical density (at 600 nm) of ~0.4. Cultures were quickly chilled on ice, and the bacteria were recovered by centrifugation at 2700xg for 10 minutes. The supernatant was discarded and pellets were gently suspended in 30 ml of an ice-cold 80 mM MgCl₂, 20 mM CaCl₂ solution. Cells were again recovered by centrifugation at 2700xg for 10 minutes. The supernatant was discarded and pellets were gently resuspended in 2 ml of an ice-cold 0.1 M CaCl₂ solution.

The competent cells were used directly for the following co-transformations:
*p*BAD*-Bs fabHA* and pZA31-*Bs bkd*
pBAD-*Bs fabHA*-His and pZA31-*Bs bkd*
pBAD-*Bs fabHB* and *pZA31-Bs bkd*
pBAD-*Bs fabHB*-His and *pZA31-Bs bkd*

Cells were transformed on ice in pre-chilled 14 ml round bottom centrifuge tubes. Approximately 25 ng of each plasmid was incubated on ice with 100 µl of competent cells for 30 minutes. The cells were heat shocked at 42°C for 90 seconds and immediately placed on ice for 2 minutes. 500 µl of pre-warmed SOC medium (Invitrogen, Carlsbad, CA) was added and the cells allowed to recover at 37°C with 225 rpm shaking. 50 µl of the transformed cell mix was spread onto selective LB agar 100 µg/ml ampicillin plates to select for cells carrying the BAD-*Bs fabH* plasmids. 50 µl of the transformed cell mix was spread onto selective LB agar 34 µg/ml chloramphenicol plates to select for cells carrying the pZA31-*Bs bkd* plasmid. 150 µl of the transformed cell mix was spread onto selective LB agar 100 µg/ml ampicillin and 34 µg/ml chloramphenicol plates to select for cells carrying both the pBAD-*Bs fabH* and pZA31-*Bs bkd* plasmids.

Individual colonies were picked from each plate and streaked onto all three varieties of LB agar plates to confirm the antibiotic resistance phenotype. Each strain was streaked to a single colony density, and a single colony was selected to be amplified for plasmid DNA isolation with QIAprep Spin Miniprep Kits (Qiagen, Valencia, CA). Restriction endonuclease digestion analysis of isolated plasmid DNA with *Hae*II verified the plasmid DNA pool for each strain.

### Example 5. Construction of an expression vector for the expression of medium branched-chain fatty acid thioesterase from Mallard uropygial gland.

This example demonstrates the construction of an expression vector for the expression of a medium branched-chain fatty acid thioesterase from Mallard uropygial gland.

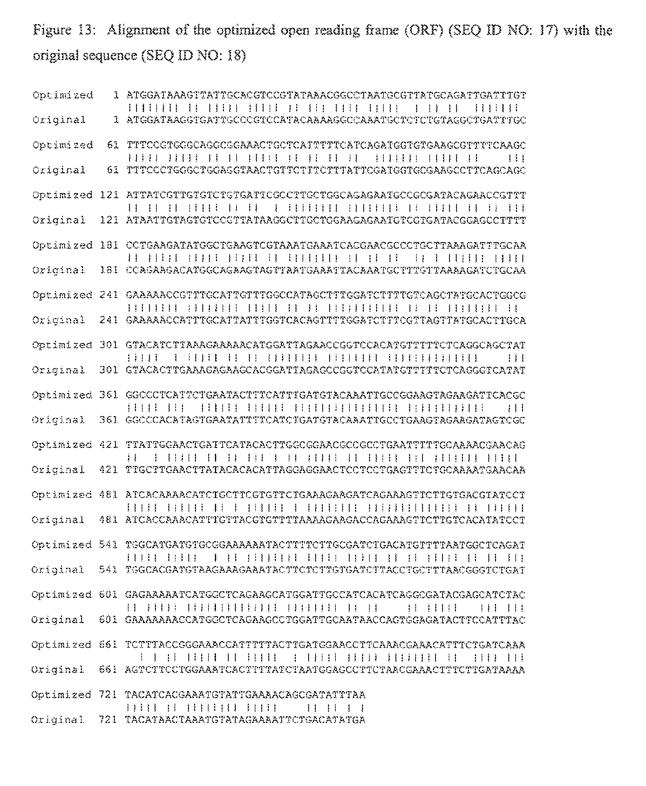

The coding sequence of the thioesterase (AAA49222.1) was codon optimized for *B*. *subtilis* expression. An alignment of the optimized open reading frame (ORF) (SEQ ID NO: 17) with the original sequence (SEQ ID NO: 18) is shown in Figure 13. The optimized ORF was synthesized (GenScript) and inserted between the *Nco*I and *Bam*HI sites of expression vector pTrcHisA (Invitrogen).

### Example 6. Construction of an expression vector for the expression of medium branched-chain fatty acid thioesterase from rat mammary gland.

This example demonstrates construction of the vector for the expression of a medium branched-chain fatty acid thioesterase from rat mammary gland.

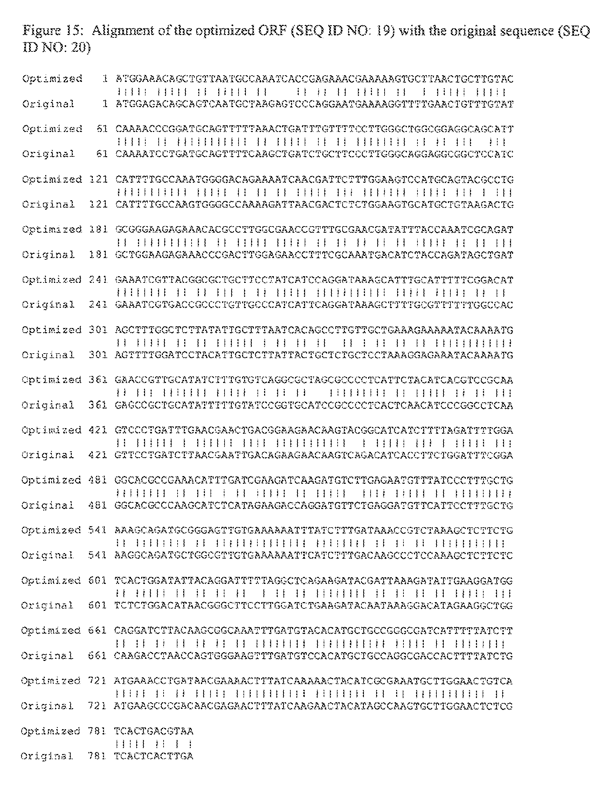

The coding sequence (AAA41578.1) was codon optimized for *B. subtilis* expression. An alignment of the optimized ORF (SEQ ID NO: 19) with the original sequence (SEQ ID NO: 20) is shown in Figure 15. The optimized ORF was synthesized (GenScript) and inserted between the *Nco*I and *Bam*HI sites of expression vector pTrcHisA (Invitrogen).

### Example 7. Extraction of lipids from culture medium.

This example demonstrates the extraction of lipids from the culture medium.

Lipids released from cells were extracted as follows: *E. coli* cells were grown in Luria Broth, Miller (BD, Sparks, MD) to an optical density (600 nm) of at least 2 absorbance units. After centrifugation to pellet the cells, the supernatant was transferred to a fresh tube, and hydrochloric acid was added to a final pH between 1 and 2. Alternatively, to concentrate the supernatant, 25-50 ml can be lyophilized (VirTis, Gardiner, NY) and suspended in 1 ml water. New or solvent-cleaned all-glass Pyrex tubes (Corning, Lowell, MA) were used for all subsequent steps. Three ml of 1:2 (v/v) chloroform:methanol was added to each 1 ml of supernatant sample. To the tube containing the supernatant, 1 ml of sterile Milli-Q (Millipore, Bedford, MA) water was added followed by 1 ml of chloroform. After briefly centrifuging the tubes (1000 rpm) for five minutes at room temperature, the top aqueous phase was removed, and the bottom organic phase was transferred to a clean, pre-weighed, and labeled 2 ml V-Vial® (15 - 415, diam. × H 17 mm × 61 mm; Corning). Samples were dried under nitrogen or open air.

### Example 8. Phospholipid hydrolysis, extraction of fatty acids from cells, and esterification of fatty acids.

This example describes phospholipid hydrolysis, extraction of fatty acids from cells, and esterification of fatty acids.

Fatty acids were extracted from the cells as follows: *E*. *coli* cells were grown in Luria Broth, Miller to an optical density (600 nm) of at least 2 absorbance units. After centrifugation (3700 rpm for 10 minutes) to pellet the cells, the supernatant was discarded. Two ml of 0.1 M NaCl + 50 mM Tris-HCl (pH between 7.5-8.0) was added to the pellet, the tube was vortexed, spun (3700 rpm for 10 minutes) to pellet the cells, and the supernatant was discarded. Sterile Milli-Q water (2 ml) was added to the tube containing the cell pellet, vortexed thoroughly, and the tube contents were transferred to a clean, pre-weighed and labeled Corning V-Vial® with solid-top cap capacity (2.0 ml, screw-cap size, 415, diam. × H: 17 mm × 61 mm). Aluminum foil was placed over vials containing the 2 ml pellet, and the sample was frozen at -80°C for 30 minutes and placed into the Virtis Freezemobile (VirTis, Gardiner, NY) lyophilizer overnight (at 27°C).

Dry weights of lyophilized samples were recorded, chloroform (0.75 ml) and 15% sulfuric acid (in methanol) were added, and tubes were placed in a 100°C heating block (in a shielded fume hood). After four hours, the reaction mixture was transferred using a glass Pasteur pipette to a 13 x 100 mm Pyrex tube (Corning). Chloroform (1 ml) and 1 M sodium chloride (1 ml) were added to each tube and mixed by hand prior to a brief spin at 1000 rpm for 5 minutes. Using a glass Pasteur pipette, the top aqueous layer was discarded and a saturating amount of anhydrous sodium sulfate (~50 mg) was added to the tube. The remaining volume (~ 1 ml) was carefully removed using a Pasteur pipette and transferred to a pre-weighed glass GC tube. The tube can be dried under nitrogen or overnight in a fume hood. To the dried tube, 1g (~700 µL) of chloroform was added along with 0.1 g (~70µL) of a 0.1g/l methyl benzoate solution.

### Example 9. Analysis of fatty acid methyl esters.

This example demonstrates gas chromatography and mass spectrometry analysis of fatty acid methyl esters.

Fatty acid methyl esters were analyzed by gas chromatography, using hydrogen as a carrier gas at an initial flow rate of 1 cm³/sec. The injector temperature was set at 275°C and the FID detector at 340°C. The oven temperature was kept at 70°C for 1 minute following a 1µL injection (50:1 split) with a temperature ramp of 10°C/min or 3°C/min to 325°C. The siloxane column used on the HP GC 6890 was a J&W Scientific DB-1 (part #122-1131), 60 m x 0.25 mm ID x 0.1 µm film thickness.

Fatty acid methyl esters were also analyzed by gas chromatography and mass spectrometry, using helium as a carrier gas at an initial flow rate of 0.9 ml/min (7.98 psi, 36cm/sec). The injector temperature was set at 250°C. The oven temperature was kept at 70°C for one minute following a 1µL injection (20:1 split) with a temperature ramp of 10°C/min to 325°C. The column type used on the HP GC 6890 was a HP-5 Crosslinked 5% PhMe (Silicone; HP Part No. 19091J-433) with a 30 m x 0.25 mm x 0.25 µm film thickness.

### Example 10. Production of anteiso and iso fatty acids by BW25113 harboring pBAD-Bs fabHA-His and pZA31-Bs bkd.

This example demonstrates the production of anteiso and iso fatty acids by BW25113 harboring pBAD-*Bs fabHA*-His and pZA31-*Bs bkd.*

Cells (50 ml) were cultured in Luria broth (BD, Sparks, MD). With the culture at an optical density (600 nm) of 0.4-0.6, arabinose (0.2%) was added to induce *fabHA* expression. Lipid was harvested from the cell pellet (Example 8) and examined by gas chromatography (Example 7), revealing peaks that matched the mobility of C15 anteiso fatty acid standards. The identification of these peaks was confirmed by gas chromatography followed by mass spectrometry.

This example illustrates a method of producing anteiso and iso fatty acids in a microbe that does not naturally produce anteiso fatty acids (*E. coli*) by expressing in the microbe heterologous polynucleotides encoding a 3-ketoacyl-ACP synthase (*fabHA* from *B. subtilis*) and a branched-chain α-keto acid dehydrogenase (*bkd* from *B. subtilis*).

### Example 11. Increasing anteiso or iso fatty acid production by increasing the respective precursors isoleucine, leucine or valine.

This example demonstrates a method of increasing anteiso or iso fatty acid production by increasing precursors isoleucine, leucine or valine.

BW25113 harboring pBAD-*Bs fabHA*-His and pZA31-*Bs bkd* was cultured and its fatty acid profile characterized as described in Example 10. To demonstrate the influence of available isoleucine, a parallel culture was prepared in the presence of one gram per liter isoleucine.

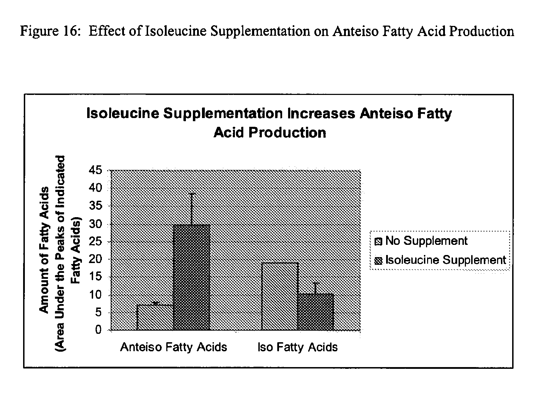

The samples were separated by gas chromatography. The peak areas were calculated using an algorithm in ChemStation® software Rev A.06.06 [509]. These peak sizes were added for all anteiso fatty acids and, separately, for all iso fatty acids. The presence of 1 gram per liter isoleucine was associated with an increase in anteiso fatty acids and a decrease in iso fatty acids, as shown in Figure 16.

The results of this example show that increasing carbon flow to the isoleucine pathway of branched fatty acid synthesis increases the amount of anteiso branched-chain fatty acid produced in the host cell.

### Example 12. Analysis of fatty acids.

This example describes a method for analyzing fatty acids, such as fatty acids produced in bacterial cells, using gas chromatography.

Samples for analysis were prepared as follows. Bacterial cultures (approximately 1.5 ml) were frozen in 2.0 ml glass vials and stored at -15°C until ready for processing. Samples were chilled on dry ice for 30 minutes and then lyophilized overnight (~16 hours) until dry. A 10 µl aliquot of internal standard (glyceryl trinonadecanoate (Sigma catalog number T4632-1G)) was added to each vial, followed by addition of 400 µL of 0.5 N NaOH (in methanol). The vial was capped and vortexed for 10 seconds. Samples were then incubated at 65°C for 30-50 minutes, removed from the incubator, and 500 µl of boron trifluoride reagent (Aldrich catalog number B1252) was added. The samples were vortexed for 10 seconds. Samples were then incubated at 65°C for 10-15 minutes and cooled to room temperature (approximately 20 minutes). Hexane (350 µl) was added, and the samples were vortexed for 10 seconds. If the phases did not separate, 50-100 µl of saturated salt solution (5 g NaCl to 5 ml water) was added, and the sample again vortexed for 10 seconds. At least 100 µl of the top hexane layer was placed into a gas chromatography (GC) vial, which was capped and stored at 4°C or -20°C until analysis.

Gas chromatography was performed as described in Table B below. A bacterial acid methyl ester standard (Sigma catalog number 47080-U) and a fatty acid methyl ester standard (Sigma catalog number 47885-U) were used to identify peaks in samples. A sample check standard using glyceryl tripalmitate 1 (Sigma catalog number T5888-1G) was employed to confirm esterification of samples. A blank standard (internal standard only) was used to assess background noise.

**TABLE B**

| | | | | | | |
|---|---|---|---|---|---|---|
| Gas Chromatograph | HP 5890 GC Series II | | | | | |
| Detector | FID 360°C 40 ml/min Hydrogen, 400 ml/min Air | | | | | |
| Carrier Gas | Helium | | | | | |
| Quantitative Program | GC Chemstation A.09.03. (Agilent) | | | | | |
| Column | VF-5ms 15M x 0.150mm x 0.15 µm Varian catalog number CP9035 | | | | | |
| Injection Liner | Gooseneck (with glass wool packing) | | | | | |
| Injector | HP 7673 | | | | | |
| Injection Syringe | 10 µL | | | | | |
| Injection Mode | Split 25:1 | | | | | |
| Injection volume | 4 µL (Plunger Speed = fast; 5 sample pumps) | | | | | |
| Pre Injection Solvent Washes | 2 samples | | | | | |
| Post Injection Solvent Washes | 3 for both acetone and hexane | | | | | |
| Injector Temperature | 325°C | | | | | |
| Total Program Time | 16 minutes | | | | | |

| Thermal Program | | Initial Temp. (°C) | Initial Time (min) | Rate (°C/min) | Final Temp (°C) | Final Time (min) |
|---|---|---|---|---|---|---|
| | | 90 | 0.75 | 20.0 | 325 | 1.0 |
| | | | | 25.0 | 350 | 2.5 |

### Example 13. Increasing anteiso fatty acid production by increasing carbon flow through the threonine-dependent pathway.

There are two primary pathways responsible for production of 2-oxobutanoate (also known as α-ketobutyrate), which is an intermediate in the synthesis of 2-methylbutyryl-CoA, the primer for anteiso fatty acid synthesis. One pathway generates 2-oxobutanoate from threonine (Figure 2), while the second pathway uses citramalate as a precursor (Figure 17). This example demonstrates that increasing carbon flow through a pathway utilizing threonine increases anteiso fatty acid production in host cells.

An *E*. *coli* strain was modified to increase production of threonine deaminase and, in some instances, acetohydroxy acid synthase (AHAS). Threonine deaminase and AHAS are the first two enzyme activities in the threonine-dependent pathway for anteiso fatty acid production. Threonine deaminase promotes the conversion of threonine to 2-oxobutanoate, which is converted to 2-aceto-2-hydroxy-butyrate via AHAS. An expression vector comprising an *E*. *coli* threonine deaminase coding sequence, *tdcB,* operably linked to a trc promoter was constructed. An expression vector comprising a gene fusion wherein an AHAS III coding sequence, *ilvIH,* is fused to the *tdcB* coding sequence also was prepared.

To isolate *tdcB,* genomic DNA was prepared from *E*. *coli* BW25113 (*E*. *coli* Genetic Stock Center, Yale University, New Haven, CT) by picking an isolated colony from a Luria agar plate, suspending the colony in 100 µl Tris (1 mM; pH 8.0), 0.1 mM EDTA, boiling the sample for five minutes, and removing the insoluble debris by centrifugation. *tdcB* was amplified from the genomic DNA sample by PCR using primers GTGCCATGGCTCATA TTACATACGATCTGCCGGTTGC (SEQ ID NO: 2) and GATCGAATTCATCCTTAGGCGTCAACGAAACCGGTGATTTG (SEQ ID NO: 3). PCR was performed on samples having 1 µl of *E*. *coli* BW25113 genomic DNA, 1 µl of a 10 µM stock of each primer, 25 µl of *Pfu* Ultra II Hotstart 2X master mix (Agilent Technologies, Santa Clara, CA), and 22 µl of water. PCR conditions were as follows: the samples were initially incubated at 95°C for two minutes, followed by three cycles at 95°C for 20 seconds (strand separation), 56°C for 20 seconds (primer annealing), and 72°C primer extension for 30 seconds. In addition, 27 cycles were run at 95°C for 20 seconds, 60°C for 20 seconds, and 72°C primer extension for 30 seconds. There was then a three-minute incubation at 72°C, and the samples were held at 4°C.

The PCR products were purified using a QIAquick® PCR Purification Kit (Qiagen), double digested with restriction enzymes *Hind*III and *Nco*I, and ligated (Fast-Link Epicentre Biotechnologies, Madison, WI) with *Hind*III/Nc.oI-digested pTrcHisA vector (Invitrogen, Carlsbad, CA). The ligation mix was used to transform OneShot Top10™ *E*. *coli* cells (Invitrogen, Carlsbad, CA). Transformants were selected on Luria agar plates containing 100 µg/ml ampicillin. The recombinant plasmid was isolated using a Qiagen HiSpeed Plasmid Midi Kit and characterized by gel electrophoresis of restriction digests with *Hind*III and *Nco*I*.* DNA sequencing confirmed that the *tdcB* insert had been cloned and that the insert encoded the published amino acid sequence (Genbank number U00096.2) (SEQ ID NOs: 4 and 33). The resulting plasmid was designated pTrcHisA *Ec tdcB.*

A gene fusion was constructed wherein AHAS genes were placed behind *Ec tdcB* so that both TdcB and the recombinant AHAS would be produced from the same message. In some instances, AHAS is encoded by two subunits. For example, *E. coli* AHAS III is encoded by two genes, *IlvI* (SEQ ID NO: 34) and *IlvH* (SEQ ID NO: 35). To fuse the AHAS III genes, *ilvIH* (SEQ ID NO: 36), to *tdcB, ilvIH* was amplified from the *E. coli* BW25113 genomic DNA sample PCR using primer sequences set forth in SEQ ID NO: 37 and SEQ ID NO: 38, which incorporated flanking restriction sites for *Eco*RI onto *ilvIH* during the PCR reaction.

The PCR was performed with 25 µl of *Pfu* Ultra II Hotstart 2X master mix (Agilent Technologies, Santa Clara, CA), 1 µl of a mix of the two primers (10 µmoles of each), 1 µl of *E*. *coli* BW25113 genomic DNA, and 23 µl of water. The PCR began with a two-minute incubation at 95 °C, followed by two cycles of 20 seconds at 95 °C for denaturation, 20 seconds for annealing at 55°C, and 90 seconds at 72°C for extension. The product was further amplified by 28 cycles of 20 seconds at 95°C for denaturation, 20 seconds for annealing at 62°C, and 90 seconds at 72°C for extension. The samples were incubated at 72°C for three minutes and then held at 4°C. The PCR product was purified using a QIAquick® PCR Purification Kit (Qiagen, Valencia, CA) and digested with *Eco*RI restriction enzyme.

The bacterial expression vector pTrcHisA *Ec tdcB* (prepared as described above) was digested with *EcoRI,* and the digested vector and insert were ligated using Fast-Link (Epicentre Biotechnologies, Madison, WI). The ligation mix was then used to transform *E. coli* TOP10 cells (Invitrogen, Carlsbad, CA). Recombinant plasmids were isolated using a QIAPrep® Spin Miniprep Kit (Qiagen) and characterized by gel electrophoresis of restriction digests with *Xmn*I. DNA sequencing confirmed that the *ilvIH* insert had been cloned and that the insert encoded the published amino acid sequences (*ilvI,* Swiss-Prot # P00893.2; *ilvH* Swiss-Prot # P00894.3) (SEQ ID NO: 39 and SEQ ID NO: 40, respectively). The resulting plasmid was designated pTrc *Ec tdcB Ec ilvIH.*

Carbon flow to 2-oxobutanoate is increased by the use of an AHAS III that is feedback insensitive to valine. Valine insensitivity is conferred by, for example, substituting an aspartic acid for glycine at the fourteenth amino acid (G14D) of IlvH (SEQ ID NO: 41; Vyazmensky et al., Biochemistry, 35: 10339-46 (1996)). An expression vector for expressing an *E. coli tdcB* gene followed by an *E*. *coli ilvIH* G14D was prepared. The fourteenth codon of *E. coli ilvH,* GGC (encoding glycine) was mutated to GAC (encoding aspartic acid) by site-directed mutagenesis ("SDM") (GenScript, Piscataway, NJ) using the plasmid pTrc *Ec tdcB Ec ilvIH* as a template. The generated SDM variant region was sub-cloned back into the original pTrc-*Ec tdcB Ec ilvIH* template, using an *Aat*I site in the large subunit *E. coli ilvI* gene and an *Xba*I site in the multiple cloning site (MCS). The SDM variant region DNA sequence is provided as SEQ ID NO: 42, and the corresponding original DNA sequence is provided as SEQ ID NO: 43. DNA sequencing confirmed the SDM product as *ilvIH* (G14D). The SDM *ilvH* G14D open reading frame (ORF) is presented as SEQ ID NO: 41. Restriction digest of plasmid DNA with *Afl*III confirmed the presence of a new *Afl*III site created by the SDM. The resulting plasmid was designated pTrc *Ec tdcB Ec ilvIH* G14D.

*E. coli* AHAS II is the product of two genes, *ilvG* (SEQ ID NO: 44) and *ilvM* (SEQ ID NO: 45). AHAS II is not functionally active in *E. coli* K-12 strains due to a mutation in *ilvG.* To generate active AHAS II, *ilvG* and *ilvM* were synthesized according to the genome sequences of BL21 (DE3) (GenBank accession No. CP001509.3 from base 3840800 to 3842706). A *NotI* site was added to the 5' end of *ilvG* and an *EcoRI* site was added to the 3' end of *ilvM.* The synthesized gene (SEQ ID NO: 46) was ligated to pTrcHisA *Ec tdcB* at the *NotI* and *Eco*RI sites following *tdcB.* Both *tdcB* and *ilvGM* are designed to be transcribed by the same promoter. DNA sequencing confirmed that the *ilvGM* insert had been cloned and that the insert encoded the published amino acid sequences (GenBank Accession No. CAQ34112 (*ilvG*) and GenBank CAQ34113 (*ilvM*); SEQ ID NO: 47 and SEQ ID NO: 48, respectively). The resulting plasmid was designated pTrc *Ec tdcB Ec ilvGM.*

Similar to other AHAS enzymes, *B. subtilis* AHAS comprises products from two genes, *ilvB* (SEQ ID NO: 49) and *ilvH* (SEQ ID NO: 50). The *B. subtilis* AHAS genes were synthesized (GenScript, Piscataway, NJ) using sequences from strain 168. An internal *EcoRI* site was present in the natural gene but removed from the synthetic gene to facilitate subsequent subcloning. A *NotI* site was added to the 5' end of the *ilvB* sequence and an *EcoRI* site was added to the 3' end of the *ilvH* sequence. The synthesized genes (SEQ ID NO: 51) were ligated to pTrcHisA *Ec tdcB* at the *NotI* and *EcoRI* sites following *tdcB.* Both *tdcB* and *ilvBH* are designed to be transcribed by the same promoter. DNA sequencing confirmed that the *ilvBH* insert had been cloned and that the insert encoded the published amino acid sequences (GenBank Accession No. CAA99561 (*ilvB*) and Swiss-Prot No. P37252.2 (*ilvH*); SEQ ID NO: 52 and SEQ ID NO: 53, respectively). The resulting plasmid was designated pTrc *Ec tdcB Bs ilvBH.*

To allow for *E. coli* to be transformed with an increased number of expression vectors, a portion of the polyhistidine-tagged *B. subtilis fabHA* vector (Example 2; pBAD Bs *fabHA*+His), including the regulation control gene *araC* and *araBAD* promoter (SEQ ID NO: 54), was cloned into a vector containing *B. subtilis bkd* (including *lpdV, bkdAA, bkdAB, and bkdB* genes of the larger *bkd* operon) (pZA31 *Bs bkd*).

The ClonEZ PCR cloning Kit (GenScript, Piscataway, NJ) was utilized as follows: the target region was first amplified from the linearized pBAD *Bs fabHA*+His plasmid template by PCR using a (i) 5' primer (SEQ ID NO: 55) containing 15 base pairs of homology sequence downstream (and including) the *Mlu*I site of pZA31 *Bs bkd*, and (ii) a 3' primer (SEQ ID NO: 56) containing 15 base pairs of homology sequence upstream (and including) the *Mlu*I site of *pZA31-Bs bkd.* PCR was performed with 25 µl of *Pfu* Ultra II Hotstart 2X master mix (Agilent Technologies, Santa Clara, CA), 1 µl of a mix of the two primers (10 µmoles of each), 1 µl of linearized pBAD *Bs fabHA*+*His* (20 ng) plasmid DNA, and 23 µl of water. The PCR began with a two minute incubation at 95°C, followed by 30 cycles of 20 seconds at 95°C for denaturation, 20 seconds for annealing at 62°C, and 90 seconds at 72°C for extension. The samples were incubated at 72°C for three minutes and then held at 4°C. The PCR product was purified using a QIAquick® PCR Purification Kit (Qiagen, Valencia, CA).

The ClonEZ reaction was set up with 6 µl (105 ng) of *pZA31 Bs bkd* (restriction digested with *Mlu*I), 8 µl (211 ng) of PCR-amplified insert, 2 µl of 10X ClonEZ buffer, 2 µl of 10X ClonEZ enzyme mix, and 2 µl of distilled water. The reaction proceeded for 30 minutes at 22°C. Some (8 µl) of the reaction mix was used to transform *E. coli* TOP-10 competent cells (Invitrogen, Carlsbad, CA). Isolated colonies were screened by PCR. Recombinant plasmids were isolated using a QIAPrep® Spin Miniprep Kit (Qiagen) and characterized by gel electrophoresis of restriction digests with *Hae*II and with *Eco*RV. DNA sequencing confirmed that the *Ec araC Bs fabHA* insert had been cloned into pZA31 *Bs bkd* and that the insert sequence matched the template sequence. The resulting plasmid was designated *pZA31 Bs bkd fabHA.*

An *E. coli* strain deficient in fatty acid degradation (Voelker et al., J. Bacteriology, 176: 7320-7327 (1994)) and able to regulate transcription of recombinant genes was generated. An *E*. *coli* K-12 strain defective *in fadD,* thus lacking fatty acyl-CoA synthetase, was used as starting material. The strain K27 (F-, *tyrT58*(AS), *fadD88, mel-1;* CGSC Strain No. 5478) was obtained from the *E. coli* Genetic Stock Center (New Haven, CT). A genomic regulation cassette from strain DH5αZ1 *[laci^{q},* PN25-*tetR*, Sp^{R}, *deoR, supE44, Δ*(*lacZYA-argFV169*), ϕ80 *lacZΔM15* (Expressys, Ruelzheim, Germany)] was transducted into the host strain. The transducing phage P1ᵥᵢᵣ was charged with DH5αZ1 DNA as follows. A logarithmically growing culture (5 ml LB broth containing 0.2% glucose and 5 mM CaCl₂) of donor strain, DH5αZ1, was infected with 100 µl of a lysate stock of P1ᵥᵢᵣ phage. The culture was further incubated three hours for the infected cells to lyse. The debris was pelleted, and the supernatant was further cleared through a 0.45 µm syringe filter unit. The fresh lysate was titered by spotting 10 µL of serial 1:10 dilutions of lysate in TM buffer (10 mM MgSO₄/10 mM Tris•Cl, pH 7.4) onto a 100 mm LB (with 2.5 mM CaCl₂) plate overlayed with a cultured lawn of *E. coli* in LB top agar (with 2.5 mM CaCl₂). The process was repeated using the newly created phage stock until the phage titer surpassed 10⁹ pfu/mL.

The higher titer phage stock was used to transduce fragments of the DH5aZ1 genome into a recipient K27 strain as follows. An overnight culture (1.5 ml) of K27 was pelleted and resuspended in 750 µl of a P1 salts solution (10 mM CaCl₂/5 mM MgSO₄). An aliquot (100 µl) of the suspended cells was inoculated with varying amounts of DH5αZ1 donor P1ᵥᵢᵣ lysate (1, 10, and 100 µl) in sterile test tubes. The phage was allowed to adsorb to the cells for 30 minutes at 37°C. The absorption period was terminated by addition of 1 ml LB broth plus 200 µl of 1 M sodium citrate, and the cultures were further incubated for 1 hour at 37°C with aeration. The cultures were pelleted, the cells suspended in 100 µl of LB broth (plus 0.2 M sodium citrate), and were spread onto LB agar plates with 50 µg/mL spectinomycin. Spectinomycin-resistant strains were isolated, and genomic DNA was screened by PCR for the presence of *tetR, lacI^{q}* and *fadD88.* One such transductant was designated K27-Z1 and used in further studies.

To transform K27-Z1 cells, competent cells were placed on ice in pre-chilled 14 ml round bottom centrifuge tubes. Approximately 30 ng of each plasmid was incubated with 50 µl of chemically competent K27-Z1 cells (Cohen et al., Proceedings National Academy Sciences U.S.A., 69: 2110-4 (1972)) for 30 minutes. The cells were heat shocked at 42°C for 90 seconds and immediately placed on ice for two minutes. Pre-warmed SOC medium (250 µl) (Invitrogen, Carlsbad, CA) was added, and the cells were allowed to recover at 37°C with 125 rpm shaking for one hour. Transformed cell mix (20 µl) was spread onto selective LB agar with 100 µg/ml ampicillin to select for cells carrying any of the pTrc-HisA-based plasmids. Transformed cell mix (50 µl) was spread onto LB agar with 34 µg/ml chloramphenicol to select for cells carrying the pZA31 *Bs bkd Bs fabH* plasmid. Transformed cell mix (150 µl) was spread onto LB agar with 100 µg/ml ampicillin and 34 µg/ml chloramphenicol to select for cells carrying both the pTrc-HisA-based and pZA31 *Bs bkd Bs fabH* plasmids. In some cases, the creation of triple transformants required two transformations: a double transformant was originally created, made competent, and transformed by a third plasmid.

Individual colonies were picked for each strain and streaked to a single colony density on appropriate antibiotic selection plates. A single colony was selected to be amplified for plasmid DNA isolation with QIAprep Spin Miniprep Kits (Qiagen, Valencia, CA). Restriction endonuclease digestion analysis with *Afl*III of isolated plasmid DNA verified the plasmid DNA pool for each strain.

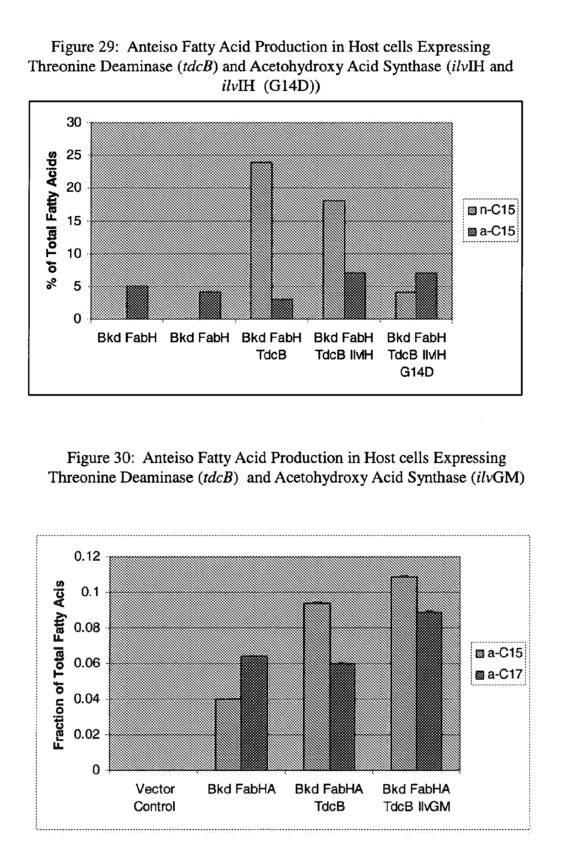

The resulting expression vectors were introduced into *E. coli* host cells comprising *B*. *subtilis bkd* and *fabH,* which were cultured in M9 glycerol medium comprising IPTG, tetracycline, and arabinose to induce recombinant gene expression. A sample of K27-Z1 comprising *pZA31 Bs bkd fabHA* and pTrc *Ec tdcB Ec ilvGM* was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE]. Other AHAS genes also were tested for enhancement of anteiso fatty acid production. Fatty acids produced by the bacterial cells were isolated and separated. The amount of anteiso fatty acid produced by the modified bacteria was compared to the amount of fatty acid produced by an unmodified parental *E. coli* strain and *E*. *coli* producing *B. subtilis* Bkd and FabH. The quantity of each type of fatty acid was divided by the total amount of fatty acids produced. Unexpectedly, host cells expressing *tdcB* exhibited a decrease in anteiso fatty acid production. Co-expression of *Ec tdcB* (encoding threonine deaminase) and *Ec ilvIH* genes (encoding AHAS III) increased anteiso C15 fatty acid production in the *E*. *coli* strain also carrying *Bs fabH* and *Bs bkd,* relative to the *E*. *coli* strain carrying *Bs fabH Bs bkd* and that was not modified with the threonine-dependent pathway enzymes. Increased anteiso fatty acid production was observed for the valine-insensitive *ilvIH* G14D (Figure 27), *Ec ilvIH* (Figures 28 and 29), the exogenous *B. subtilis* gene *Bs ilvBH* (Figure 28), and *E*. *coli ilvGM* (Figure 30).

The results of this example demonstrate that genetic modifications designed to increase carbon flow through the threonine-dependent pathway enhances anteiso fatty acid production.

### Example 14. Increasing anteiso fatty acid production by increasing carbon flow through the citramalate-dependent pathway.

This example describes the generation of a recombinant microbe that produces exogenous citramalate synthase to further increase anteiso fatty acid production.

The native *Methauococcus jauuaschii* citramalate synthase coding sequence also was mutated through directed evolution to improve enzyme activity and feedback resistance to create *cimA3.7* (SEQ ID NO: 58) (Atsumi et al., Applied and Environmental Microbiology 74: 7802-8 (2008)). *E. coli* is not known to have citramalate synthase activity, and a strain was engineered to produce exogenous citramalate synthase while overproducing several native *E. coli* enzymes: LeuB, LeuC, LeuD, and each of several AHASs. Citramalate synthase, LeuB, LeuC, LeuD, and IlvIH (G14D) mediate the first five chemical conversions in the citramalate pathway to produce anteiso fatty acids (Figure 17).

To generate a synthetic CimA3.7 gene codon-optimized for *E. coli* expression, a DNA fragment (SEQ ID NO: 57) containing a restriction site *Bsp*HI (bases 1-6), codon-optimized *cimA3.7* fragment (bases 3-1118), stop codon TGA (bases 1119-1121), a fragment of 52 bases from the start of the *E. coli leuB* gene (bases 1121-1173), and a linker sequence (bases 1174-1209) containing *Not*I, *Pac*I, *Pme*I, *Xba*I and *Eco*RI sites was synthesized (GenScript, Piscataway, NJ). The stop codon of *cimA3.7* (TGA) and start codon (ATG) of *leuB* overlaps one base (A), presumably to enable translational coupling. This overlap mimics the native *leuA* and *leuB* coupling in *E. coli.* The synthesized fragment was digested with *Bsp*HI and *Eco*RI and cloned into pTricHisA (Invitrogen) at the *Nco*I and *Eco*RI sites, using the compatible ends generated by *Bsp*HI and *Nco*I. The end of the *leuB* fragment (bases 1168-1173) also contains a *Bsp*EI site (underlined) for cloning of *leuBCD.* This vector was designated as pTrcHisA *Mj cimA.*

The *leuB* (SEQ ID NO: 59) gene encodes 3-isopropylmalate dehydrogenase. The *leuC* (SEQ ID NO: 60) and *leuD* (SEQ ID NO: 61) genes encode isopropylmalate isomerase large subunit and small subunit, respectively. To fuse the three-gene complex *leuBCD* (SEQ ID NO: 57) behind *Mj cimA, E. coli leuBCD* cDNA was amplified from an *E. coli* BW25113 genomic DNA sample using PCR primers (SEQ ID NO: 63 and SEQ ID NO: 64), which included a *Bsp*EI restriction site in *leuB* and incorporated a *Not*I restriction site 3' of the stop codon of *leuD* during the PCR reaction. The PCR was performed with 50 µl of Pfu Ultra II Hotstart 2X master mix (Agilent Technologies, Santa Clara, CA), 1 µl of a mix of the two primers (10 µmoles of each), 1 µl of *E. coli* BW25113 genomic DNA, and 48 µl of water. The PCR began with a two minute incubation at 95°C, followed by 30 cycles of 20 seconds at 95°C for denaturation, 20 seconds for annealing at 64°C, and two minutes at 72°C for extension. The sample was incubated at 72°C for three minutes and then held at 4°C. The PCR product (*leuBCD* insert) was purified using a QIAquick® PCR Purification Kit (Qiagen, Valencia, CA).

The *leuBCD* insert and the bacterial expression vector pTrcHisA *Mj cimA* were digested with BspEI. The digested vector and *leuBCD* insert were again purified using a QIAquick® PCR purification columns prior to being restriction digested with *Not*I. Following final column purification, the digested vector and insert were ligated using Fast-Link (Epicentre Biotechnologies, Madison, WI). The ligation mix was then used to transform *E. coli* TOP10 cells (Invitrogen, Carlsbad, CA). Recombinant plasmids were isolated using a QIAPrep® Spin Miniprep Kit (Qiagen) and characterized by gel electrophoresis of restriction digests with *Afl*III. DNA sequencing confirmed that the *leuBCD* insert had been cloned and that the insert encoded the published amino acid sequences (GenBank Accession No. AAC73184 (*Ec leuB*) (SEQ ID NO: 65); GenBank Accession No. AAC73183 (*Ec leuC*) (SEQ ID NO: 66); and GenBank Accession No. AAC73182 (*Ec leuD*) (SEQ ID NO: 67)). The resulting plasmid was designated pTrc *Mj cimA Ec leuBCD.*

The AHAS genes (*ilvIH, ilvIH* G14D, *ilvGM,* and *Bs ilvBH*), flanked by 5' *Not*I and 3' *Eco*RI sites (described above), were cloned into the *Not*I and *EcoRI* sites of the expression plasmid pTrc *Mj cimA Ec leuBCD* and designated as follows:
*E. coli* AHAS III *ilvIH* (SEQ ID NO: 36) → pTrc *Mj cimA Ec leuBCD Ec ilvIH*
*E. coli* AHAS III *ilvIH* (G14D) (SEQ ID NO: 41) → pTrc *Mj cimA Ec leuBCD Ec ilvI*H (G14D)
*E. coli* BL21(DE3) AHAS II *ilvGM* (SEQ ID NO: 46) → pTrc *Mj cimA Ec leuBCD Ec ilvGM*
*B. subtilis* AHAS *ilvBH* (SEQ ID NO: 51) → pTrc *Mj cimA Ec leuBCD Ec ilvBH.*

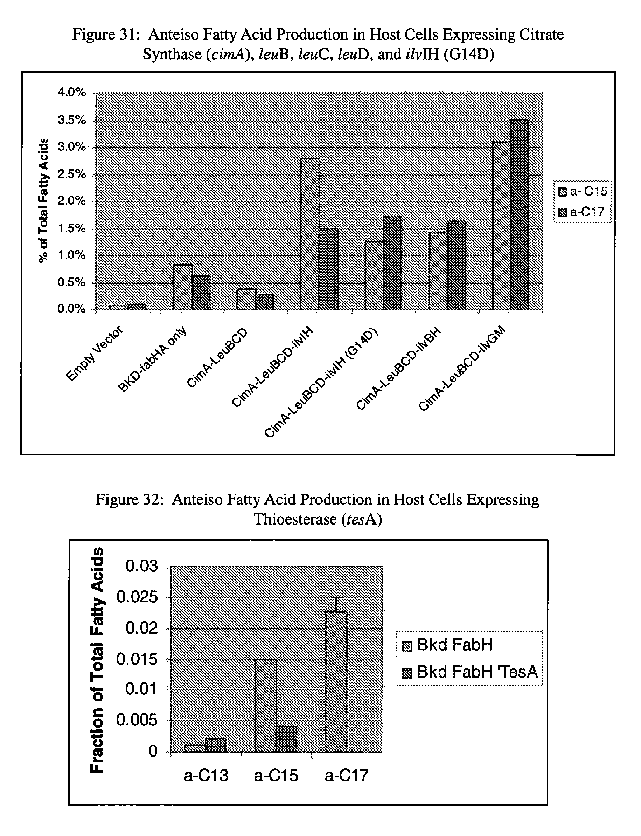

A sample of K27-Z1 comprising pZA31 *Bs bkd fabHA* and pTrc *Mj cimA Ec leuBCD Ec ilvGM* was deposited with American Type Culture Collection (ATCC), 10801 University Blvd., Manassas, VA, on December 14, 2010, under the provisions of the Budapest Treaty for the International Recognition of the Deposit of Microorganisms for the Purpose of Patent Procedure ("Budapest Treaty"), and assigned Deposit Accession No. [XXX] on [DATE]. Expression of these recombinant polynucleotides in an *E. coli* host producing FabH and Bkd from *B. subtilis* further increased anteiso fatty acid production, as demonstrated for anteiso fatty acids of chain lengths of fifteen and seventeen carbons (Figure 31).

### Example 15: Tailoring anteiso fatty acid chain length with thioesterase.

This example illustrates a method of tailoring anteiso fatty acid chain length using thioesterase. The method described herein is useful for, e.g., producing a pool of fatty acids of predetermined chain length for commercial applications.

An expression vector (pTrc *Ec 'tesA*) was constructed comprising a nucleic acid sequence encoding the *E*. *coli* enzyme 'TesA, which has thioesterase activity (Cho et al., J. Biological Chemistry, 270: 4216-9 (1995)). A truncated *E*. *coli tesA* ('*tesA*) cDNA (SEQ ID NO: 68) was created by PCR amplification of the *E. coli tesA* gene (GenBank Accession No. L06182). A 5' primer (SEQ ID NO: 69) was designed to anneal after the 26th codon of *tesA,* modifying the 27th codon from an alanine to a methionine and creating a *Nco*I restriction site. A 3' primer (SEQ ID NO: 71) was designed to incorporate a *Bam*HI restriction site. PCR was performed with 50 µl of Pfu Ultra II Hotstart 2X master mix (Agilent Technologies, Santa Clara, CA), 1 µl of a mix of the two primers (10 µmoles of each), 1 µl of *E. coli* BW25113 genomic DNA, and 48 µl of water. The PCR began with a two minute incubation at 95°C, followed by 30 cycles of 20 seconds at 95°C for denaturation, 20 seconds for annealing at 58°C, and 15 seconds at 72°C for extension. The sample was incubated at 72°C for three minutes and then held at 4°C. The PCR product (*Ec* '*tesA*) was purified using a QIAquick® PCR Purification Kit (Qiagen, Valencia, CA). The bacterial expression vector pTrcHisA and '*tesA* PCR product were digested with *Nco*I and *Bam*HI. The digested vector and insert were ligated using Fast-Link (Epicentre Biotechnologies, Madison, WI). The ligation mix was then used to transform *E*. *coli* TOP10 cells (Invitrogen, Carlsbad, CA). Recombinant plasmids were isolated using a QIAPrep® Spin Miniprep Kit (Qiagen) and characterized by gel electrophoresis of restriction digests with *Hae*II. DNA sequencing confirmed that the '*tesA* insert had been cloned and that the insert encoded the expected amino acid sequences (SEQ ID NO: 73). The resulting plasmid was designated pTrc *Ec 'tesA.*

To limit gene expression, the truncated *E*. *coli 'tesA* gene was subcloned into a low-copy bacterial expression vector pZS21-MCS (Expressys, Ruelzheim, Germany). The expression vector pTrc *Ec 'tesA* was a template in a PCR reaction using a 5' primer (SEQ ID NO: 74) designed to create a flanking *Xho*I restriction site and include the pTrcHisA lac promoter (to replace the pZS21-MCS vector *tet* promoter) and a 3' primer (SEQ ID NO: 75) incorporating a *Hind*III restriction site. The PCR was performed with 50 µl of Pfu Ultra II Hotstart 2X master mix (Agilent Technologies, Santa Clara, CA), 1 µl of a mix of the two primers (10 µmoles of each), 1 µl of pTrc *Ec 'tesA* plasmid DNA (6 ng), and 48 µl of water. The PCR began with a two-minute incubation at 95 °C, followed by 30 cycles of 20 seconds at 95 °C for denaturation, 20 seconds for annealing at 57°C, and 20 seconds at 72°C for extension. The sample was incubated at 72°C for three minutes and then held at 4°C. The PCR product was purified using a QIAquick® PCR Purification Kit (Qiagen, Valencia, CA). The bacterial expression vector pZS21-MCS and the *Ec 'tesA* PCR product were digested with *Xho*I and *Hind*III. The digested vector and insert were ligated using Fast-Link (Epicentre Biotechnologies, Madison, WI). The ligation mix was then used to transform *E*. *coli* TOP10 cells (Invitrogen, Carlsbad, CA). Recombinant plasmids were isolated using a QIAPrep® Spin Miniprep Kit (Qiagen) and characterized by gel electrophoresis of restriction digests with *HaeII.* DNA sequencing confirmed that the '*tesA* insert had been cloned and that the insert encoded the expected amino acid sequences (SEQ ID NO: 73). The resulting plasmid was designated pZS22 *Ec 'tesA.*

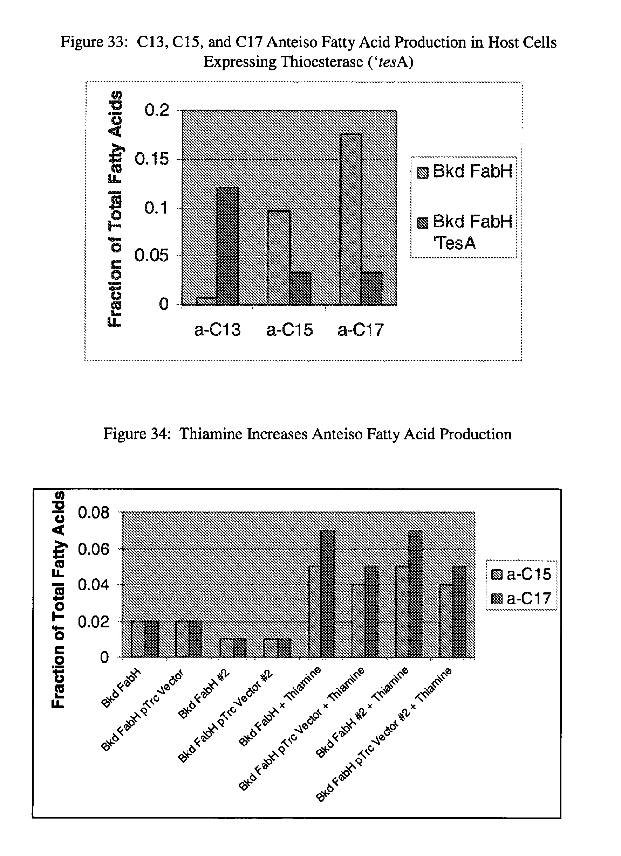

The expression vectors were introduced into *E. coli* host cells. Host cells producing 'TesA generated more mid-chain-length (thirteen carbons) anteiso fatty acids and less longer-chain fatty acids (fifteen and seventeen carbons) compared to host cells that did not produce 'TesA (Figure 32). '*tesA* expression also led to the production of shortened anteiso fatty acids in a BL21 Star (DE3) strain of *E. coli* (Figure 33). Surprisingly, the *Ec 'tesA*-containing *E. coli* BL21 Star (DE3) strain produced more anteiso fatty acids than with the *Ec 'tesA*-containing *E*. *coli* K-12 derivative strain.

This example demonstrates that overexpression of a thioesterase increases the proportion of medium chain length anteiso fatty acids (e.g., anteiso fatty acids 13 carbons in length) produced by a host microorganism.

### Example 16. Thiamine increases anteiso fatty acid synthesis.

Thiamine (vitamin B1) is a cofactor for two enzymes (AHAS and Bkd) responsible for production of anteiso fatty acids. Thiamine was added to LB (modified for lower salt) and an increase in anteiso C15 and C17 fatty acids was observed (Figure 34).

### Example 17. Synthesis of anteiso fatty acid in E. coli producing Listeria FabH.

This example demonstrates the production of anteiso and iso fatty acids by a microbe engineered to produce exogenous 3-ketoacyl-ACP synthase.

The *L. monocytogenes* 10403S 3-ketoacyl-ACP synthase III (*fabH*) gene (GenBank Accession No. FJ749129.1; SEQ ID NO: 77) was codon-optimized for expression in *E. coli* and synthesized to include 5'-*Xho*I and 3'-*Pst*I restriction sites (SEQ ID NO: 78). The resulting synthesized and sequenced DNA was sub-cloned into a pMA vector (GENEART Inc., Toronto, ON, Canada). To generate an expression plasmid where *Listeria fabH* is fused to a polyhistidine tag, the pMA vector containing the *L. monocytogenes fabH* gene was digested with *Xho*I and *Pst*I and ligated (Fast-Link Epicentre Biotechnologies, Madison, WI) with *Xho*I/*Pst*I-digested pBAD/HisA (Invitrogen, Carlsbad, CA). The ligation mix was used to transform *E*. *coli* DH5α™ (Invitrogen Carlsbad, CA). Isolated colonies were screened by PCR using a sterile toothpick stab as an inoculum into a reaction tube containing only water, followed by addition of PCR reaction cocktail (AccuPrime™ SuperMixII, Invitrogen Carlsbad, CA) and primers as described above (SEQ ID NO: 79, SEQ ID NO: 80). Recombinant plasmids were isolated and purified using the QIAPrep® Spin Miniprep Kit (Qiagen) and characterized by restriction enzyme digestion (*Dra*I, *Mfe*I, and *Hae*II (New England Biolabs, Beverly, MA)). The plasmids were subsequently used to transform BW25113 (*E*. *coli* Genetics Stock Center, New Haven, CT) made competent using the calcium chloride method. Transformants were selected on Luria agar plates containing 100 µg/ml ampicillin. Plasmid DNA was isolated and purified using the QIAfilter™ Plasmid Midi Kit (Qiagen). The resulting plasmid incorporating a polyhistidine tag was designated pBAD *Lm_fabH+.* This plasmid and pZA31 were used together to transform BW25113.

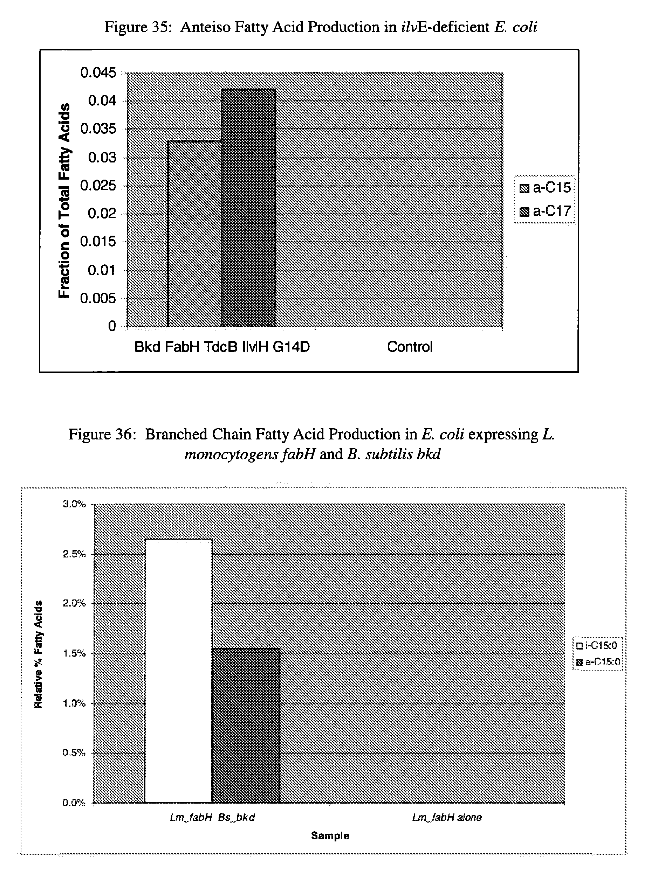

Transduced cells were cultured in Luria broth. When the culture reached an optical density (600 nm) of 0.4-0.6, arabinose (0.2%) was added to induce *fabH* expression. Lipid was harvested from the cell pellet and examined by gas chromatography, revealing peaks that matched the mobility of C15 anteiso and C15 iso fatty acid standards. The identity of the peaks was confirmed by gas chromatography followed by mass spectrometry (Figure 36).

This example demonstrates the production of anteiso and iso fatty acids by a microbe (*E*. *coli* strain BW25113) expressing exogenous 3-ketoacyl-ACP synthase (*Listeria fabH*) and exogenous branched-chain α-ketoacid dehydrogenase (*Bacillus bkd*).

### Example 18. Acetohydroxy acid isomeroreductase and dihydroxyacid dehydratase enhance anteiso fatty acid production.

*E. coli* strains expressing recombinant *Ec tdcB* exhibit increased linear C15 (n-C15) fatty acid production (Figure 29), suggesting that 2-oxobutanoate (also referred to as 2-ketobutyrate) gives rise to an increase in propionyl-CoA, which is used as a primer for synthesis of straight fatty acids with an odd number of carbons (Figure 2). Production of a recombinant AHAS decreases n-C15 fatty acid levels and increases C15 anteiso (a-C15) fatty acid levels, suggesting depletion of 2-oxobutanoate by AHAS (Figure 30). The greater effect is on n-C15 depletion, suggesting that not all 2-oxobutanoate is directed to anteiso fatty acid production. In one embodiment of the invention, IlvC and/or IlvD, the enzymes that catalyze the two chemical conversions following production of 2-oxobutanoate in the anteiso fatty acid synthesis pathway, are overexpressed to increase anteiso fatty acid production. To generate a transcriptional fusion of *E. coli* genes *ilvC* (encoded by the nucleic acid sequence set forth in GenBank Accession No. U00096.2 at position 3955993 to position 3957468; SEQ ID NO: 81) and *ilvD* (encoded by the nucleic acid sequence set forth in GenBank Accession No. U00096.2 at position 3951501 to position 3953351; SEQ ID NO: 82) encoding acetohydroxy acid isomeroreductase and dihydroxyacid dehydratase, respectively, codon-optimized synthetic DNA is flanked with *Xho*I and *Mlu*I sites (SEQ ID NO: 83) and ligated into the expression vector pZS22-MCS. The *ilvCD* genes are operably linked to a *trc* promoter from pTrc HisA. The insert encodes the *trc* promoter, *lac* operator, *rrnB* anti-termination sequences, T7 gene 10 translational enhancer, ribosome binding site, the published amino acid sequences for IlvC (GenBank Accession No. AAC76779) and IlvD (GenBank Accession No. AAT48208.1) (SEQ ID NO: 84 and SEQ ID NO: 85 respectively), and unique restriction sites for *Not*I, *Pme*I, *Eco*RI, and *Xba*I.

### Example 19. Attenuation of transaminase activity encoded by Ec ilvE in anteiso fatty acid biosynthesis.

Carbon flow through the metabolic pathway for anteiso fatty acid production can be diverted to isoleucine production via the transaminase IlvE (Figure 2). This example illustrates a method of enhancing anteiso fatty acid by attenuating IlvE activity.

An *ilvE* deletion mutant, *E. coli* JW5606-1 (*E. coli* Genetic Stock Center, Yale University, New Haven, CT), was made chemically competent using calcium chloride. Cells were transformed with recombinant plasmids containing *B. subtilis bkd, B. subtilis fabHA, E. coli tdcB,* and *E. coli ilvIH,* or empty vector controls, pZA31MCS & pTrcHisA. Transformed cells (40 ml) were cultured in M9 minimal media supplemented with L-isoleucine, L-valine and L-leucine, each at 0.1% final concentration. When the culture reached an optical density (600 nm) of 0.4-0.6, arabinose (0.2%), isopropyl β-D-thiogalactopyranoside (1 mM) and anhydrotetracycline (100 ng/ml) were added to induce gene expression. After approximately 48 hours, lipid was harvested from the cell culture suspension, hydrolyzed, converted to methyl esters, and examined by gas chromatography. The presence of recombinant *Bs fabHA, Bs bkd, Ec tdcB,* and *Ec ilvlH* G14D led to anteiso fatty acid production in a strain deficient in *Ec ilvE* (Figure 35).

### Example 20. Construction of enoyl-ACP reductase expression vector.

Enoyl-ACP reductase, the *E. coli fabI* product, catalyzes a rate-limiting step in fatty acid synthesis (Zheng et al., J. Microbiol. Biotechnol. 20: 875-80 (2010)). This example provides a method for producing an expression vector encoding an enoyl-ACP reductase. In some embodiments, an expression construct encoding enoyl-ACP reductase is introduced into a microbe that does not naturally generate branched fatty acids, such as *E. coli,* to enhance branched fatty acid production. In one embodiment, the enoyl-ACP reductase is modified to increase activity on branched fatty acids.

To construct an expression vector encoding *B. subtilis* enoyl-CoA reductase (encoded by *fabI* (SEQ ID NO: 92)), *B. subtilis* genomic DNA was prepared from *B. subtilis* strain 168 (*Bacillus* Genetic Stock Center, Columbus, OH) by picking an isolated colony from a Luria agar plate, suspending the colony in 41 µL of sterile Milli-Q water, and directly amplifying using a PCR reaction with gene-specific primers. To generate an expression plasmid not encoding a polyhistidine tag, *B. subtilis fabI* was amplified from the genomic DNA sample by PCR using primers (SEQ ID NO: 87 and SEQ ID NO: 88), which incorporated flanking restriction sites for *Nco*I and *Pst*I into the amplified DNA (SEQ ID NO: 89). To generate an expression plasmid where *fabI* would be fused to a polyhistidine tag, *B. subtilis fabI* was amplified from the genomic DNA sample by PCR using primers (SEQ ID NO: 91 and SEQ ID NO: 88), which incorporated flanking restriction sites for *Xho*I and *Pst*I into the amplified DNA (SEQ ID NO: 90).

PCR was run on samples having 41 µl of water and one suspended colony of *B. subtilis* 168, 1.5 µl of a 10 µM stock of each primer, 5 µl of 10X Pfx reaction mix (Invitrogen Carlsbad, CA), and 0.5 µl of Pfx DNA polymerase (1.25 units). PCR conditions were as follows: the samples were initially incubated at 95°C for three minutes, followed by 30 cycles at 95°C for 30 seconds (strand separation), 58°C for 30 seconds (primer annealing), and 68°C primer extension for 1.5 minutes. Following these cycles, there was a ten minute incubation at 68°C, and the samples were then held at 4°C.

The PCR products were purified using a QIAquick® PCR Purification Kit (Qiagen), double digested with restriction enzymes *Xho*I/*Pst*I or *Nco*I/*Pst*I, and ligated (Fast-Link Epicentre Biotechnologies, Madison, WI) into *Xho*I/*Pst*I or *Nco*I/*Pst*I-digested pTrc/His A (Invitrogen, Carlsbad, CA). The ligation mix was used to transform *E. coli* DH5α™ (Invitrogen Carlsbad, CA). Isolated colonies were screened by PCR using a sterile toothpick stab as an inoculum into a reaction tube containing only water, followed by addition of PCR reaction cocktail (AccuPrime™ SuperMixII, Invitrogen Carlsbad, CA) and primers as described above.

Recombinant plasmids were isolated and purified using the QIAPrep® Spin Miniprep Kit (Qiagen) and characterized by restriction enzyme digestion (*Xho*I+*Pst*I, *Nco*I+*Pst*I, *Dra*I, *Mfe*I, and *Hae*II (Invitrogen, Carlsbad, CA or New England Biolabs, Beverly, MA)). The plasmids were subsequently used to transform chemically competent BL21 STAR (DE3) (Invitrogen, Carlsbad, CA). Transformants were selected on Luria agar plates containing 100 µg/ml ampicillin. Plasmid DNA was isolated and purified using the QIAfilter™ Plasmid Midi Kit (Qiagen). DNA sequencing confirmed that the *fabI* inserts had been cloned and that the inserts encoded the FabI amino acid sequence (SEQ ID NO: 89). The resulting plasmid lacking a polyhistidine tag was designated pTrc *Bs_fabI-* and the plasmid incorporating a polyhistidine tag was designated pTrc *Bs_fabI+.*

## Claims

1. A method for producing anteiso fatty acid, the method comprising culturing a cell comprising at least one overexpressed polynucleotide, wherein an overexpressed polynucleotide is a polynucleotide native to the cell, the product of which is generated in a greater amount than that normally found in the cell, or exogenous polynucleotide, said exogenous or overexpressed polynucleotide comprising a nucleic acid sequence encoding a polypeptide that catalyzes at least one of the following reactions:
(aa) conversion of pyruvate to citramalate;
(bb) conversion of citramalate to citraconate;
(cc) conversion of citraconate to β-methyl-D-malate;
(dd) conversion of β-methyl-D-malate to 2-oxobutanoate; or
(ee) conversion of threonine to 2-oxobutanoate
under conditions allowing expression of the polynucleotide(s) and production of anteiso fatty acid, wherein the cell produces more anteiso fatty acids than an otherwise similar cell that does not comprise the polynucleotide(s).

2. The method of claim 1, further comprising extracting from the culture the anteiso fatty acid or a product derived from anteiso fatty acid.

3. The method of claim 1, wherein the cell further comprises at least one exogenous or overexpressed polynucleotide comprising a nucleic acid sequence encoding a polypeptide that catalyzes at least one of the following reactions:
(ff) conversion of 2-oxobutanoate to 2-aceto-2-hydroxy-butyrate,
(gg) conversion of 2-aceto-2-hydroxy-butyrate to 2,3-dihydroxy-3-methylvalerate, or
(hh) conversion of 2,3-dihydroxy-3-methylvalerate to 2-keto-3-methylvalerate, and, optionally, the cell is modified to attenuate branched-chain amino acid aminotransferase activity.

4. The method of claim 3, wherein the cell comprises exogenous or overexpressed polynucleotides encoding polypeptides that catalyze reactions (ee) and (ff).

5. The method of claim 4, wherein the cell comprises an exogenous or overexpressed polynucleotide encoding a threonine deaminase and an exogenous or overexpressed polynucleotide encoding an acetohydroxy acid synthase.

6. The method of claim 5, wherein the threonine deaminase is *E. coli* TdcB and/or the acetohydroxy acid synthase is *E. coli* IlvIH, *E. coli* IlvIH (G14D), *E. coli* IlvGM, or *B. subtilis* IlvBH.

7. The method of claim 3, wherein the cell further comprises an exogenous or overexpressed polynucleotide comprising a nucleic acid sequence encoding a branched-chain amino acid aminotransferase, an exogenous or overexpressed polynucleotide comprising a nucleic acid sequence encoding a branched-chain α-keto acid dehydrogenase, an exogenous or overexpressed polynucleotide comprising a nucleic acid sequence encoding an acyl transferase, an exogenous or overexpressed polynucleotide comprising a nucleic acid sequence encoding a 3-ketoacyl-ACP synthase, an exogenous or overexpressed polynucleotide comprising a nucleic acid sequence encoding an enoyl-ACP reductase, an exogenous or overexpressed polynucleotide comprising a nucleic acid sequence encoding a thioesterase, or a combination thereof.

8. The method of claim 7, wherein the cell comprises an exogenous or overexpressed polynucleotide comprising a nucleic acid sequence encoding a branched-chain α-keto acid dehydrogenase, an exogenous or overexpressed polynucleotide comprising a nucleic acid sequence encoding a 3-ketoacyl-ACP synthase, and, optionally, an exogenous or overexpressed polynucleotide comprising a nucleic acid sequence encoding a thioesterase.

9. A cell comprising:
(i) an exogenous or overexpressed polynucleotide comprising a nucleic acid sequence encoding a threonine deaminase or an exogenous or overexpressed polynucleotide encoding citramalate synthase;
(ii) an exogenous or overexpressed polynucleotide comprising a nucleic acid sequence encoding a branched-chain α-keto acid dehydrogenase; and
(iii) an exogenous or overexpressed polynucleotide comprising a nucleic acid sequence encoding a 3-ketoacyl-ACP synthase,
wherein an overexpressed polynucleotide is a polynucleotide native to the cell, the product of which is generated in a greater amount than that normally found in the cell, and
wherein the polynucleotides are expressed and the cell produces more antéiso fatty acid than an otherwise similar cell that does not comprise the polynucleotide(s).

10. The cell of claim 9, wherein (i) the threonine deaminase is *E. coli* TdcB or the citramalate synthase is CimA derived from *M. jannaschii,* (ii) the branched-chain α-keto acid dehydrogenase is *B. subtilis* Bkd, and (iii) the 3-ketoacyl-ACP synthase is *B. subtilis* FabH.

## Patentansprüche

1. Verfahren zum Herstellen von Anteisofettsäure, wobei das Verfahren das Kultivieren einer Zelle umfasst, die mindestens ein überexprimiertes Polynucleotid umfasst, wobei ein überexprimiertes Polynucleotid ein für die Zelle natives Polynucleotid, deren Produkt in einer größeren Menge generiert wird, als normalerweise in der Zelle gefunden wird, oder ein exogenes Polynucleotid ist, wobei das exogene oder überexprimierte Polynucleotid eine Nucleinsäuresequenz umfasst, die für ein Polypeptid kodiert, das wenigstens eine der folgenden Reaktionen katalysiert:
(aa) Umwandlung von Pyruvat in Citramalat;
(bb) Umwandlung von Citramalat in Citraconat;
(cc) Umwandlung von Citraconat in β-Methyl-D-malat;
(dd) Umwandlung von β-Methyl-D-malat in 2-Oxobutanoat; oder
(ee) Umwandlung von Threonin in 2-Oxobutanoat
unter Bedingungen, die die Expression des Polynucleotids/der Polynucleotide und Produktion von Anteisofettsäure erlauben, wobei die Zelle mehr Anteisofettsäuren als eine ansonsten ähnliche Zelle produziert, die das Polynucleotid/die Polynucleotide nicht umfasst.

2. Verfahren nach Anspruch 1, ferner umfassend das Extrahieren der Anteisofettsäure oder eines von Anteisofettsäure abgeleiteten Produkts aus der Kultur.

3. Verfahren nach Anspruch 1, wobei die Zelle zudem wenigstens ein exogenes oder überexprimiertes Polynucleotid umfasst, das eine Nucleinsäuresequenz umfasst, die für ein Polypeptid kodiert, das wenigstens eine der folgenden Reaktionen katalysiert:
(ff) Umwandlung von 2-Oxobutanoat in 2-Aceto-2-hydroxybutyrat,
(gg) Umwandlung von 2-Aceto-2-hydroxybutyrat in 2,3-Dihydroxy-3-methylvalerat oder
(hh) Umwandlung von 2,3-Dihydroxy-3-methylvalerat in 2-Keto-3-methylvalerat, und die Zelle wahlweise modifiziert ist, um die Aktivität der Aminotransferase für verzweigtkettige Aminosäuren abzuschwächen.

4. Verfahren nach Anspruch 3, wobei die Zelle exogene oder überexprimierte Polynucleotide umfasst, die für Polypeptide kodieren, die Reaktionen (ee) und (ff) katalysieren.

5. Verfahren nach Anspruch 4, wobei die Zelle ein exogenes oder überexprimiertes Polynucleotid, das für eine Threonindeaminase kodiert, und ein exogenes oder überexprimiertes Polynucleotid umfasst, das für eine Acetohydroxysäuresynthase kodiert.

6. Verfahren nach Anspruch 5, wobei die Threonindeaminase *E. coli* TdcB ist und/oder die Acetohydroxysäuresynthase *E. coli* IlvIH, *E. coli* IlvIH (G14D), *E. coli* IlvGM oder *B. subtilis* IlvBH ist.

7. Verfahren nach Anspruch 3, wobei die Zelle zudem ein exogenes oder überexprimiertes Polynucleotid, das eine Nucleinsäuresequenz umfasst, die für eine Aminotransferase für verzweigtkettige Aminosäuren kodiert, ein exogenes oder überexprimiertes Polynucleotid, das eine Nucleinsäuresequenz umfasst, die für eine verzweigtkettige α-Ketosäuredehydrogenase kodiert, ein exogenes oder überexprimiertes Polynucleotid, das eine Nucleinsäuresequenz umfasst, die für eine Acyltransferase kodiert, ein exogenes oder überexprimiertes Polynucleotid, das eine Nucleinsäuresequenz umfasst, die für eine 3-Ketoacyl-ACP-synthase kodiert, ein exogenes oder überexprimiertes Polynucleotid, das eine Nucleinsäuresequenz umfasst, die für eine Enoyl-ACP-reductase kodiert, ein exogenes oder überexprimiertes Polynucleotid. das eine Nucleinsäuresequenz umfasst, die für eine Thioesterase kodiert, oder eine Kombination davon umfasst.

8. Verfahren nach Anspruch 7, wobei die Zelle ein exogenes oder überexprimiertes Polynucleotid, das eine Nucleinsäuresequenz umfasst, die für eine verzweigtkettige α-Ketosäuredehydrogenase kodiert, ein exogenes oder überexprimiertes Polynucleotid, das eine Nucleinsäuresequenz umfasst, die für eine 3-Ketoacyl-ACP-synthase kodiert, und wahlweise ein exogenes oder überexprimiertes Polynucleotid, das eine Nucleinsäuresequenz umfasst, die für eine Thioesterase kodiert, umfasst.

9. Zelle, umfassend:
(i) ein exogenes oder überexprimiertes Polynucleotid, das eine Nucleinsäuresequenz umfasst, die für eine Threonindeaminase kodiert, oder ein exogenes oder überexprimiertes Polynucleotid, das für Citramalatsynthase kodiert;
(ii) ein exogenes oder überexprimiertes Polynucleotid, das eine Nucleinsäuresequenz umfasst, die für eine verzweigtkettige α-Ketosäuredehydrogenase kodiert, und
(iii) ein exogenes oder überexprimiertes Polynucleotid, das eine Nucleinsäuresequenz umfasst, die für eine 3-Ketoacyl-ACP-synthase kodiert,
wobei ein überexprimiertes Polynucleotid ein für die Zelle natives Polynucleotid ist, deren Produkt in einer größeren Menge generiert wird, als normalerweise in der Zelle gefunden wird, und
wobei die Polynucleotide exprimiert werden und die Zelle mehr Anteisofettsäure als eine ansonsten ähnliche Zelle produziert, die das Polynucleotid/die Polynucleotide nicht umfasst.

10. Zelle nach Anspruch 9, wobei (i) die Threonindeaminase *E. coli* TdcB ist oder die Citramalatsynthase CimaA ist, die von *M. jannaschii* abgeleitet ist, (ii) die verzweigtkettige α-Ketosäuredehydrogenase *B. subtilis* Bkd ist, und (iii) die 3-Ketoacyl-ACP-synthase *B. subtilis* FabH ist.

## Revendications

1. Procédé de production d'acide gras antéiso, le procédé comprenant la culture d'une cellule comprenant au moins un polynucléotide surexprimé, dans lequel un polynucléotide surexprimé est un polynucléotide dans lequel un polynucléotide surexprimé est un polynucléotide natif de la cellule dont le produit est généré en une quantité supérieure à celle que l'on trouve normalement dans la cellule, et natif de la cellule dont le produit est généré en une quantité supérieure à celle que l'on trouve normalement dans la cellule, ou un polynucléotide exogène, ledit polynucléotide exogène ou surexprimé comprenant une séquence d'acide nucléique codant pour un polypeptide qui catalyse au moins l'une des réactions suivantes :
(aa) conversion de pyruvate en citramalate ;
(bb) conversion de citramalate en citraconate ;
(cc) conversion de citraconate en β-méthyl-D-malate ;
(dd) conversion de β-méthyl-D-malate en 2-oxobutanoate ; ou
(ee) conversion de thréonine en 2-oxobutanoate
dans des conditions permettant l'expression du ou des polynucléotide(s) et la production d'acide gras antéiso, dans lequel la cellule produit plus d'acides gras antéiso qu'une cellule par ailleurs similaire qui ne comprend pas le ou les polynucléotide(s).

2. Procédé selon la revendication 1, comprenant en outre l'extraction de la culture de l'acide gras antéiso ou d'un produit dérivé de l'acide gras antéiso.

3. Procédé selon la revendication 1, dans lequel la cellule comprend en outre au moins un polynucléotide exogène ou surexprimé comprenant une séquence d'acide nucléique codant pour un polypeptide qui catalyse au moins l'une des réactions suivantes :
(ff) conversion de 2-oxobutanoate en 2-acéto-2-hydroxy-butyrate,
(gg) conversion de 2-acéto-2-hydroxy-butyrate en 2,3-dihydroxy-3-méthylvalérate, ou
(hh) conversion de 2,3-dihydroxy-3-méthylvalérate en 2-céto-3-méthylvalérate, et, facultativement, la cellule est modifiée pour atténuer l'activité de l'aminotransférase d'acide aminé à chaîne ramifiée.

4. Procédé selon la revendication 3, dans lequel la cellule comprend des polynucléotides exogènes ou surexprimés codant pour des polypeptides qui catalysent les réactions (ee) et (ff).

5. Procédé selon la revendication 4, dans lequel la cellule comprend un polynucléotide exogène ou surexprimé codant pour une thréonine désaminase et un polynucléotide exogène ou surexprimé codant pour une acétohydroxy acide synthase.

6. Procédé selon la revendication 5, dans lequel la thréonine désaminase est l'E. *coli* TdcB et/ou l'acétohydroxy acide synthase est l'*E*. *coli* IlvIH, l'*E*. *coli* IlvIH (G14D), *l'E. coli* IlvGM, ou le *B. subtilis* IlvBH.

7. Procédé selon la revendication 3, dans lequel la cellule comprend en outre un polynucléotide exogène ou surexprimé comprenant une séquence d'acide nucléique codant pour une aminotransférase d'acide aminé à chaîne ramifiée, un polynucléotide exogène ou surexprimé comprenant une séquence d'acide nucléique codant pour une α-cétoacide déshydrogénase à chaîne ramifiée, un polynucléotide exogène ou surexprimé comprenant une séquence d'acide nucléique codant pour une acyltransférase, un polynucléotide exogène ou surexprimé comprenant une séquence d'acide nucléique codant pour une 3-cétoacyl-ACP synthase, un polynucléotide exogène ou surexprimé comprenant une séquence d'acide nucléique codant pour une énoyl-ACP-réductase, un polynucléotide exogène ou surexprimé comprenant une séquence d'acide nucléique codant pour une thioestérase, ou une combinaison de ceux-ci.

8. Procédé selon la revendication 7, dans lequel la cellule comprend un polynucléotide exogène ou surexprimé comprenant une séquence d'acide nucléique codant pour une α-cétoacide déshydrogénase à chaîne ramifiée, un polynucléotide exogène ou surexprimé comprenant une séquence d'acide nucléique codant pour une 3-cétoacyl-ACP synthase, et, facultativement, un polynucléotide exogène ou surexprimé comprenant une séquence d'acide nucléique codant pour une thioestérase.

9. Cellule comprenant :
(i) un polynucléotide exogène ou surexprimé comprenant une séquence d'acide nucléique codant pour une thréonine désaminase ou un polynucléotide exogène ou surexprimé codant pour une citramalate synthase ;
(ii) un polynucléotide exogène ou surexprimé comprenant une séquence d'acide nucléique codant pour une α-céto-acide déshydrogénase à chaîne ramifiée, et
(iii) un polynucléotide exogène ou surexprimé comprenant une séquence d'acide nucléique codant pour une 3-cétoacyl-ACP synthase,
dans lequel les polynucléotides sont exprimés et la cellule produit plus d'acide gras antéiso qu'une cellule par ailleurs similaire qui ne comprend pas le ou les polynucléotide(s).

10. Cellule selon la revendication 9, dans lequel (i) la thréonine désaminase est l'E. *coli* TdcB ou la citramalate synthase est CimA dérivée du M. *jannaschii,* (ii) la α-cétoacide déshydrogénase à chaîne ramifiée est le *B. subtilis* Bkd et (iii) la 3-cétoacyl-ACP synthase est le *B. subtilis* FabH.
